(19) Europäisches Patentamt
European Patent Office
Office européen des brevets

(11) EP 1 243 648 A1

(12) # EUROPEAN PATENT APPLICATION
published in accordance with Art. 158(3) EPC

(43) Date of publication:
**25.09.2002 Bulletin 2002/39**

(21) Application number: **00985985.1**

(22) Date of filing: **28.12.2000**

(51) Int Cl.7: **C12N 15/09**, C12N 1/15, C12N 1/19, C12N 1/21, C12N 5/10, C07K 14/705, C07K 16/28, C12P 21/02, C12Q 1/02, C12Q 1/68, A61K 31/711, A61K 48/00, A61P 43/00, G01N 33/15, G01N 33/50

(86) International application number:
**PCT/JP00/09408**

(87) International publication number:
**WO 01/048188 (05.07.2001 Gazette 2001/27)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.12.1999 JP 37515299**
**31.03.2000 JP 2000101339**

(71) Applicant: **Mitsubishi Pharma Corporation**
**Osaka-shi, Osaka 541-0046 (JP)**

(72) Inventors:
- **MATSUMOTO, Shun-Ichiro**
  **Funabashi-shi, Chiba 273-0005 (JP)**
- **ODA, Tamaki**
  **Kisarazu-shi, Chiba 292-0054 (JP)**
- **SAITO, Youko**
  **Kisarazu-shi, Chiba 292-0043 (JP)**
- **MORIKAWA, Noriyuki**
  **kisarazu-shi, Chiba 292-0833 (JP)**
- **YOSHIDA, Kenji**
  **Kisarazu-shi, Chiba 292-0043 (JP)**
- **SUWA, Makiko**
  **Ota-ku, Tokyo 144-0052 (JP)**
- **SUGIYAMA, Tomoyasu**
  **Kisarazu-shi, Chiba 292-0045 (JP)**
- **KISHIMOTO, Toshimitsu**
  **2-chome, Chuo-ku, Tokyo 103-8405 (JP)**
- **KANZAKI, Kouji**
  **2-chome, Chuo-ku Tokyo 103-8405 (JP)**
- **YASUDA, Shin-Ichiro**
  **2-chome, Chuo-ku Tokyo 103-8405 (JP)**
- **INOUE, Yoshihisa**
  **2-chome, Chuo-ku Tokyo 103-8405 (JP)**

(74) Representative: **Grünecker, Kinkeldey, Stockmair & Schwanhäusser Anwaltssozietät**
**Maximilianstrasse 58**
**80538 München (DE)**

(54) **NOVEL GUANOSINE TRIPHOSPHATE-BINDING PROTEIN-COUPLED RECEPTORS, GENES THEREOF AND PRODUCTION AND USES THEREOF**

(57) Nine novel genes sustaining hydrophobic domains, which are estimated to be seven transmembrane domains characteristic to G protein-coupled receptors, are successfully isolated by human tissue cDNA screening. These genes and proteins which are the expression products thereof are usable in screening ligands, screening agonists or antagonists which are useful as drugs, diagnosing diseases in which these gene participate, etc.

EP 1 243 648 A1

## Description

### Technical Field

The present invention relates to novel G protein-coupled receptors and genes thereof, and production and uses thereof.

### Background Art

G protein-coupled receptor is a generic name for the group of cell membrane receptors transducing signals into cells *via* the activation of trimer-type GTP-binding proteins. The G protein-coupled receptor has structural characteristic of seven transmembrane domains in a molecule, and thus called also as "a seven-transmembrane receptor". The G protein-coupled receptor transmits the information consisting of various physiologically active substances into cells across the cell membrane *via* the activation of the trimer-type GTP-binding protein and the change of the intracellular second messengers caused thereby. Well-known intracellular second messengers that are regulated by the trimer-type GTP-binding protein include cAMP mediated by adenylate cyclase, and $Ca^{2+}$ mediated by phospholipase C. Recent studies have shown that many types of intracellular proteins serve as the targets thereof; for example, the regulation of channels and activation of phosphorylation enzymes are mediated by the trimer-type GTP-binding protein (*Annu. Rev. Neurosci.* (97) 20:399). There are a wide variety of substrates (ligands) for the G protein-coupled receptor, for example, protein hormone; chemokine; peptide; amine; substances derived from lipids; and protease, such as thrombin, is also one such example. The number of human G protein-coupled receptors whose genes have been identified recently, is a little under 300, excluding the sensory-type receptors. However, the number of G protein-coupled receptors to which the ligands have been identified is only about 140 types. Thus, there are 100 or more, ligand-unknown, "orphan G protein-coupled receptors". The human genome has been assumed to contain at least 400 types, and possibly up to 1000 types of G protein-coupled receptors (*Trends Pharmacol. Sci.* (97) 18:430). This means that the number of functionally unknown orphan G protein-coupled receptors can be exploding accompanied by the rapid progress of the genome analysis.

Ninety % or more drugs that have so far been produced by the pharmaceutical companies in the world aim at the interaction in extracellular spaces, and low-molecular-weight drugs comprises the majority of those relating to G protein-coupled receptors. The reason is that the G protein-coupled receptor-related diseases include many types of diseases, such as those of the cerebral nervous system, circulatory system, digestive system, immune system, locomotor system, urinary system, and genital system, including genetic diseases. Thus, in recent years, many pharmaceutical companies retain their orphan G protein-coupled receptors found through the genome analysis, and are competing fiercely with each other to reveal the ligands and physiological functions. Based on this, successful cases of physiological screening of ligands to some novel G protein-coupled receptors have begun to be reported recently. For example, the cases of a calcitonin-related peptide receptor (*J. Biol. Chem.* (96) 271:11325), orexin (*Cell* (98) 92: 573), and prolactin-releasing peptide (*Nature* (98) 393:272) gave a great impact to basic studies in the field of life science.

In particular, as potential new targets to bring about the drug development, the orphan G protein-coupled receptors have become a center of attraction. In general, since there are no specific ligands to the orphan G protein-coupled receptors, it has been difficult to develop agonists or antagonists . However, in recent years, creation of orphan G protein-coupled receptor-targeted drugs by combining the enriched compound libraries and high-throughput screening methods has been proposed (*Trends Pharmacol. Sci.* (97) 18:430, *Br. J. Pharm.* (98) 125:1387). Specifically, in the creation comprises identifying physiological agonists of an orphan G protein-coupled receptor identified by genetic engineering, by functional screening utilizing alterations in the level of an intracellular messenger, cAMP or $Ca^{2+}$, as an index, and then analyzing the *in vivo* functions. In this method, high-throughput screening achieved by using a compound library allows theoretically to discover surrogate agonists and antagonists specific to the orphan G protein-coupled receptor, and further, to develop therapeutic agents for particular diseases.

### Disclosure of the Invention

The present invention was achieved considering the present situation surrounding G protein-coupled receptors, and an objective thereof is to provide novel G protein-coupled receptors and their genes, and a method for producing and uses of them. Another objective of the present invention is to provide these molecules as targets for the study of drug development.

The present inventors studied strenuously to achieve the above-mentioned objectives, and successfully isolated nine novel genes comprising nucleotide sequences encoding hydrophobic regions considered to be seven transmembrane domains, which are characteristic of the G protein-coupled receptors, by polymerase chain reaction using cDNAs from human tissues as templates. These genes and the proteins as the translation products can be used in the screening of ligand and of agonist or antagonist useful as a pharmaceutical, or can be used for diagnosing diseases relating to these genes.

Thus, the present invention relates to novel G protein-coupled receptors and the genes encoding them, and the uses and production thereof. More specifically, the present invention provides:

(1) a DNA that encodes a guanosine triphosphate-binding protein-coupled receptor, wherein said DNA is selected from the group consisting of the following (a) to (d):

(a) a DNA encoding a protein comprising the amino acid sequence of any one of SEQ ID NOs: 1 to 4 and 17 to 21;
(b) a DNA comprising a coding region of the nucleotide sequence of any one of SEQ ID NOs: 5 to 8 and 22 to 26;
(c) a DNA encoding a protein comprising the amino acid sequence of any one of SEQ ID NOs: 1 to 4 and 17 to 21 in which one or more amino acids are substituted, deleted, added, and/or inserted; and
(d) a DNA hybridizing under stringent conditions to the DNA comprising the nucleotide sequence of any one of SEQ ID NOs: 5 to 8 and 22 to 26;

(2) a DNA encoding a partial peptide of a protein comprising the amino acid sequence of any one of SEQ ID NOs: 1 to 4 and 17 to 21;
(3) a vector comprising the DNA of any one of (1) and (2) ;
(4) a transformant carrying the DNA of any one of (1) and (2) or the vector of (3);
(5) a protein or a peptide encoded by the DNA of any one of (1) and (2);
(6) a method for producing the protein or the peptide of (5), said method comprising the steps of culturing the transformant of (4) and recovering an expressed protein or peptide from the transformant or culture supernatant thereof;
(7) a method of screening for ligands that bind to the protein of (5), said method comprising the steps of:

(a) contacting a test sample with the protein or the peptide of (5); and
(b) selecting compounds that binds to said protein or said peptide;

(8) a method of screening for compounds that have activity of inhibiting the binding between the protein of (5) and a ligand thereof, said method comprising the steps of:

(a) contacting the protein of (5) or a partial peptide thereof with the ligand in the presence of a test sample and detecting a binding activity of said protein or said partial peptide with said ligand; and
(b) selecting compounds that reduces the binding activity detected in step (a) as compared with a binding activity detected in the absence of the test sample;

(9) a method of screening for compounds that inhibit or enhance activity of the protein of (5), said method comprising the steps of:

(a) contacting a ligand of said protein with cells expressing said protein in the presence of a test sample,
(b) detecting an alteration in the cells that results from binding of said ligand to said protein, and
(c) selecting compounds that suppress or enhance the alteration detected in step (b) as compared with an alteration detected in the cells in the absence of the test sample;

(10) the method of (8) or (9), wherein the alteration in cells is a change in cAMP concentration or calcium concentration;
(11) an antibody binding to the protein of (5);
(12) a compound isolated by the method of any one of (7) to (10);
(13) a pharmaceutical composition comprising the compound of (12) as an active ingredient;
(14) the pharmaceutical composition of (13), wherein said pharmaceutical composition is formulated for the treatment of a disease selected from the group consisting of cancer, cirrhosis, and Alzheimer's disease;
(15) a polynucleotide comprising at least 15 nucleotides, wherein said polynucleotide is complementary to the DNA comprising the nucleotide sequence of any one of SEQ ID NOs: 5 to 8 and 22 to 26 or a complementary strand thereof;
(16) a method for diagnosing a disease selected from the group consisting of cancer, cirrhosis, and Alzheimer's disease, said method comprising the steps of detecting expression of the DNA of (1) in tissues related to the disease derived from a subject, or mutation in the DNA of (1) in the subject; and
(17) a agent for diagnosing a disease selected from the group consisting of cancer, cirrhosis, and Alzheimer's disease, said agent comprising the antibody of (11) or the nucleotide of (15).

As used herein, the term "G protein-coupled receptor" means a cell membrane receptor transducing signals into cells *via* the activation of the GTP-binding protein.
As used herein, the term "ligand" means a physiological substance binding to the G protein-coupled receptor and transducing signals into cells. Herein, the term "physiological substance" means a compound bound to the G protein-coupled receptor *in vivo.*
As used herein, the term "agonist" means a compound capable of binding to the G protein-coupled receptor and transducing signals into cells, including biological substances, artificially synthesized compounds, and naturally occurring compounds.
As used herein, the term "antagonist" means a compound inhibiting the binding of ligand to the G protein-coupled receptor or inhibiting the signal transduction into cells, including biological substances, artificially synthesized compounds, and naturally occurring compounds.
The present invention provides novel G protein-coupled receptors and the DNAs encoding the proteins. The nine human cDNA clones, isolated by the present inventors and included by the present invention, were named "GPRv8", "GPRv12", "GPRv16", "GPRv21", "GPRv40", "GPRv47", "GPRv51", "GPRv71", and "GPRv72" (as required, these clones are collectively referred to as "GPRv"). The nucleotide sequences of the cDNAs are shown in SEQ ID NOs: 5 to 8 and 22 to 26; the amino acid sequences of the proteins encoded by the cDNAs are shown in SEQ ID NOs: 1 to 4 and 17 to 21.
A result obtained by BLAST search showed that amino acid sequence of all the proteins encoded by GPRv cDNAs exhibited significant homology to those of known G protein-coupled receptors. Specifically, "GPRv8" exhibited 36% homology to HUMAN VASOPRESSIN V1B RECEPTOR (P47901, 424 aa); "GPRv12" exhibited 27% homology to RAT 5-HYDROXYTRYPTAMINE 6 RECEPTOR (P31388, 436 aa); "GPRv16" exhibited 28% homology to MOUSE GALANIN RECEPTOR TYPE 1 (P56479, 348 aa); "GPRv21" exhibited 30% homology to BOVIN NEUROPEPTIDE Y RECEPTOR TYPE 2 (P79113, 384 aa); "GPRv40" exhibited 34% homology to OXYTOCIN RECEPTOR (P97926, 388 aa); "GPRv47" exhibited 43% homology to GPRX_ORYLA PROBABLE G PROTEIN-COUPLED RECEPTOR (Q91178, 428 aa); "GPRv51" exhibited 37% homology to PROBABLE G PROTEIN-COUPLED RECEPTOR RTA (P23749, 343 aa); "GPRv71" exhibited 45% homology to Chicken P2Y PURINOCEPTOR 3 (P2Y3) (Q98907, 328 aa) ; "GPRv72" exhibited 30% homology to ALPHA-1A ADRENERGIC RECEPTOR (002824, 466 aa).
Further, all the proteins encoded by GPRv cDNAs (hereinafter also may be referred to as "GPRv protein") , isolated by the present inventors, contained hydrophobic regions, which were assumed to correspond to the seven transmembrane domains characteristic of the G protein-coupled receptor. Based on these findings, all the GPRv cDNAs can be considered to encode proteins belonging to the G protein-coupled receptor family. The G protein-coupled receptors have the activity for transducing signals into cells *via* the activation of the G protein, which is mediated by the ligand. As described above, the receptor are involved in many types of diseases, such as those of the cerebral nervous system, circulatory system, digestive system, immune system, locomotor system, urinary system, and genital system, including genetic diseases. Accordingly, the GPRv proteins can be used to screen for agonists and antagonists regulating the functions of GPRv proteins, and thus become important targets of drug development for the above diseases.
The present invention also provides proteins functionally equivalent to the GPRv proteins. As used herein, the term "functionally equivalent" means that a protein of interest has biological properties identical to those of the GPRv proteins. The biological properties of GPRv proteins include the activity of transducing signals into cells *via* the activation of the trimer-type GTP-binding protein. According to the types of activated systems of intracellular signal transduction, the trimer-type GTP-binding proteins are categorized into three classes, namely, Gq type that increases the $Ca^{2+}$ level, Gs type that increases the cAMP level, and Gi type that reduces the cAMP level (*Trends Pharmacol. Sci.* (99) 20:118). Thus, it can be assessed whether the protein of interest has biological properties identical to those of GPRv proteins, for example, by detecting concentration changes of cAMP or calcium in cells depending on the activation.
In an embodiment, the method for preparing a protein functionally identical to the GPRv protein includes a method of introducing mutations in the amino acids sequence of the protein. Such method includes, for example, site-directed mutagenesis (*Current Protocols in Molecular Biology,* edit. Ausubel et al. (1987) Publish. John Wiley & Sons Section 8.1-8.5)). The amino acid mutations in the protein can be also occurred naturally. The present invention includes mutant proteins, regardless of being generated artificially or naturally, in which one or more amino acids have been substituted, deleted, inserted and/or added in the amino acid sequences (SEQ ID NOs: 1 to 4 and 17 to 21) of GPRv proteins, but the mutant proteins are functionally equivalent to the GPRv proteins. There is no limitation on the number of amino acid mutations and positions of the mutations in the proteins, as far as the functions of GPRv proteins are retained. The number of mutations is assumed to range typically within 10% of the entire amino acids, preferably within 5% of the entire amino acids, further preferably within 1% of the entire amino acids.

In another embodiment of the invention, the method for preparing a protein functionally equivalent to the GPRv protein includes a method using the hybridization technique or gene amplification technique. Specifically, those skilled in the art can typically isolate a DNA having high homology to the DNA sequence encoding the GPRv protein (SEQ ID NOs: 5 to 8 and 22 to 26) or a partial sequence thereof from a DNA sample derived from a homologous or heterologous using the hybridization technique (*Current Protocols in Molecular Biology*, edit. Ausubel et al. (1987) Publish. John Wiley & Sons Section 6.3-6.4) and then obtain a protein functionally equivalent to the GPRv protein. Thus, the protein of the present invention also include a protein encoded by a DNA capable of hybridizing to the DNA encoding the GPRv protein, which is functionally equivalent to the GPRv protein.

Organisms to be used for isolating such a protein include, for example, rat, mouse, rabbit, chicken, pig, cattle, and so forth, in addition to human, but not limited thereto.

Typical stringent hybridization conditions for isolating a DNA encoding a protein functionally equivalent to the GPRv protein are those of "1x SSC, 0.1% SDS, 37°C" or the like; more stringently, those of "0.5x SSC, 0.1% SDS, 42°C" or the like; much more stringently, those of "0.2x SSC, 0.1% SDS, 65°C" or the like. As the hybridization conditions become more stringent, a DNA with higher homology to the probe sequence can be expected to be isolated. However, the above combinations of SSC, SDS, and temperature are only examples, and those skilled in the art can achieve the stringencies equivalent to the above by appropriately combining the above or other factors determining the hybridization stringency (for example, probe concentration, probe length, time of hybridization reaction, and so forth).

The protein encoded by a DNA isolated by using such hybridization technique typically has high homology of amino acid sequence to those of the GPRv protein. The term "high homology" means the degree of sequence homology of at least 40% or higher, preferably 60% or higher, further preferably 80% or higher (for example, 90% or higher, or 95% or higher).

Identity of amino acid sequence or nucleotide sequence can be determined with the BLAST algorithm of Karlin and Altschul (Proc. Natl. Acad. Sci. USA 90:5873-5877, 1993). Based on this algorithm, the programs, BLASTN and BLASTX, have been developed (Altschul et al. J. Mol. Biol. 215: 403-410, 1990). When nucleotide sequences are analyzed by BLASTN based on BLAST, the parameters are set, for example, as follows: score= 100; and wordlength= 12. Alternatively, when amino acid sequences are analyzed by BLASTX based on BLAST, the parameters are set, for example, as follows: score= 50; and wordlength= 3. When BLAST and the Gapped BLAST program are used for the analysis, the default parameters are used in each program. The specific techniques used in these analysis methods are already known (http://www.ncbi.nlm.nih.gov.).

Further, primers are designed based on a part of the DNA sequence (SEQ ID NOs: 5 to 8 and 22 to 26) encoding the GPRv protein, a DNA fragment having high homology to the DNA sequence encoding the GPRv protein is isolated by the gene amplification technique (PCR) (*Current protocols in Molecular Biology,* edit. Ausubel et al. (1987) Publish. John Wiley & Sons Section 6.1-6.4), and then the protein functionally equivalent to the GPRv protein can be obtained.

The present invention also includes partial peptides of the protein of the present invention. These partial peptides include peptides binding to the ligand but not transducing signals. An affinity column prepared using such a peptide can be used suitably for ligand screening. In addition, the partial peptides of the protein of the present invention can be used for preparing antibodies. The partial peptides of the present invention can be produced, for example, by using genetic engineering techniques, known peptide synthetic methods, or methods of digesting the protein of the present invention with an appropriate peptidase. The partial peptides of the present invention typically consist of 8 or more amino acid residues, preferably 12 or more amino acid residues (for example, 15 or more amino acid residues).

The protein of the present invention can be prepared as a recombinant protein or natural protein. The recombinant protein can be prepared, for example, as follows, by introducing a DNA encoding the protein of the present invention, which has been inserted in a vector, into an appropriate host cell and purifying the protein expressed in the transformant. On the other hand, the natural protein can be prepared, for example, by using the affinity column, in which an antibody against the protein of the present invention has been immobilized, as follows (*Current Protocols in Molecular Biology,* edit. Ausubel et al. (1987) Publish. John Wiley & Sons Section 16.1-16.19). The antibody to be used in the affinity purification may be a polyclonal or monoclonal antibody. Further, the protein of the present invention can be prepared by *in vitro* translation (see, for example, "On the fidelity of mRNA translation in the nuclease-treated rabbit reticulocyte lysate system. Dasso, M.C., Jackson, R. J. (1989) *NAR* 17:3129-3144"), or the like.

The present invention also provides DNAs encoding the above-mentioned proteins of the present invention. There is no limitation on the type of DNA of the present invention, as far as it can encode the protein of the present invention; comprising cDNA, genomic DNA, chemically synthesized DNA, etc. Further, when it encodes the protein of the present invention, a DNA having any nucleotide sequence based on the degeneration of genetic code is included. The DNA of the present invention can be isolated according to a standard method, such as the hybridization method using a DNA sequence encoding the GPRv protein (SEQ ID NOs: 5 to 8 and 22 to 26) or a partial sequence thereof as a probe or PCR method using primers synthesized based on these DNA sequence, as described above.

In addition, the present invention also provides a vector, in which the DNA of the present invention has been inserted. There is no limitation on the type of vector of the present invention, as far as it stably retains the inserted DNA. For example, when *E. coli* is used as a host, the preferable cloning vector is pBluescript vector (Stratagene) or the like. When the vector is used for the purpose of producing the protein of the present invention, an expression vector is especially useful. There is no limitation on the type of expression vector, as far as it direct the expression of the protein *in vitro*, in *E. coli,* in culture cells, in the living body, for example, pBEST (Promega) for *in vitro* expression; pET (Invitrogen) for in E. coli, ; pME18S-FL3 (GenBank Accession No. AB009864) for in culture cells; and, pME18S (*Mol Cell Biol.* 8:466-472(1988)) for in the living body of an organism are preferred vector. The insertion of the DNA of the present invention into a vector can be achieved according to a standard method, for example, by ligation using restriction enzyme sites (*Current protocols in Molecular Biology,* edit. Ausubel et al. (1987) Publish. John Wiley & Sons. Section 11.4-11.11).

Also, the present invention provides a transformant containing the DNA of the present invention or the vector of the present invention. There is no limitation on the type of host cell into which the vector of the present invention is to be introduced, and various types of host cells can be used depending on the purposes. Exemplary eukaryotic cells, in which the protein is to be expressed at high levels, include COS cell and CHO cell. The vector can be introduced into the host cell, by a known method such as, for example, calcium-phosphate precipitation method, electroporation method, (*Current protocols in Molecular Biology*, edit. Ausubel et al. (1987) Publish. John Wiley & Sons. Section 9.1-9.9), a method with lipofectamine (GIBCO-BRL), microinjection, and so forth.

The present invention also provides nucleotides comprising at least 15 nucleotide residues, which is complementary to the DNA encoding the protein of the present invention (DNA comprising any one of the nucleotide sequences of SEQ ID NOs: 5 to 8 and 22 to 26 or the complementary strand thereof). The term "complementary strand" means one strand complementary to the other strand of the two of double-stranded nucleic acid consisting of A:T (U in the case of RNA) and G:C nucleotide pairs. Further, the term "complementary" means not only being a perfect complementary sequence in a region of at least consecutive 15 nucleotide residues, but also nucleotide sequences with at least 70% homology, preferably at least 80%, more preferably 90%, further preferably 95% of homology or higher. The algorithm described herein can be used for determining homology. These nucleotides can be used as probes for detecting and isolating the DNA of the present invention, and as primers for amplifying the DNA of the present invention. When used as the primer, it typically comprises 15 bp -100 bp, preferably of 15 bp -35bp of nucleotides. Alternatively, when used as the probe, it is at least 15 bp of nucleotide containing at least a part of the DNA of the present invention or the entire sequence. Preferably, such nucleotide specifically hybridize to the DNA encoding the protein of the present invention. The term "specifically hybridizing" means that a DNA hybridizes to the DNA encoding the protein of the present invention (SEQ ID NOs: 5 to 8 and 22 to 26) but not to DNAs encoding other proteins, under typical hybridization conditions, preferably under stringent conditions.

These nucleotides can be used for testing and diagnosing abnormalities of the protein of the present invention. For example, abnormal expression of the DNA encoding the protein of the present invention can be tested by Northern hybridization or RT-PCR using these nucleotides as probes or primers. The nucleotides can be used, for example, in the tests for cancers, cirrhosis, or Alzheimer's disease. In addition, the DNA encoding the protein of the present invention or the regulatory region for the expression is amplified by polymerase chain reaction (PCR) using the nucleotides as primers, and then abnormalities in the DNA sequence can be tested and diagnosed by using the methods such as RFLP analysis, SSCP, and sequencing.

Moreover, the antisense DNA for suppressing expression of the protein of present invention is included in these nucleotides. In order to cause the antisense effect, antisense DNA comprises at least 15 bp of nucleotides or more, preferably 100 bp, more preferably 500 bp or more, and usually comprises 3000 bp or less, preferably 2000 bp or less. Such antisense DNA may be applied to the gene therapy for the disease resulting from the abnormalities (abnormalities of function or expression) of the protein of present invention and so forth. This antisense DNA can be prepared, for example, based on the sequence information of DNA (for example, from SEQ ID NO: 5 to 8 and 22 to 26) encoding the protein of the present invention, by the phosphorothioate method (Stein, 1988 Physicochemical properties of phosphorothioate oligodeoxynucleotides. Nucleic Acids Res 16, 3209-21 (1988)), etc.

For gene therapy, the nucleotide of a present invention can be administered to a patient by ex vivo method, in vivo method, and so forth using virus vectors, such as a retrovirus vector, an adenovirus vector, and an adeno associated virus vector, and non-virus vectors, such as liposome, etc.

Further, the present invention provides the antibody bound with the protein of the present invention. There is no limitation in the form of the antibody of the present invention, and a polyclonal antibody and a monoclonal antibody, or a part thereof having antigen affinity are also included. Moreover, the antibody of all classes is included. Furthermore, the antibody of a present invention also include special antibodies, such as a humanized antibody.

For a polyclonal antibody, the antibody of the present invention can be obtained by synthesizing oligopeptides corresponding to the amino acid sequence of the protein of the present invention according to a standard, and then immunized to rabbit (Current protocols in Molecular Biology, edit. Ausubel et al. (1987) Publish. John Wiley & Sons.

Section 11.12-11.13). For a monoclonal antibody, the hybridoma cell prepared by the cell fusion of the spleen cell and myeloma cell of the mouse immunized using the protein expressing in E. coli and then purified according to the standard method, and the antibody of the present invention can be obtained from this hybridoma cell (Current protocols in Molecular Biology, edit. Ausubel et al. (1987) Publish. John Wiley & Sons. Section 11.4-11.11).

In addition to purifying of the protein of the present invention, the antibody bound with the protein of the present invention may be also used for a test and a diagnosis of the abnormalities in expression or in structure of the protein of a present invention. Specifically, protein can be extracted from tissue, blood, or cell, and then can be used for the test and the diagnose for presence or absence of the abnormalities of expression or structure, via a detection of the protein of the present invention by Western blotting method, immunoprecipitation, ELISA, and so forth. The antibody of the present invention may be used for a test of cancer, liver cirrhosis, or Alzheimer's disease.

Moreover, the antibody bound with the protein of the present invention may be used for the purposes of, such as treatment of the disease relevant to the protein of the present invention. The antibody of the present invention can effect as the agonist and antagonist for the protein of the present invention. When using an antibody for the purpose of treatment of a patient, an antibody derived from human or a humanized antibody is preferable because of little immunogenicity. An antibody derived from human can be prepared by immunizing the mouse of which immune system is replaced with those of human (for example, refer to "Functional transplant of megabase human immunoglobulin loci recapitulates human antibody response in mice" Mendez, M.J. et al. (1997) Nat. Genet. 15: 146-156). Moreover, a humanized antibody can be prepared by recombination with the hypervariable region of a monoclonal antibody (Methods in Enzymology 203, 99-121 (1991)).

Further, the present invention also provides a screening method for ligands binding to the protein of the present invention using the protein of the present invention. This screening method comprises the step of: (a) contacting a test sample with the protein of the present invention or a partial peptide thereof; and (b) selecting compounds binding to the protein or the partial peptide thereof.

Without limiting, the test sample Include, for example, known compounds or peptides (for example, deposited in the Chemical File) whose activities as the ligands to the various G protein-coupled receptors have not yet been identified or a group of random peptides which have been prepared by phage display method (*J. Mol. Biol.* (1991) 222, 301-310). Further, culture supernatants of microorganisms, natural ingredients from plants or marine organisms, and, in addition to these, biological extracts from tissues including brain, cell extracts, expression products of gene libraries, but not limited thereto, can be screened.

The protein of the present invention to be used for the screening can be, for example, the form displayed on cell surface, the form as the cell membrane fraction of the cells, or the form immobilized in an affinity column.

Specific screening methods include many known methods such as, for example, a method of contacting a test sample with an affinity column of the protein of the present invention and purifying compounds bound to the protein of the present invention; and Western blotting method. When these methods are used, the test sample is labeled appropriately and the binding with the protein of the present invention can be detected by using the label. In addition to these methods, another method can be used; in which cell membranes expressing the protein of the present invention are prepared and immobilized on a chip, and the alterations in surface plasmon resonance, which represent the dissociation of the trimer-type GTP-binding protein during the ligand binding, are detected (*Nature Biotechnology* (99) 17: 1105).

Further, the binding activity between a test sample and the protein of the present invention can be detected for alterations as indices in cells, which is caused by the binding of the test sample to the protein of the present invention expressed on cell surface. Such alterations include, for example, alterations of intracellular $Ca^{2+}$ level and cAMP levels, but not limited thereto. Specifically, the agonist activity to the G protein-coupled receptor can be assayed by GTPγS binding method.

In an example where this method is used, cell membranes on which the G protein-coupled receptor has been displayed are mixed with 400 pM $^{35}$S-labeled GTPγS in a solution containing 20 mM HEPES (pH 7.4), 100 mM NaCl, 10 mM $MgCl_2$, and 50 μM GDP, the mixture is incubated either in the presence or in absence of a test sample and then filtrated, and the radioactivities of the bound GTPγS are compared.

Further, the G protein-coupled receptors share the system of transducing signals into cells *via* the activation of the trimer-type GTP-binding protein. The trimer-type GTP-binding proteins are categorized into three classes depending on the types of activated systems of intracellular signal transduction: namely, Gq type that increases the $Ca^{2+}$ level; Gs type that increases the cAMP level; and Gi type that reduces the cAMP. Thus, the use of a chimeric protein consisting of α-subunit from Gq protein and α-subunit from another type of G protein, or promiscuous Gα proteins, Gα15 and Gα16, allows the positive signal in the ligand screening to result in increased $Ca^{2+}$ levels in the pathway of Gq intracellular signal transduction. The increased $Ca^{2+}$ levels can be detected by using, as indices, altered levels of a reporter gene having TRE (TPA responsive element) or MRE (multiple responsive element) on upstream, dye indicator such as Fura-2 and Fluo-3, and fluorescent protein aequorin. Similarly, the use of a chimeric protein consisting of α-subunit from Gs protein and α-subunit from another type of G protein allows the positive signal to result in increased cAMP levels in the pathway of Gs intracellular signal transduction, and the increased levels can be detected by using, as indices, altered levels of a reporter gene having CRE (cAMP-responsive element) on upstream (*Trends Pharmacol. Sci.* (99) 20:118).

There is no limitation on the type of host cell to be used for the expression of the protein of the present invention in this screening system, and various types of host cells can be used depending on the purposes. Such host cells include, for example, COS cell, CHO cell, HEK293 cell, etc. The vectors directing the expression of the protein of the present invention in vertebrate cells, comprising the promoter upstream of the gene encoding the protein of the present invention, RNA splice site, polyadenylation site, and transcription termination sequence and replication origin, and so forth can be preferably used. For example, pSV2dhfr (*Mol. Cell. Biol.* (1981) 1, 854-864), pEF-BOS (*Nucleic Acids Res.* (1990) 18, 5322), pCDM8 (*Nature* (1987) 329, 840-842), and pCEP4 (Invitrogen), containing the SV40 early promoter, are useful vectors for the expression of the G protein-coupled receptor. The insertion of the DNA of the present invention into a vector can be achieved according to a standard method by ligation using restriction enzyme sites (*Current protocols in Molecular Biology*, edit. Ausubel et al. (1987) Publish. John Wiley & Sons. Section 11.4-11.11). Further, the vector introduction into a host cell can be achieved by a known method, for example, such as calcium-phosphate precipitation method, electroporation method (*Current protocols in Molecular Biology*, edit. Ausubel et al. (1987) Publish. John Wiley & Sons. Section 9.1-9.9), a method with lipofectamine (GIBCO-BRL), a method with FuGENE6 reagent (Boehringer-Manheim), microinjection method, etc.

Once the ligands are isolated by the above screening method for ligands binding to the protein of the present invention, screening of compounds inhibiting the interaction between the protein of the present invention and the ligands can be achieved. Thus, the present invention provides a screening method for compounds having the activity of inhibiting the binding of the protein of the present invention and the ligand thereof. This screening method comprises the step of: (a) contacting the ligand with the protein of the present invention or a partial peptide thereof in the presence of a test sample, and detecting the binding activity of the protein or a partial peptide thereof with the ligand; and (b) selecting compounds reducing the binding activity detected in the step (a) relative to the binding activity in the absence of the test sample.

Without limiting, the test sample include, for example, a group of compounds obtained by combinatorial chemistry technology (Tetrahedron (1995) 51, 8135-8137), a group of random peptides prepared by phage display method (*J. Mol. Biol.* (1991) 222, 301-310), and such. Further, culture supernatants of microorganisms and natural ingredients from plants or marine organisms, and in addition to these, biological extracts from tissues including brain, cell extracts, expression products of gene libraries, synthetic low-molecular-weight compounds, synthetic peptides, natural compounds, and so forth can be screened, but not limited thereto.

The protein of the present invention to be used for the screening can be, for example, the form expressed on cell surface, the form in the cell membrane fraction of the cells, or the form immobilized in an affinity column.

Specific methods that can be used for the screening include, for example, a method in which the ligand is labeled with a radioisotope or the like, and contacted with the protein of the present invention in the presence of a test sample, and then, based on the label linked to the ligand, compounds reducing the binding activity of the protein of the present invention to the ligand are detected as compared to those detected in the absence of the test sample. Further, the screening can also be achieved by using the intracellular alterations as an index by the same method as used in the above-mentioned screening to isolate ligands capable of binding to the protein of the present invention. Specifically, the screening for a compound inhibiting the binding of the protein of the present invention with the ligand can be carried out by contacting cells expressing the protein of the present invention with the ligands in the presence of a test sample, and selecting a compound decreasing the degree of alteration in the cells as compared with those detected in the absence of the test sample. The cells expressing the protein of the present invention can be prepared by the same method as used in the above-described screening of ligands binding to the protein of the present invention. The compounds isolated by the screening can be candidates for the agonist or antagonist to the protein of the present.

Further, the present invention provides a screening method for compounds inhibiting or enhancing the activity of the protein of the present invention. This screening method comprises the step of: (a) contacting the ligand to the protein with cells expressing the protein of the present invention in the presence of a test sample; (b) detecting an alteration in the cells due to the binding of the ligand to the protein of the present invention; and (c) selecting compounds suppressing or enhancing the alteration in the cells detected in the step (b) as compared with the alteration of the cells in the absence of the test sample.

Such test samples to be used, like those to be used in the above-mentioned screening method for ligands binding to the protein of the present invention, include a group of compounds obtained by combinatorial chemistry technology, a group of random peptides prepared by using phage display method, culture supernatants of microorganisms, natural ingredients from plants or marine organisms, biological tissue extracts, cell extracts, expression products of gene libraries, synthetic low-molecular-weight compounds, synthetic peptides, natural compounds, and such. Further, the compounds isolated by the above-mentioned screening of ligands binding to the protein of the present invention can be used as the test samples. The cells expressing the protein of the present invention can be prepared by the same method as the above-described screening of ligands binding to the protein of the present invention. The alteration in the cells after contacted with the test sample can be detected by using the alteration of intracellular $Ca^{2+}$ level or cAMP level as an index, as with the above screening method. Further, the intracellular signal transduction can also be detected by using an assay system such as a reporter assay using luciferase as a reporter gene.

When the result of the detection shows that the alteration in the cells contacted with a test sample is suppressed as compared to those in the cells contacted with the ligand in the absence of the test sample, the test sample used is determined to be a compound inhibiting the activity of the protein of the present invention. Conversely, when the test sample enhances the alteration in the cells, the compound is determined to be a compound enhancing the activity of the protein of the present invention. The term "enhancing or inhibiting the activity of protein of the present invention" means that, regardless of a direct or an indirect interaction to the protein of the present invention, the interaction results in the enhancement or inhibition of the activity of protein of the present invention. Accordingly, the compounds isolated by the screening include compounds acting on the protein of the present invention or the ligand and inhibiting or enhancing the activity of the protein of the present invention by inhibiting or enhancing the binding, as well as compounds which do not inhibit nor enhance the binding itself but result in the inhibition or enhancement of the activity of the protein of the present invention. Such compounds include, for example, compounds which do not inhibit nor enhance the binding of the protein of the present invention and the ligand but inhibit or enhance the pathway of intracellular signal transduction.

When the compounds isolated by the screening method of the present invention are used as pharmaceuticals, the isolated compound not only can be directly administered itself to patients but also can be administered as pharmaceutical compositions which have been formulated by a known pharmaceutical method. For example, the compound can be formulated, in a form suitable for oral or parenteral administration, as a pharmaceutical composition obtained by combining the compound with pharmaceutically acceptable carrier (for example, excipient, binder, disintegrator, flavor, corrigent, emulsifier, diluent, solubilizer, etc.), or preparations, such as tablet, pill, powder, granule, capsule, troche, syrup, liquid drug, emulsion, suspension, injection (e.g. liquid drug and suspension), suppository, inhalant, percutaneous absorbent, eye drop, eye ointment, and so forth,. In general, the administration to patients can be carried out by a method known to those skilled in the art, including intraarterial injection, intravenous injection, subcutaneous injection, etc. While the doses are different depending on the weight and age of patient, administration method, and such, those skilled in the art can chose proper administration doses if necessary. Further, when the compound is encoded by a DNA, the DNA can be inserted into a vector for gene therapy and thus can be used for gene therapy. The compound isolated by the screening method of the present invention is expected to be applied to the treatment of, for example, cancers, cirrhosis, and Alzheimer's disease.

The present invention also provides a disease diagnosing method for cancers, cirrhosis, or Alzheimer's disease, comprising the step of detecting the expression of the gene encoding the GPRv protein of the present invention.

In the Example herein, it has been found that the expression levels of the genes encoding the GPRv proteins of the present invention in affected tissues associated with cancers, cirrhosis, or Alzheimer's disease are significantly different as compared to those in normal tissues. Thus, these diseases can be diagnosed by detecting the expression of the genes encoding the GPRv proteins of the present invention in tissues of subjects. The term "gene expression" means both transcription and translation.

The diagnosis method of the present invention can be carried out, for example, as follows.

The diagnosis can be achieved by extracting RNA from an aliquot of a tissue collected by biopsy or blood sample according to a standard method, and quantifying GPRv mRNA by quantitative PCR, Northern hybridization, or dot blot hybridization, and such, as described in the Example herein. Alternatively, the diagnosis can also be achieved by quantifying the GPRv protein in a protein extract from the above tissue by a method such as Western blotting, immunoprecipitation, ELISA, and such, or by a noninvasive method where a labeled compound or antibody binding to the GPRv protein is administered to patients and detected by PET (positron emission tomography) or the like.

When the result of the diagnosis shows that the gene expression in the tissues of a subject exhibits a pattern (for example, an increased or decreased gene expression level as compared to that in the normal tissue) identical to that of the gene expression in the tissue derived from a patient affected with any one of the above diseases, the subject is determined as being affected or as being at a risk of affection with the disease.

For example, the expression of GPRv8 was detectable in the colon, and the expression level was markedly higher in colon cancers. Accordingly, when the expression of GPRv8 is detected at a high level in the colon tissue of a subject, the subject is suspected of colon cancer. Alternatively, the expression of GPRv8 was undetectable in the normal pancreas and uterus, but GPRv8 was expressed at a moderate level after canceration. Accordingly, when the expression of GPRv8 can be detected in the pancreas or uterus of a subject, the subject is suspected of pancreatic cancer or uterine cancer.

The expression of GPRv12 was undetectable in the normal ovary and testis, but was detectable after canceration. Further, the expression level decreased in the hippocampus with Alzheimer's disease. Accordingly, when the expression of GPRv12 is detected in the ovary or testis of a subject, the subject is suspected of ovary cancer or testicular cancer. Similarly, when the expression of GPRv12 is detected in the hippocampus of a subject at a lower level than the normal level, the subject is suspected of Alzheimer's disease.

GPRv16 was expressed in the colon, but was undetectable after canceration. The expression level increased in the brain after canceration. In the liver, the expression was undetectable after cirrhosis. In the brain of patients with Alzheimer's disease, the expression level was elevated at the hippocampus. Accordingly, when the expression of GPRv16 is detected in the colon of a subject at a lower level than the normal level, the subject is suspected of colon cancer. Further, when the expression is detected in the brain at a higher level than the normal level, the subject is suspected of brain cancer. Further, the expression of GPRv16 is detected in the liver at a lower level than the normal level, the subject is suspected of cirrhosis. Further, when the expression of GPRv16 is detected in the hippocampus at a higher level than the normal level, the subject is suspected of Alzheimer's disease.

The expression of GPRv21 was undetectable in the colon and testis after canceration. Accordingly, when the expression of GPRv21 is detected in the colon or testis of a subject at a lower level than the normal level, the subject is suspected of colon cancer or testicular cancer.

The expression level of GPRv40 increased in the brain and testis after canceration, and decreased in the liver after cirrhosis. Accordingly, when the expression of GPRv40 is detected in the brain or testis at a higher level than the normal level, the subject is suspected of brain tumor or testicular cancer. Further, when the expression of GPRv40 was detected in the liver at a lower level than the normal level, the subject is suspected of cirrhosis.

The expression level of GPRv47 increased in the brain and kidney and decreased in the testis, after canceration. The expression was undetectable in the liver after cirrhosis. Accordingly, when the expression of GPRv47 is detected in the brain or kidney at a higher level than the normal level, the subject is suspected of brain tumor or kidney cancer. Further, when the expression of GPRv47 is detected in the liver at a lower level than the normal level, the subject is suspected of cirrhosis.

The expression level of GPRv51 decreased in the colon and testis after canceration. The expression level also decreased in the liver after cirrhosis as compared to the normal liver. The expression level increased in the hippocampus with Alzheimer's disease. Accordingly, when the expression of GPRv51 is detected in the colon and testis at a lower level than the normal level, the subject is suspected of colon cancer or testicular cancer. Further, when the expression of GPRv51 is detected in the liver at a lower level than the normal level, the subject is suspected of cirrhosis. Further, when the expression of GPRv51 is detected in the hippocampus at a higher level than the normal level, the subject is suspected of Alzheimer's disease.

The expression level of GPRv71 decreased in the colon and kidney, and was undetectable in the liver, after cirrhosis. In Alzheimer's disease, the expression level decreased in the frontal lobe. Accordingly, when the expression of GPRv71 is detected in the colon or kidney at a lower level than the normal level, the subject is suspected of colon cancer or kidney cancer. Further, when the expression of GPRv71 is detected in the liver at a lower level than the normal level, the subject is suspected of cirrhosis. Further, when the expression of GPRv71 is detected in the frontal lobe at a lower level than the normal level, the subject is suspected of Alzheimer's disease.

GPRv72 was expressed strongly in the colon, but the expression was undetectable after canceration. The expression level of GPRv72 increased in the hippocampus with Alzheimer's disease. Accordingly, when the expression of GPRv72 is detected in the colon at a lower level than the normal level, the subject is suspected of colon cancer. Further, when the expression of GPRv72 is detected in the hippocampus at a higher level than the normal level, the subject is suspected of Alzheimer's disease.

Furthermore, mutations in the genes encoding GPRv proteins of the present invention may result in the onset of the above-mentioned diseases. Thus, the diagnosis for the above-mentioned diseases can be carried out by detecting such mutations in the genes encoding GPRv proteins of the present invention.

Such gene diagnosis can be carried out, for example, as follows.

As a nucleic acid to be used for the diagnosis, genomic DNA or cDNA may be amplified directly or by PCR or other amplification technique. Deletions and insertions can be detected based on size differences of the amplification products as compared with that of the normal gene. Point mutations can be identified based on the differences in the melting temperature of the amplified DNA hybridized with DNA encoding GPRv. Differences between DNA sequences can be found by detecting alterations in the electrophoretic mobility of DNA fragment in a denaturant-containing or denaturant-free gel or by direct sequencing of nucleotide sequence of DNA.

When the diagnosis result shows that the gene encoding the GPRv protein from a subject has mutations as compared with the wild-type sequence, the subject diagnosed to be suspected of the above disease.

Namely, a method for diagnosing cancers, cirrhosis, or Alzheimer's disease or a method for diagnosing the susceptibility to the diseases are provided by detecting, according to the method described herein, mutations in the genes encoding the GPRv proteins or increase or decrease in the expression levels of the mRNAs or proteins.

Brief Description of the Drawings

Figure 1 shows a result of BLAST SEARCH with the "GPRv8" amino acid sequence as the query against the entire sequence data in SWISS-PROT. The sequence showed 36% homology to HUMAN VASOPRESSIN V1B RECEPTOR.

Figure 2 shows a result of BLAST SEARCH with the "GPRv12" amino acid sequence as the query against the entire sequence data in SWISS-PROT. The sequence showed 27% homology to RAT 5-HYDROXYTRYPTAMINE 6 RECEPTOR.

Figure 3 shows a result of BLAST SEARCH with the "GPRv16" amino acid sequence as the query against the entire sequence data in SWISS-PROT. The sequence showed 28% homology to MOUSE GALANIN RECEPTOR TYPE 1.

Figure 4 shows a result of BLAST SEARCH with the "GPRv21" amino acid sequence as the query against the entire sequence data in SWISS-PROT. The sequence showed 30% homology to BOVIN NEUROPEPTIDE Y RECEPTOR TYPE 2.

Figure 5 shows a result of BLAST SEARCH with the "GPRv40" amino acid sequence as the query against the entire sequence data in SWISS-PROT. The sequence showed 34% homology to OXYTOCIN RECEPTOR (P97926).

Figure 6 shows a result of BLAST SEARCH with the "GPRv47" amino acid sequence as the query against the entire sequence data in SWISS-PROT. The sequence showed 43% homology to GPRX_ORYLA PROBABLE G PROTEIN-COUPLED RECEPTOR (Q91178).

Figure 7 shows a result of BLAST SEARCH with the "GPRv51" amino acid sequence as the query against the entire sequence data in SWISS-PROT. The sequence showed 37% homology to PROBABLE G PROTEIN-COUPLED RECEPTOR RTA (P23749).

Figure 8 shows a result of BLAST SEARCH with the "GPRv71" amino acid sequence as the query against the entire sequence data in SWISS-PROT. The sequence showed 45% homology to P2Y PURINOCEPTOR 3 (P2Y3) (Q98907).

Figure 9 shows a result of BLAST SEARCH with the "GPRv72" amino acid sequence as the query against the entire sequence data in SWISS-PROT. The sequence showed 30% homology to ALPHA-1A ADRENERGIC RECEPTOR (002824).

Figure 10 shows a hydropathy plot for GPRv8.

Figure 11 shows an alignment of GPRv8 and similar families. The mark '*' means that the amino acid is completely conserved in all the sequences at the position marked therewith. The mark ':' means that amino acids at the position marked therewith are conserved within any one of the following groups: {STA}, {NEQK}, {NHQK}, {NDBQ}, {QHRK}, {MILV}, {MILF}, {HY}, and {FYW}. The mark '.' means that amino acids at the position marked therewith are conserved within any one of the following groups: {CSA}, {ATV}, {SAG}, {STNK}, {STPA}, {SGND}, {SNDEQK}, {NDEQHK}, and {NEQHRK}.

Figure 12 is continued from Figure 11.

Figure 13 shows a hydropathy plot for GPRv12.

Figure 14 shows an amino acid sequence alignment of GPRv12 and AF208288. The mark '*' means that the amino acid is completely conserved in all the sequences at the position marked therewith. The mark ':' means that amino acids at the position marked therewith are conserved within any one of the following groups: {STA}, {NEQK}, {NHQK}, {NDBQ}, {QHRK}, {MILV}, {MILF}, {HY}, and {FYW}. The mark '.' means that amino acids at the position marked therewith are conserved within any one of the following groups: {CSA}, {ATV}, {SAG}, (STNK), {STPA}, {SGND}, {SNDEQK}, {NDEQHK}, and {NEQHRK}.

Figure 15 shows a hydropathy plot for GPRv16.

Figure 16 shows a summary of HMMPFAM, transmembrane domain, and S-S bond of GPRv16. The mark "***" indicates a region assigned as 7tm_1 based on the result of HMMPFAM. The mark "###" represents transmembrane domain. The mark "@" indicates Cys capable of forming S-S bond.

Figure 17 shows a hydropathy plot for GPRv21.

Figure 18 shows an amino acid sequence alignment of GPRv21 and the related proteins. The mark '*' means that the amino acid is completely conserved in all the sequences at the position marked therewith. The mark ':' means that amino acids at the position marked therewith are conserved within any one of the following groups: {STA}, {NEQK}, {NHQK}, {NDBQ}, {QHRK}, {MILV}, {MILF}, {HY}, and {FYW}. The mark '.' means that amino acids at the position marked therewith are conserved within any one of the following groups: (CSA), {ATV}, (SAG), {STNK}, {STPA}, (SGND), {SNDEQK), {NDEQHK), and {NEQHRK).

Figure 19 is continued from Figure 18.

Figure 20 shows a hydropathy plot for GPRv40.

Figure 21 shows a summary of HMMPFAM, transmembrane domain, and S-S bond of GPRv40. The mark "***" indicates a region assigned as 7tm_1 based on the result of HMMPFAM. The mark "###" indicates transmembrane domain. The mark "@" indicates Cys capable of forming S-S bond.

Figure 22 shows a hydropathy plot for GPRv47.

Figure 23 shows an alignment of GPRv47 and the related proteins. The mark '*' means that the amino acid is completely conserved in all the sequences at the position marked therewith. The mark ':' means that amino acids at the position marked therewith are conserved within any one of the following groups: {STA}, {NEQK}, {NHQK}, {NDBQ}, {QHRK}, {MILV}, {MILF}, {HY}, and {FYW}. The mark '.' means that amino acids at the position marked therewith are conserved within any one of the following groups: {CSA}, {ATV}, {SAG}, {STNK), {STPA}, {SGND}, {SNDEQK}, {NDEQHK}, and {NEQHRK}.

Figure 24 is continued from Figure 23.

Figure 25 is continued from Figure 24.

Figure 26 shows a hydropathy plot for GPRv51.

Figure 27 shows an alignment of GPRv51 and the related proteins. The mark '*' means that the amino acid is completely conserved in all the sequences at the position marked therewith. The mark ':' means that amino acid at the position marked therewith are conserved within any one of the following groups: {STA}, {NEQK}, {NHQK}, {NDBQ}, {QHRK}, {MILV}, {MILF}, {HY}, and {FYW}. The mark '.' means that amino acid at the position marked therewith are conserved within any one of the following groups: {CSA}, {ATV}, {SAG}, {STNK}, {STPA}, {SGND}, {SNDEQK}, {NDEQHK}, and {NEQHRK}.

Figure 28 shows a hydropathy plot for GPRv71.

Figure 29 shows an alignment of GPRv71 and related proteins. The mark '*' means that the amino acid is completely conserved in all the sequences at the position marked therewith. The mark ':' means that amino acid at the position marked therewith are conserved within any one of the following groups: {STA}, {NEQK}, {NHQK}, {NDBQ}, {QHRK}, {MILV}, {MILF}, {HY}, and {FYW}. The mark '.' means that amino acid at the position marked therewith are conserved within any one of the following groups : {CSA}, {ATV}, {SAG}, {STNK}, {STPA}, {SGND}, {SNDEQK}, {NDEQHK}, and {NEQHRK}.

Figure 30 is continued from Figure 29.

Figure 31 shows a hydropathy plot for GPRv72.

Figure 32 shows an alignment of GPRv72 and related proteins. The mark '*' means that the amino acid is completely conserved in all the sequences at the position marked therewith. The mark ':' means that amino acid at the position marked therewith are conserved within any one of the following groups: {STA}, {NEQK}, {NHQK}, {NDBQ}, {QHRK}, {MILV}, {MILF}, {HY}, and {FYW}. The mark '.' means that amino acid at the position marked therewith are conserved within any one of the following groups: {CSA}, {ATV}, {SAG}, {STNK}, {STPA}, {SGND}, {SNDEQK}, {NDEQHK}, and {NEQHRK}.

Figure 33 is continued from Figure 32.

Figure 34 is continued from Figure 33.

## Best Mode for Carrying out the Invention

The present invention is specifically illustrated below with reference to Examples, but it is not to be construed as being limited thereto. Unless otherwise stated, they can be carried out by known methods (Maniatis, T. et al. (1982): "Molecular Cloning - A Laboratory Manual", Cold Spring Harbor Laboratory, NY).

[Example 1] Isolation of the genes encoding the novel G protein-coupled receptors

The full-length cDNAs encoding the novel G protein-coupled receptors of the present invention (GPRv8, GPRv12, GPRv16, GPRv21, GPRv40, GPRv47, GPRv51, GPRv71, and GPRv72) were obtained by PCR.

The amplification of the novel G protein-coupled receptor GPRv8 was carried out using a Marathon Ready cDNA (Clontech) derived from human fetus as a template, and forward primer: 5'-ATGCCAGCCAACTTCACAGAGGGCAGCT-3' (SEQ ID NO: 9) and reverse primer: 5'-CTAGATGAATTCTGGCTTGGACAGAATC-3' (SEQ ID NO: 10). PCR was carried out with Pyrobest DNA polymerase (Takara); the thermal cycling profile consisted of preheat at 94°C (2.5 minutes) and 25 cycles of 94°C (30 seconds)/60°C (30 seconds)/72°C (1 minute). The amplification resulted in about 1.1-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems). The determined sequence is shown in SEQ ID NO: 5.

The sequence comprises an open reading frame of 1116 nucleotides (from the first nucleotide to the 1116th nucleotide in SEQ ID NO: 5). An amino acid sequence deduced from the open reading frame (371 amino acids) is shown in SEQ ID NO: 1. Since the deduced amino acid sequence contains hydrophobic regions corresponding to seven transmembrane domains characteristic of G protein-coupled receptor, the gene is found to encode a G protein-coupled receptor.

The amplification of the novel G protein-coupled receptor GPRv12 was carried out using a Marathon Ready cDNA (Clontech) derived from human fetal brain as a template, and forward primer: 5'-ATGGGCCCCGGCGAGGCGCTGCTGGCGG-3' (SEQ ID NO: 11) and reverse primer: 5'-TCAGTGTGTCTGCTGCAGGCAGGAATCA-3' (SEQ ID NO: 12). PCR was carried out with Pyrobest DNA polymerase (Takara) under the presence of 5% formamide; the thermal cycling profile consisted of preheat at 94°C (2.5 minutes), 5 cycles of 94°C (5 seconds)/72°C (4 minutes) , 5 cycles of 94°C (5 seconds)/70°C (4 minutes) , and 25 cycles of 94°C (5 seconds)/68°C (4 minutes). The amplification resulted in about 1.1-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems). The determined sequence is shown in SEQ ID NO: 6.

The sequence comprises an open reading frame of 1092 nucleotides (from the first nucleotide to the 1092th nucleotide in. SEQ ID NO: 6). An amino acid sequence deduced from the open reading frame (363 amino acids) is shown in SEQ ID NO: 2. Since the deduced amino acid sequence contains hydrophobic regions corresponding to seven transmembrane domains characteristic of G protein-coupled receptor, the gene is found to encode a G protein-coupled receptor.

The amplification of the novel G protein-coupled receptor GPRv16 was carried out using a Marathon Ready cDNA (Clontech) derived from human brain as a template, and forward primer: 5'-ATGCTGGCAGCTGCCTTTGCAGACTCTAAC-3' (SEQ ID NO: 13) and reverse primer: 5'-CTATTTAACACCTTCCCCTGTCTCTTGATC-3' (SEQ ID NO: 14). PCR was carried out with Pyrobest DNA polymerase (Takara); the thermal cycling profile consisted of preheat at 94°C (2 minutes) and 30 cycles of 94°C (30 seconds)/60°C (30 seconds)/72°C (1 minute). The amplification resulted in about 1.2-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems) . The determined sequence is shown in SEQ ID NO: 7.

The sequence comprises an open reading frame of 1260 nucleotides (from the first nucleotide to the 1260th nucleotide in SEQ ID NO: 7). An amino acid sequence deduced from the open reading frame (419 amino acids) is shown in SEQ ID NO: 3. Since the deduced amino acid sequence contains hydrophobic regions corresponding to seven transmembrane domains characteristic of G protein-coupled receptor, the gene is found to encode a G protein-coupled receptor.

The amplification of the novel G protein-coupled receptor GPRv21 was carried out using a Marathon Ready cDNA (Clontech) derived from human fetus as a template, and forward primer: 5'-ATGGAGACCACCATGGGGTTCATGGATG-3' (SEQ ID NO: 15) and reverse primer: 5'-TTATTTTAGTCTGATGCAGTCCACCTCTTC-3' (SEQ ID NO: 16). PCR was carried out with Pyrobest DNA polymerase (Takara) under the presence of 5% formamide; the thermal cycling profile consisted of preheat at 94°C (2.5 minutes), 5 cycles of 94°C (5 seconds)/72°C (4 minutes), 5 cycles of 94°C (5 seconds)/70°C (4 minutes), and 25 cycles of 94°C (5 seconds)/68°C (4 minutes). The amplification resulted in about 1.2-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems). The determined sequence is shown in SEQ ID NO: 8.

The sequence comprises an open reading frame of 1182 nucleotides. An amino acid sequence deduced from the open reading frame (333 amino acids) is shown in SEQ ID NO: 4. Since the deduced amino acid sequence contains hydrophobic regions corresponding to seven transmembrane domains characteristic of G protein-coupled receptor, the gene is found to encode a G protein-coupled receptor.

The amplification of the novel G protein-coupled receptor GPRv40 was carried out using a Marathon Ready cDNA (Clontech) derived from human fetus as a template, and forward primer: 5'-ATGGAGGATCTCTTTAGCCCCTCAATTC-3' (SEQ ID NO: 27) and reverse primer: 5'-CTAGAAGGCACTTTCGCAGGAGCAAGGC-3' (SEQ ID NO: 28). PCR was carried out with Pyrobest DNA polymerase (Takara) under the presence of 5% formamide; the thermal cycling profile consisted of preheat at 98°C (2.5 minutes), 5 cycles of 98°C (5 seconds)/72°C (4 minutes), 5 cycles of 98°C (5 seconds)/70°C (4 minutes), and 25 cycles of 98°C (5 seconds)/68°C (4 minutes). The amplification resulted in about 1.3-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems). The determined sequence is shown in SEQ ID NO: 22.

The sequence comprises an open reading frame of 1305 nucleotides (SEQ ID NO: 22). An amino acid sequence deduced from the open reading frame (434 amino acids) is shown in SEQ ID NO: 17. Since the deduced amino acid sequence contains hydrophobic regions corresponding to seven transmembrane domains characteristic of G protein-coupled receptor, the gene is found to encode a G protein-coupled receptor.

The amplification of the novel G protein-coupled receptor GPRv47 was carried out using a Marathon Ready cDNA (Clontech) derived from human fetal brain as a template, and forward primer: 5'-ATGGAGTCCTCACCCATCCCCCAGTCATC-3' (SEQ ID NO: 29) and reverse primer: 5'-TCATGACTCCAGCCGGGGTGAGGCGGCAG-3' (SEQ ID NO: 30). PCR was carried out with Pyrobest DNA polymerase (Takara) under the presence of 5% formamide; the thermal cycling profile consisted of preheat at 94°C (2 minutes) and 35 cycles of 94°C (30 seconds)/50°C (30 seconds) /72°C (1.5 minutes). The amplification resulted in about 1.4-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems). The determined sequence is shown in SEQ ID NO: 23.

The sequence comprises an open reading frame of 1356 nucleotides (SEQ ID NO: 23). An amino acid sequence deduced from the open reading frame (451 amino acids) is shown in SEQ ID NO: 18. Since the deduced amino acid sequence contains hydrophobic regions corresponding to seven transmembrane domains characteristic of G protein-coupled receptor, the gene is found to encode a G protein-coupled receptor.

The amplification of the novel G protein-coupled receptor GPRv51 was carried out using a Marathon Ready cDNA (Clontech) derived from human testis as a template, and forward primer: 5'-ATGAACCAGACTTTGAATAGCAGTGG-3' (SEQ ID NO: 31) and reverse primer: 5'-TCAAGCCCCCATCTCATTGGTGCCCACG-3' (SEQ ID NO: 32). PCR was carried out with Pyrobest DNA polymerase (Takara); the thermal cycling profile consisted of preheat at 98°C (2.5 minutes) and 35 cycles of 98°C (30 seconds)/50°C (30 seconds)/68°C (4 minutes) . The amplification resulted in about 1.0-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems) . The determined sequence is shown in SEQ ID NO: 24.

The sequence comprises an open reading frame of 966 nucleotides (SEQ ID NO: 24). An amino acid sequence deduced from the open reading frame (321 amino acids) is shown in SEQ ID NO: 19. Since the deduced amino acid sequence contains hydrophobic regions corresponding to seven transmembrane domains characteristic of G protein-coupled receptor, the gene is found to encode a G protein-coupled receptor.

The amplification of the novel G protein-coupled receptor GPRv71 was carried out using a Marathon Ready cDNA (Clontech) derived from human fetus as a template, and forward primer: 5'-ATGGAGAAGGTGGACATGAATACATCAC-3' (SEQ ID NO: 33) and reverse primer: 5'-TTACCCAGATCTGTTCAACCCTGGGCATC-3' (SEQ ID NO: 34). PCR was carried out with Pyrobest DNA polymerase (Takara); the thermal cycling profile consisted of preheat at 94°C (2.5 minutes) , 5 cycles of 98°C (5 seconds)/72°C (4 minutes), 5 cycles of 98°C (5 seconds)/70°C (4 minutes), and 25 cycles of 98°C (5 seconds)/68°C (4 minutes). The amplification resulted in about 1.0-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems). The determined sequence is shown in SEQ ID NO: 25.

The sequence comprises an open reading frame of 1002 nucleotides (SEQ ID NO: 25). An amino acid sequence deduced from the open reading frame (333 amino acids) is shown in SEQ ID NO: 20. Since the deduced amino acid sequence contains hydrophobic regions corresponding to seven transmembrane domains characteristic of G protein-coupled receptor, the gene is found to encode a G protein-coupled receptor.

The amplification of the novel G protein-coupled receptor GPRv72 was carried out using human genome DNA (Clontech) as a template, and forward primer: 5'-ATGACGTCCACCTGCACCAACAGCACGC-3' (SEQ ID NO: 35) and reverse primer: 5'-TCAAGGAAAAGTAGCAGAATCGTAGGAAG-3' (SEQ ID NO: 36). PCR was carried out with Pyrobest DNA polymerase (Takara); the thermal cycling profile consisted of preheat at 94°C (2 minutes) and 30 cycles of 94°C (30 seconds)/55°C (30 seconds)/68°C (4 minutes) . The amplification resulted in about 1.5-kbp DNA fragments. The fragments were cloned into pCR2.1 plasmid (Invitrogen). The nucleotide sequence of the resultant clone was determined by dideoxy terminator method in an ABI377 DNA Sequencer (Applied Biosystems). The determined sequence is shown in SEQ ID NO: 26.

The sequence comprises an open reading frame of 1527 nucleotides (SEQ ID NO: 26). An amino acid sequence deduced from the open reading frame (508 amino acids) is shown in SEQ ID NO: 21. Since the deduced amino acid sequence contains hydrophobic regions corresponding to seven transmembrane domains characteristic of G protein-coupled receptor, the gene is found to encode a G protein-coupled receptor.

[Example 2] BLAST SEARCH of the amino acid sequences of the novel G protein-coupled receptors against SWISS-PROT

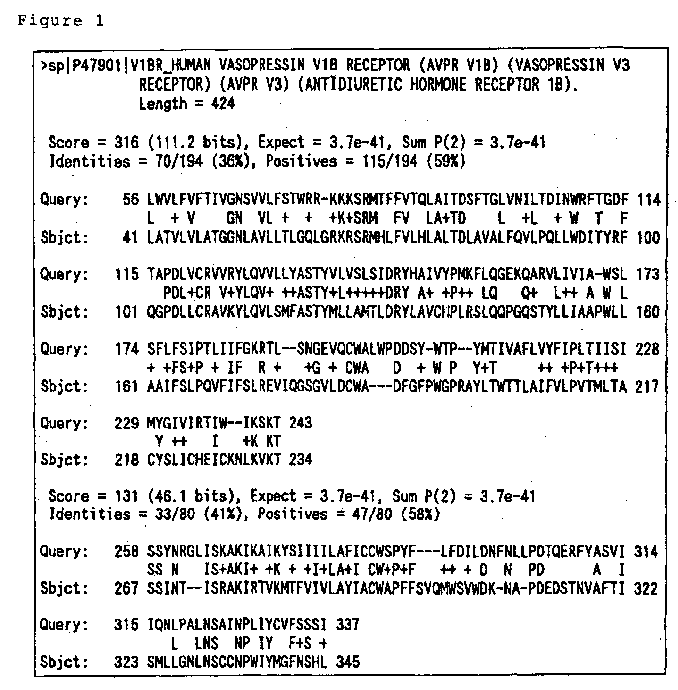

The result of BLAST SEARCH of the amino acid sequence of "GPRv8" against SWISS-PROT is shown in Figure 1. "GPRv8" exhibited the highest homology (36%) to HUMAN VASOPRESSIN V1B RECEPTOR (P47901, 424 aa) of known G protein-coupled receptors. Thus, "GPRv8" was concluded to be a novel G protein-coupled receptor.

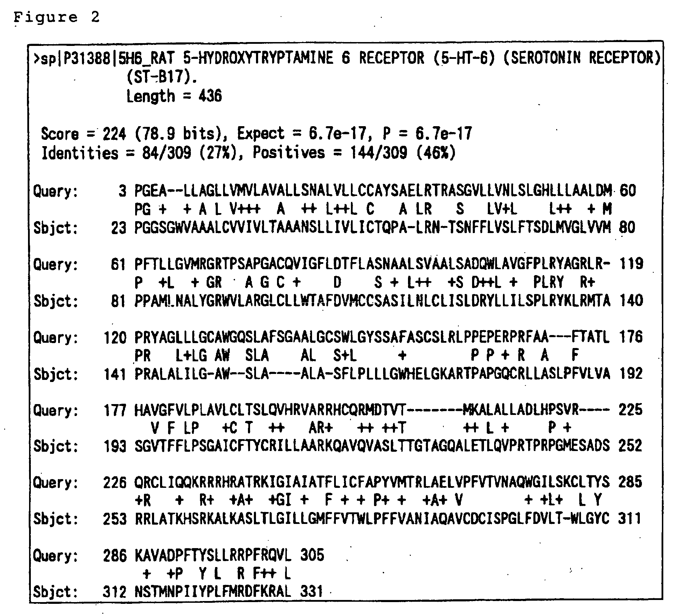

The result of BLAST SEARCH of the amino acid sequence of "GPRv12" against SWISS-PROT is shown in Figure 2. "GPRv12" exhibited the highest homology (27%) to RAT 5-HYDROXYTRYPTAMINE 6 RECEPTOR (P31388, 436 aa) of known G protein-coupled receptors. Thus, GPRv12 was concluded to be a novel G protein-coupled receptor.

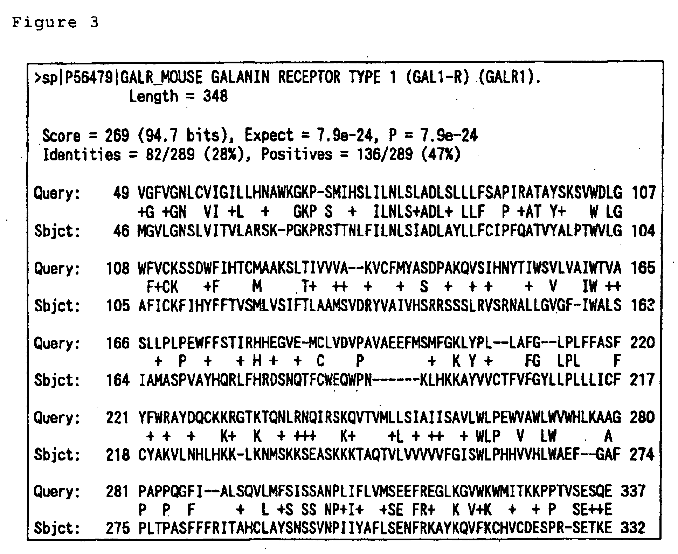

The result of BLAST SEARCH of the amino acid sequence of "GPRv16" against SWISS-PROT is shown in Figure 3. "GPRv16" exhibited the highest homology (28%) to MOUSE GALANIN RECEPTOR TYPE 1 (P56479, 348 aa) of known G protein-coupled receptors. Thus, "GPRv16" was concluded to be a novel G protein-coupled receptor.

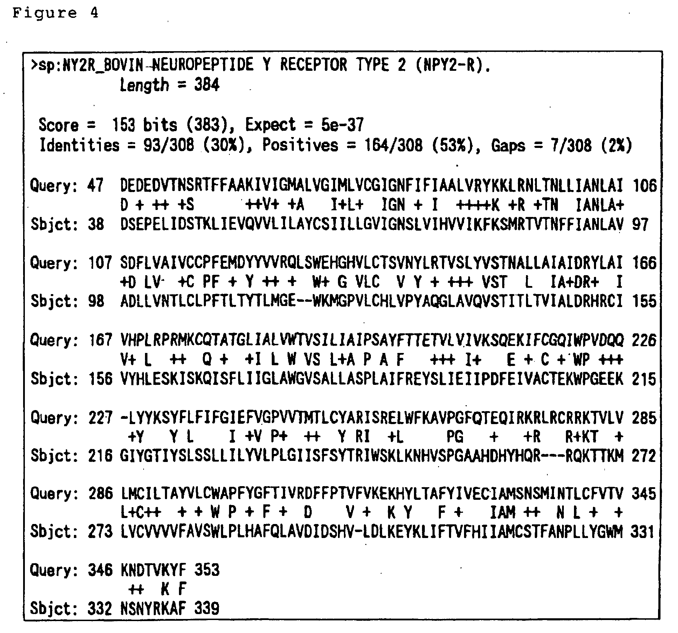

The result of BLAST SEARCH of the amino acid sequence of "GPRv21" against SWISS-PROT is shown in Figure 4. "GPRv21" exhibited the highest homology (30%) to BOVIN NEUROPEPTIDE Y RECEPTOR TYPE 2 (P79113, 384 aa) of known G protein-coupled receptors. Thus, "GPRv21" was concluded to be a novel G protein-coupled receptor.

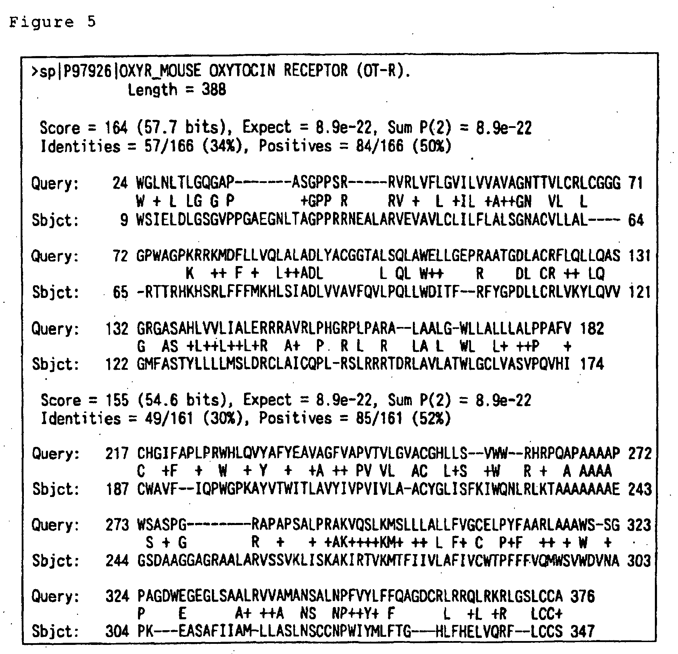

The result of BLAST SEARCH of the amino acid sequence of "GPRv40" against SWISS-PROT is shown in Figure 5. "GPRv40" was not identical to any of known G protein-coupled receptors, but exhibited the highest homology (34%) to OXYTOCIN RECEPTOR (P97926, 388 aa). Thus, "GPRv40" was concluded to be a novel G protein-coupled receptor.

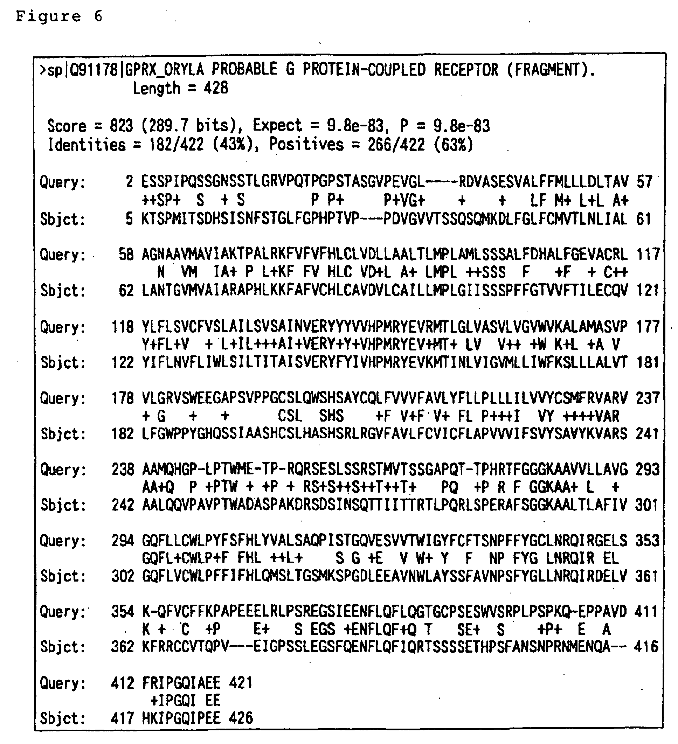

The result of BLAST SEARCH of the amino acid sequence of "GPRv47" against SWISS-PROT is shown in Figure 6. "GPRv47" was not identical to any of known G protein-coupled receptors, but exhibited the highest homology (43%) to GPRX_ORYLA PROBABLE G PROTEIN-COUPLED RECEPTOR (Q91178, 428 aa). Thus, "GPRv47" was concluded to be a novel G protein-coupled receptor.

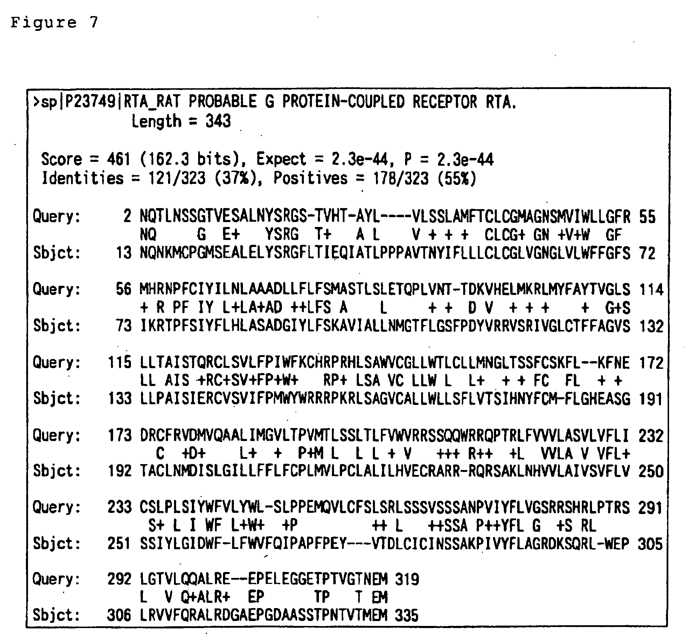

The result of BLAST SEARCH of the amino acid sequence of "GPRv51" against SWISS-PROT is shown in Figure 7. "GPRv51" was not identical to any of known G protein-coupled receptors, but exhibited the highest homology (37%) to PROBABLE G PROTEIN-COUPLED RECEPTOR RTA (P23749, 343 aa). Thus, "GPRv51" was concluded to be a novel G protein-coupled receptor.

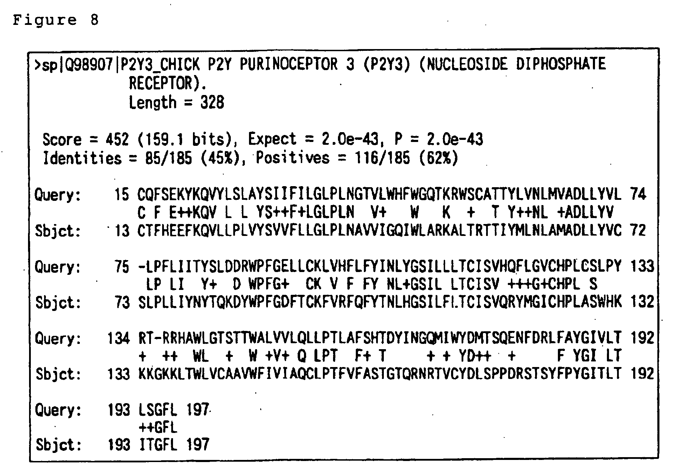

The result of BLAST SEARCH of the amino acid sequence of "GPRv71" against SWISS-PROT is shown in Figure 8. "GPRv71" was not identical to any of known G protein-coupled receptors, but exhibited the highest homology (45%) to Chicken P2Y PURINOCEPTOR 3 (P2Y3) (Q98907, 328 aa). Thus, "GPRv71" was concluded to be a novel G protein-coupled receptor.

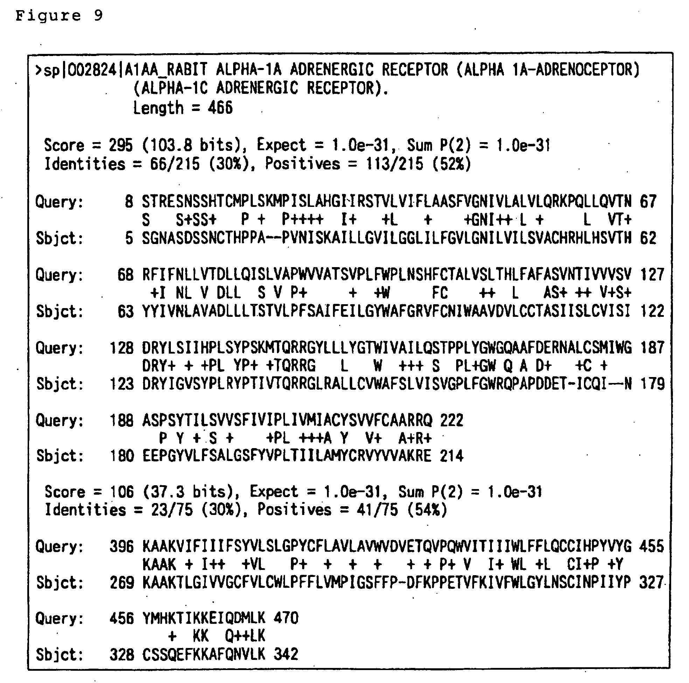

The result of BLAST SEARCH of the amino acid sequence of "GPRv72" against SWISS-PROT is shown in Figure 9. "GPRv72" was not identical to any of known G protein-coupled receptors, but exhibited the highest homology (30%) to ALPHA-1A ADRENERGIC RECEPTOR (002824, 466 aa). Thus, "GPRv72" was concluded to be a novel G protein-coupled receptor.

[Example 3] Analysis of tissue-specific expression

1. Reagents

1.1. Primers for quantitative polymerase chain reaction (PCR) and TaqMan probes:

Sense primers, antisense primers, and TaqMan probes were designed by using genetic analysis software "Primer Express version 1.0" from PE Biosystems. The ordinary custom-made primers and TaqMan probes were purchased from Amersham Pharmacia Biotech (Tokyo) and PE Biosystems Japan, respectively. The TaqMan probes were connected with a reporter pigment FAM at the 5' end and with a quencher Tamra at the 3' end. The nucleotide sequences of primers and TaqMan probes are shown below.

Synthetic DNA for GPRv8

PCR primer G8.957F: CCAGGAGCGTTTCTATGCCT (SEQ ID NO: 37)
G8.1082R: TGTGATCTTTGCTCCCTGCA (SEQ ID NO: 38)

TaqMan Probe GPRv8.987T: TCAGAACCTGCCAGCATTGAATAGTGCC (SEQ ID NO: 39)

Synthetic DNA for GPRv12

PCR primer G12.794F: ATCTGCTTTGCCCCGTATGT (SEQ ID NO: 40)
G12.903R: ACCGCCTTGCTGTAGGTCAG (SEQ ID NO: 41)

TaqMan Probe GPRv12.834T: TCGTGCCCTTCGTCACCGTGAA (SEQ ID NO : 42)

Synthetic DNA for GPRv16

PCR primer G16.1133F: CCCAGCATCCATACCAGAAAA (SEQ ID NO: 43)
G16.1254R: CTGTGTCCCTCTCATGCCAAA (SEQ ID NO: 44)

TaqMan Probe GPRv16.1193T: TGAGAAGGCAGAGATTCCCATCCTTCCT (SEQ ID NO: 45)

Synthetic DNA for GPRv21

PCR primer G21.989F: TCGCCATGAGCAACAGCAT (SEQ ID NO: 46)
G21.1114R: CACTGGACTTACCGCCATTGT (SEQ ID NO: 47)

TaqMan Probe GPRv21.1064T: AGATCATGTTGCTCCACTGGAAGGCTTCT (SEQ ID NO: 48)

Synthetic DNA for GPRv40

PCR primer G40.16F: GGATCTCTTTAGCCCCTCAATTC (SEQ ID NO: 49)
G40.99R: AAGGTCAGGTTGAGACCCCAG (SEQ ID NO: 50)

TaqMan Probe GPRv40.53T: AACATTTCCGTGCCCATCTTGCTGG (SEQ ID NO: 51)

Synthetic DNA for GPRv47

PCR primer G47.1292F: GCTGTTGACTTTCGAATCCCA (SEQ ID NO: 52)
G47.1393R: ACGGAGGTAGCTGTCTGACATGA (SEQ ID NO: 53)

TaqMan Probe GPRv47.1336T: TGAGTTCCTGGAGCAGCAACTCACCA (SEQ ID NO: 54)

Synthetic DNA for GPRv51

PCR primer G51.190F: GGCTTTCGAATGCACAGGAA (SEQ ID NO: 55)
G51.276R: GGAAGCCATGCTGAAGAGGA (SEQ ID NO: 56)

TaqMan Probe GPRv51.214T: TTCTGCATCTATATCCTCAACCTGGCGG (SEQ ID NO: 57)

Synthetic DNA for GPRv71

PCR primer G71.746F: TGGCCTCTTCACCCTCTGTTT (SEQ ID NO: 58)
G71.841R: ATCAAGAGCTGGCAGTCCTGA (SEQ ID NO: 59)

TaqMan Probe GPRv71.775T: TCCATATCACTCGCTCCTTCTACCTCACCA (SEQ ID NO: 60)

Synthetic DNA for GPRv72

PCR primer G72.101F: CCAAAATGCCCATCAGCCT (SEQ ID NO: 61)
G72.190R: GCACTATGTTGCCGACGAAA (SEQ ID NO: 62)

TaqMan Probe GPRv72.132T: CATCCGCTCAACCGTGCTGGTTATCT (SEQ ID NO: 63)

1.2. CDNA derived from patients

As cDNAs which had been derived from tumor and normal tissues from a single patient, Matched cDNA Pairs from Clontech were used. The tissues are lung, stomach, colon, ovary, prostate, uterus, and kidney.

Some cDNAs derived from following tissues were purchased from BioChain Institute: brain, pancreas, and testis from patients with tumor and normal adults; liver from cirrhosis patients and normal adults; kidney from lupus disease patients; and the hippocampus and frontal lobe from Alzheimer's disease (AD) patients and normal adults.

1.3. Reagents for quantitative PCR:

TaqMan Universal PCR Master Mix (PE Biosystems) was used in this assay. TaqMan β-actin Control Reagents (PE Biosystems) was used for measuring the internal standard.

2. Quantitative PCR:

1) Dilution of template cDNA

The cDNAs from BioChain were diluted 50 fold with water, and the cDNAs from Clontech were diluted 5 fold with water, for use.

2) Preparation of Master Mix

A reaction solution with the following composition was prepared.

| | Reaction volume | Preparation volume |
|---|---|---|
| 2x Master Mix | 12.5 µl | 1380 µl |
| Sense primer (50 µM) | 0.5 µl | 55.2 µl |
| Antisense primer (50 µM) | 0.5 µl | 55.2 µl |
| TaqMan Probe (5 µM) | 1 µl | 110.4 µl |
| Template cDNA | 2.5 µl | |
| Purified water | 8 µl | 883.2 µl |
| Total volume | 25 µl | 2484 µl |

3) Preparation of PCR solution

6 µl template cDNA solution was added to 54 µl Master Mix solution. Then, 25-µl aliquots of the mixture were added in duplicate to the sample wells of a PCR plate to be placed in a device for quantitative PCR. A 25-µl aliquot of the above-mentioned Master Mix was added to each of two wells for non-template control. The standard curve was produced using eight 10-fold serial dilutions of cDNA which had been subcloned into pCEP4 vector, where the dilution started from 100 pg/µl. A 25-µl aliquot of each mixture obtained by combining 54 µl of Master Mix prepared in Section 2) and 6 µl of each standard solution prepared above was added into a standard well. Namely, the largest amount of the plasmid DNA was 250 pg and the smallest was 25 ag (a: atto, $10^{-18}$) in the standard wells. After 8-cap strips were placed to the top of the wells, the bubbles were removed by light centrifugation.

4) PCR

The plate was placed in the device for quantitative PCR (GeneAmp 5700 Sequence Detection System: PE Biosystems), and then the reaction was carried out according to the following cycling program.

(1) 50°C, 2 minutes: 1 cycle
(2) 95°C, 10 minutes: 1 cycle
(3)

95°C, 15 seconds }: 50 cycles
60°C, 1 minutes }

5) Quantitative analysis

The quantification was carried out according to the operation manual of GeneAmp 5700, and the result was outputted.

3. Results and conclusions:

The GPCR expression profiles obtained with the cDNAs from the organs from normal human and those from patients with disease were represented as ratios relative to the expression level of the actin gene as an internal standard. The experiment was carried out in duplicate, and the average values are shown in Table 1.

Table 1

| | relative copy number | | | | | | | | |
|---|---|---|---|---|---|---|---|---|---|
| | GPRv8 | GPRv12 | GPRv16 | GPRv21 | GPRv40 | GPRv47 | GPRv51 | GPRv71 | GPRv72 |
| Brain Normal 1) | 0 | 0 | 1 | 0 | 6 | 9 | 0 | 0 | 0 |
| Tumor 1) | 5 | 2 | 11 | 0 | 23 | 76 | 2 | 5 | 0 |
| Lung Normal | 0 | 0 | 1 | 0 | 11 | 0 | 1 | 1 | 0 |
| Tumor | 1 | 0 | 1 | 0 | 11 | 2 | 1 | 1 | 1 |
| Stomach Normal | 6 | 0 | 0 | 0 | 29 | 0 | 1 | 1 | 0 |
| Tumor | 3 | 0 | 2 | 0 | 1 | 0 | 3 | 0 | 1 |
| Pancreas Normal 1) | 0 | 0 | 0 | 0 | 4 | 0 | 0 | 0 | 0 |
| Tumor 1) | 45 | 2 | 0 | 0 | 23 | 2 | 3 | 4 | 1 |
| Colon Normal | 141 | 0 | 61 | 11 | 119 | 50 | 111 | 44 | 113 |
| Tumor | 2766 | 0 | 0 | 0 | 110 | 21 | 6 | 2 | 0 |
| Ovary Normal | 0 | 0 | 1 | 0 | 2 | 1 | 2 | 1 | 1 |
| Tumor | 0 | 4 | 0 | 0 | 21 | 1 | 3 | 3 | 0 |
| Uterus Normal | 0 | 0 | 3 | 0 | 7 | 0 | 3 | 3 | 1 |
| Tumor | 19 | 0 | 0 | 0 | 9 | 1 | 21 | 8 | 1 |
| Prostate Normal | 0 | 0 | 0 | 0 | 18 | 1 | 3 | 1 | 0 |
| Tumor | 6 | 0 | 0 | 0 | 9 | 0 | 8 | 3 | 0 |
| Testis Normal 1) | 18 | 0 | 10 | 5 | 3 | 22 | 20 | 2 | 1 |
| Tumor 1) | 8 | 3 | 13 | 0 | 21 | 3 | 3 | 2 | 0 |
| Kidney Normal | 9 | 0 | 0 | 0 | 29 | 0 | 27 | 3 | 5 |
| Tumor | 9 | 0 | 0 | 0 | 28 | 10 | 15 | 0 | 0 |
| Lupus 1) | 25 | 0 | 1 | 0 | 1 | 0 | 3 | 1 | 0 |
| Liver Normal 1) | 0 | 0 | 10 | 0 | 27 | 11 | 13 | 5 | 1 |
| Cirrhosis 1) | 1 | 0 | 0 | 0 | 4 | 0 | 2 | 0 | 0 |
| Hippocampus Normal 1) | 6 | 12 | 4 | 0 | 40 | 113 | 2 | 5 | 2 |
| AD 1) | 16 | 1 | 50 | 3 | 111 | 63 | 55 | 12 | 27 |
| Frontal lobe Normal 1) | 3 | 2 | 8 | 0 | 16 | 140 | 3 | 8 | 1 |
| AD 1) | 2 | 1 | 1 | 0 | 9 | 29 | 2 | 2 | 0 |

When a 3-fold or more alternation in the expression level was reproducible, the difference is assessed as being significant. The cDNAs derived from the organs marked with 1) were purchased from BioChain; and the cDNAs derived from the organs without the mark were purchased from Clontech. The disease-dependent differences in the expression levels of the respective genes are summarized below.

The expression of GPRv8 was undetectable in the normal pancreas and uterus, but GPRv8 was expressed at a moderate level after canceration. GPRv8 was strongly expressed in the colon, and was more strongly expressed in colon cancer.

The expression level of GPRv12 was generally low. The expression was undetectable in the normal ovary and testis, but was found after canceration. The expression level decreased in the hippocampus with Alzheimer's disease.

GPRv16 was expressed in the colon, but was undetectable after canceration. The expression level increased in the brain after canceration. In the liver, the expression was undetectable after cirrhosis. In the brain of Alzheimer's disease patients, the expression level was elevated in the hippocampus.

The expression level of GPRv21 was low, and was undetectable in the colon and testis after canceration.

The expression level of GPRv40 increased in the brain and testis after canceration, and decreased in the liver after cirrhosis.

The expression level of GPRv47 increased in the brain and kidney and decreased in the testis after canceration. The expression was undetectable in the liver after cirrhosis.

GPRv51 was strongly expressed in the colon, but the expression level decreased after canceration. The expression level decreased in the testis after canceration. The expression level also decreased in the liver after cirrhosis as compared to the normal liver. The expression level was low in the brain, but increased in the hippocampus with Alzheimer's disease.

The expression level of GPRv71 decreased in the colon and kidney after canceration, and the expression thereof was undetectable in the liver after cirrhosis. In the patient with Alzheimer's disease, the expression level decreased in the frontal lobe.

GPRv72 was expressed strongly in the colon, but the expression thereof was undetectable after canceration. The expression level was low in the brain, but increased in the hippocampus with Alzheimer's disease.

[Example 4] Analysis of GPRv8 with bioinformatics

1. Homology search of GPRv8

The amino acid sequence of GPRv8 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res.* 25:3389-3402.). The result showed that GPRv8 had homology to the sequences shown in Table 2. Thus, GPRv8 was revealed to be a novel clone having homology to GPCR. The amino acid sequence of GPRv8 was analyzed by searching known sequences with an analysis program (BLAST 2.0); the result (data with the E-value lower than e-39) is shown in Table 2.

Table 2

| Hit (ID) | E-value | Identities % | Description |
|---|---|---|---|
| AE003754 | 2e-68 | 43 | gene: "CG6111"-Drosophila melanogaster |
| AF147743 | 7e-43 | 33 | vasotocin VT1 receptor-Gallus gallus |
| AF184966 | 2e-42 | 33 | arginine vasotocin receptor-Platichthys flesus |
| X93313 | 4e-42 | 36 | mesotocin receptor-giant toad |
| X76321 | 8e-42 | 32 | vasotocin receptor-white sucker |
| X87783 | 4e-41 | 33 | isotocin receptor-white sucker |
| X64878 | 3e-40 | 32 | oxytocin receptor- H.sapiens |
| U82440 | 7e-40 | 32 | oxytocin receptor-Macaca mulatta |

2. Prediction of transmembrane domain

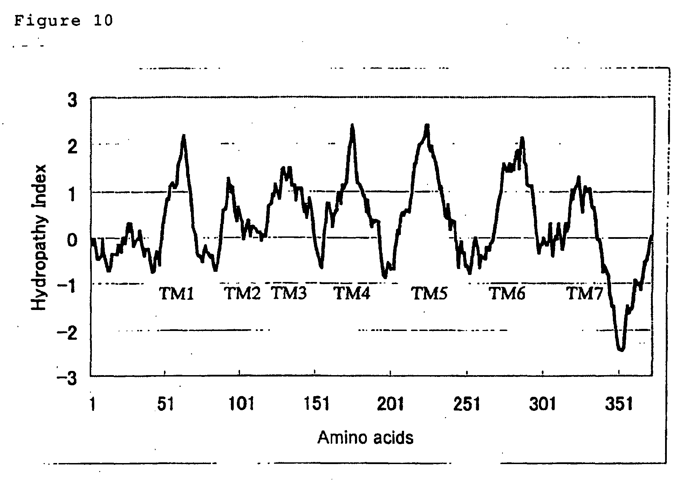

The amino acid sequence of GPRv8 was analyzed according to the method of Kyte-Doolittle (J. Kyte and R. F. Doolittle, (1982), *J. Mol. Biol.*, 157,105-132.), for obtaining a hydropathy plot and used to predict the transmembrane domain. The result showed that GPRv8 had seven transmembrane domains (TM1-TM7) (Figure 10).

3. HMMPfam search

Using the amino acid sequence of GPRv8 as the query, PFAM search based on the hidden Markov model (HMMPFAM (Sonnhammer EL, et al., *Nucleic Acids Res* 1998 Jan 1; 26 (1) :320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv8 comprises tm7_1 (Rhodopsin family). The result of HMMPfam search is shown in Table 3.

Table 3

| Hit | Score | Expect | Q from | Q to | Description |
|---|---|---|---|---|---|
| 7tm_1 | 164.2 | 5.1e-51 | 66 | 330 | 7 transmembrane receptor (rhodopsin family) |
| Hit: name of the domain deduced by the search.<br>Score: the higher the value, the higher the reliability.<br>Expext: as the value approaches 0, the reliability becomes higher.<br>Q from: the start position of the deduced domain.<br>Q to: the termination position of the deduced domain.<br>Description: explanation of the deduced domain. | | | | | |

4. Amino acid sequence alignment

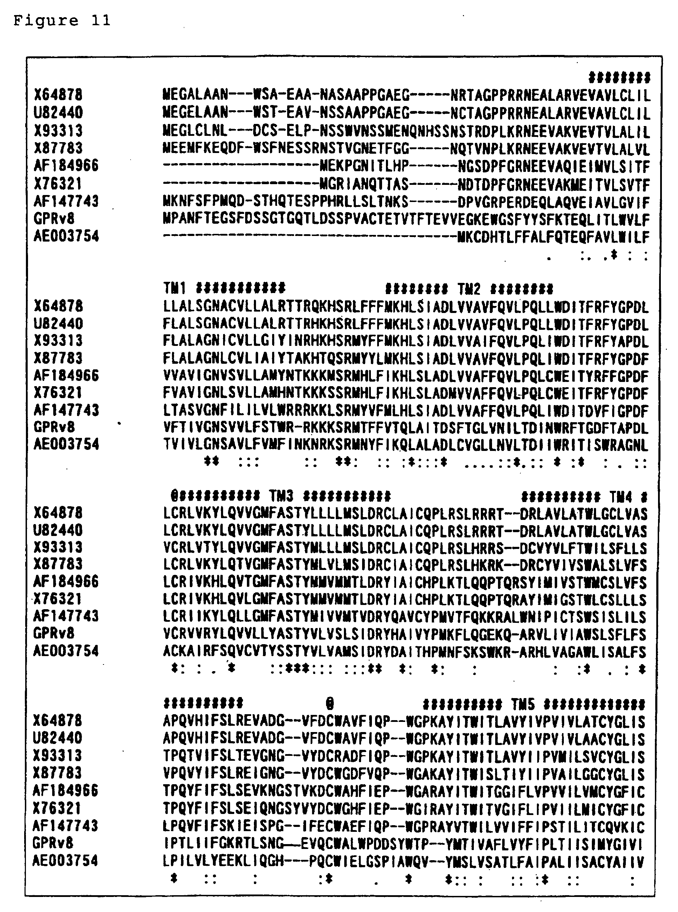

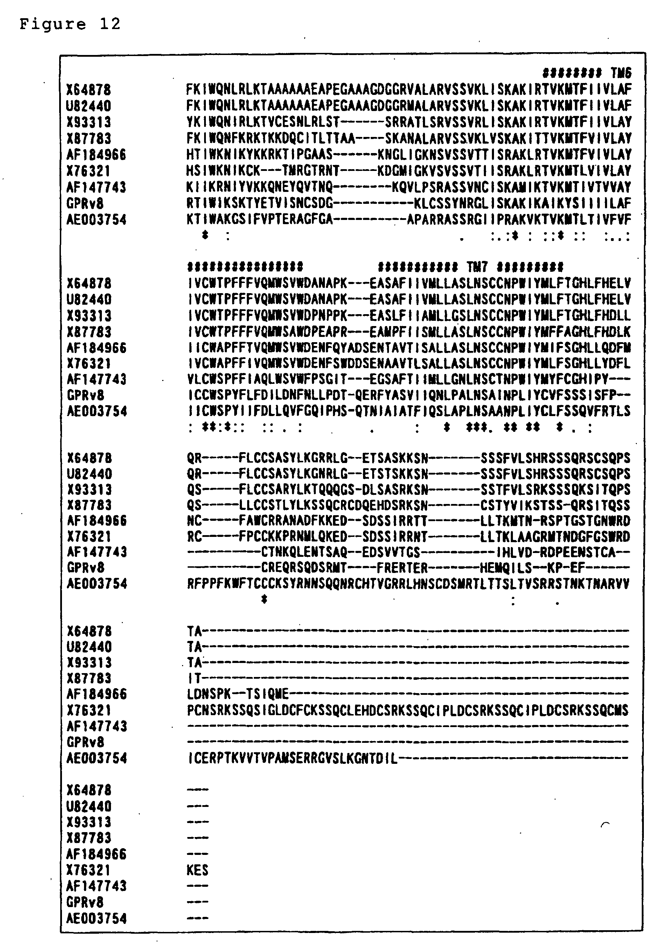

The amino acid sequences of GPRv8 and proteins shown in Table 2 were aligned together by using Clustalw 1.7 (Figures 11 and 12). The result showed that GPRv8 comprise seven transmembrane domains (### ###) and Cys (Cys marked with "@") participating in specific S-S bonding of GPCR.

[Example 5] Analysis of GPRv12 with bioinformatics

1. Homology search of GPRv12

The amino acid sequence of GPRv12 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res.* 25:3389-3402.). The result showed that GPRv12 had homology to the sequences shown in Table 4. Thus, GPRv12 was revealed to be a novel clone having homology to GPCR. The amino acid sequence of GPRv12 was analyzed by searching known sequences with an analysis program (BLAST 2.0); the result (data with the E-value lower than e-15) is shown in Table 4.

Table 4

| Hit (ID) | E-value | Identities % | Description |
|---|---|---|---|
| AF208288 | 8e-88 | 50 | orphan G protein-coupled receptor GPR26-Rattus norvegicus |
| L03202 | 2e-17 | 24 | 5-hydroxytryptamine receptor-rat |
| L41146 | 5e-17 | 23 | 5-HT6 serotonin receptor-Rattus norvegicus |
| S62043 | 2e-16 | 25 | serotonin receptor 6-rat |
| L41147 | 2e-16 | 24 | 5-HT6 serotonin receptor-Homo sapiens |
| AF134158 | 4e-16 | 23 | serotonin 6 receptor-Mus musculus |
| L14856 | 4e-16 | 26 | somatostatin receptor 4-Human |
| Y14627 | 5e-16 | 21 | Dopamine receptor-Cyprinus carpio |
| L07833 | 6e-16 | 26 | somatostatin receptor 4-Homo sapiens |
| AF069547 | 8e-16 | 21 | putative odorant receptor LOR4<br>-<br>Lampetra fluviatilis |

2. Prediction of transmembrane domain

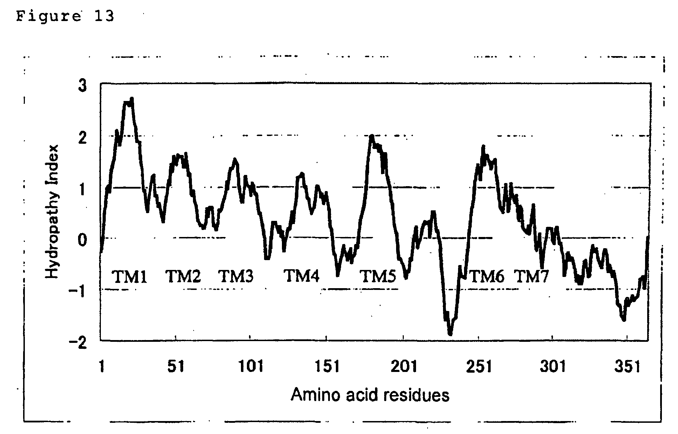

The amino acid sequence of GPRv12 was analyzed according to the method of Kyte-Doolittle (J. Kyte and R. F. Doolittle, (1982), *J. Mol. Biol.*, 157,105-132.), for obtaining a hydropathy plot and used to predict the transmembrane domain. The result showed that GPRv12 had seven transmembrane domains (TM1-TM7) (Figure 13).

3. HMMPfam search

Using the amino acid sequence of GPRv12 as the query, PFAM search based on the hidden Markov model (HMMPFAM (Sonnhammer EL, et al., *Nucleic Acids Res* 1998 Jan 1; 26 (1):320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv12 comprises tm7_1 (Rhodopsin family). The result of HMMPfam search is shown in Table 5.

Table 5

| Hit | Score | Expect | Q from | Q to | Description |
|---|---|---|---|---|---|
| 7tm_1 | 74.7 | 7.7e-23 | 22 | 294 | 7 transmembrane receptor (rhodopsin family) |

Hit: name of the domain deduced by the search.
Score: the higher the value, the higher the reliability.
Expext: as the value approaches 0, the reliability becomes higher.
Q from: the start position of the deduced domain.
Q to: the termination position of the deduced domain.
Description: explanation of the deduced domain.

4. Amino acid sequence alignment

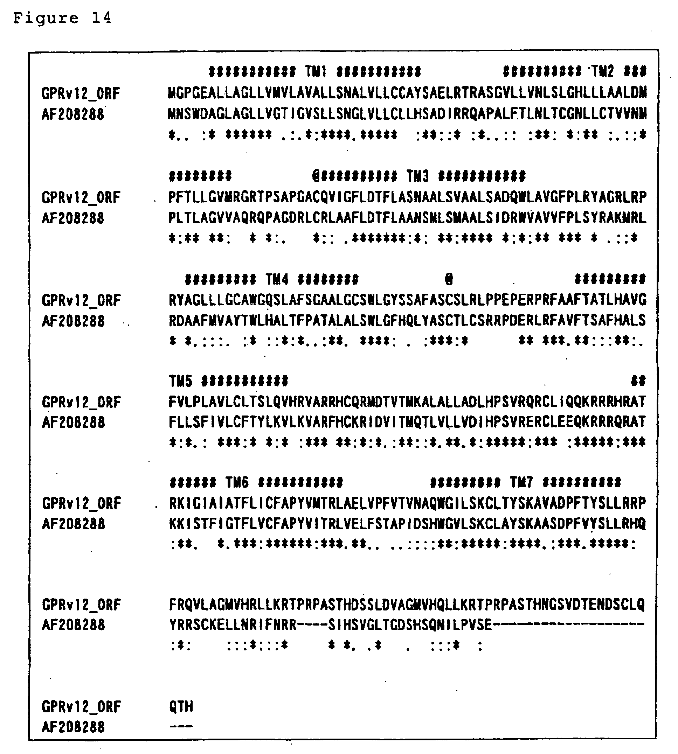

The amino acid sequences of GPRv12 and orphan G protein-coupled receptor GPR26- Rattus norvegicus (AF208288) were aligned together by using Clustalw 1.7 (Figure 14). The result showed that GPRv12 comprise seven transmembrane domains (### ###) and Cys (Cys marked with "@") participating in specific S-S bonding of GPCR.

[Example 6] Analysis of GPRv16 with bioinformatics

1. Homology search of GPRv16

The amino acid sequence of GPRv16 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res.* 25:3389-3402.). The result showed that GPRv16 had homology to the sequences shown in Table 6. Thus, GPRv16 was revealed to be a novel clone having homology to GPCR. The amino acid sequence of GPRv16 was analyzed by searching known sequences with an analysis program (BLAST 2.0); the result (data with the E-value lower than e-18) is shown in Table 6.

Table 6

| Hit (ID) | E-value | Identities % | Description |
|---|---|---|---|
| AF042784 | 4e-20 | 23 | GALANIN RECEPTOR TYPE 2-Mus musculus |
| U30290 | 4e-20 | 27 | galanin receptor GALR1-Rattus norvegicus |
| U90657 | 6e-20 | 27 | GALANIN RECEPTOR TYPE 1-mouse |
| AF042782 | 7e-20 | 25 | galanin receptor type 2-Homo sapiens |
| U94322 | 1e-19 | 24 | galanin receptor type2-Rattus norvegicus |
| AF077375 | 6e-19 | 23 | galanin receptor type2-Mus musculus |

2. Prediction of transmembrane domain

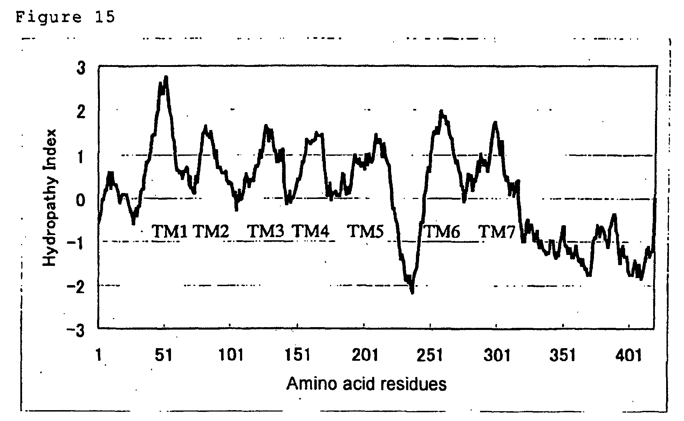

The amino acid sequence of GPRv16 was analyzed according to the method of Kyte-Doolittle (J. Kyte and R. F. Doolittle, (1982), *J. Mol. Biol.*, 157,105-132.), for obtaining a hydropathy plot and used to predict the transmembrane domain. The result showed that GPRv16 had seven transmembrane domains (TM1-TM7) (Figure 15).

3. HMMPfam search

Using the amino acid sequence of GPRv16 as the query, PFAM search based on the hidden Markov model (HMMPFAM (Sonnhammer EL, et al., *Nucleic Acids Res* 1998 Jan 1; 26 (1):320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv16 comprises tm7_1 (Rhodopsin family). The result of HMMPfam search is shown in Table 7.

Table 7

| Hit | Score | Expect | Q from | Q to | Description |
|---|---|---|---|---|---|
| 7tm_1 | 23.8 | 8.3e-7 | 155 | 306 | 7 transmembrane receptor (rhodopsin family) |
| 7tm_1 | 13.3 | 0.0017 | 53 | 133 | 7 transmembrane receptor (rhodopsin family) |

Hit: name of the domain deduced by the search.
Score: the higher the value, the higher the reliability.
Expext: as the value approaches 0, the reliability becomes higher.
Q from: the start position of the deduced domain.
Q to: the termination position of the deduced domain.
Description: explanation of the deduced domain.

4. Amino acid sequence alignment

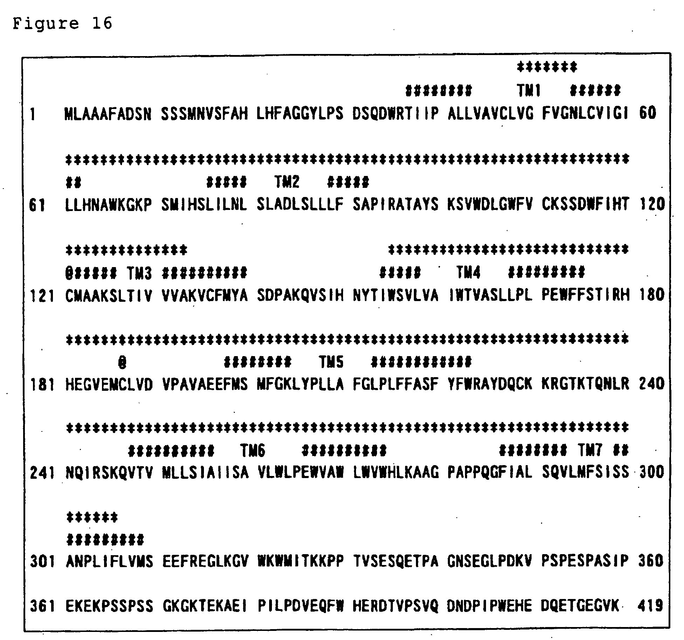

The result of sections 3 and 4 are indicated in Figure 16. The result showed that GPRv16 comprise Cys (@) participating in specific S-S bonding of GPCR.

[Example 7] Analysis of GPRv21 with bioinformatics

1. Homology search of GPRv21

The amino acid sequence of GPRv21 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res.* 25:3389-3402.). The result showed that GPRv21 had homology to the sequences shown in Table 8. Thus, GPRv21 was revealed to be a novel clone having homology to GPCR. The amino acid sequence of GPRv21 was analyzed by searching known sequences with an analysis program (BLAST 2.0); the result (data with the E-value lower than e-35) is shown in Table 8.

Table 8

| Hit (ID) | E-value | Identities % | Description |
|---|---|---|---|
| AL121755 | 0.0 | 89 | G-protein coupled receptor-Human |
| AF236082 | 0.0 | 83 | G-protein coupled receptor GPR73-Mus musculus |
| M81490 | 9e-37 | 34 | neuropeptide receptor-D. melanogaster |
| U50144 | 3e-36 | 30 | type 2 neuropeptide Y receptor-Bos taurus |
| U42766 | 6e-36 | 29 | neuropeptide y2 receptor-Human |
| AF037444 | 8e-36 | 28 | cardioexcitatory receptor-Lymnaea stagnalis |
| D86238 | 8e-36 | 28 | neuropeptideY-Y2 receptor-Mus musculus |
| U42389 | 8e-36 | 29 | neuropeptide y/peptide YY receptor type 2-human |
| U76254 | 8e-36 | 29 | neuropeptide Y receptor type 2-Human |

2. Prediction of transmembrane domain

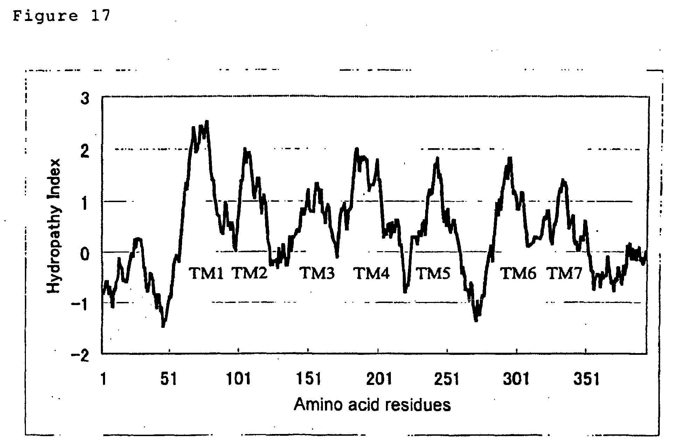

The amino acid sequence of GPRv21 was analyzed according to the method of Kyte-Doolittle (J. Kyte and R. F. Doolittle, (1982), *J. Mol. Biol.*, 157,105-132.), for obtaining a hydropathy plot and used to predict the transmembrane domain. The result showed that GPRv21 had seven transmembrane domains (TM1-TM7) (Figure 17).

3. HMMPfam search

Using the amino acid sequence of GPRv21 as the query, PFAM search based on the hidden Markov model (HMMPFAM (Sonnhammer EL, et al., *Nucleic Acids Res* 1998 Jan 1; 26 (1):320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv21 comprises tm7_1 (Rhodopsin family). The result of HMMPfam search is shown in Table 9.

Table 9

| Hit | Score | Expect | Q from | Q to | Description |
|---|---|---|---|---|---|
| 7tm_1 | 188.1 | 1.6e-58 | 79 | 338 | 7 transmembrane receptor (rhodopsin family) |

Hit: name of the domain deduced by the search.
Score: the higher the value, the higher the reliability.
Expext: as the value approaches 0, the reliability becomes higher.
Q from: the start position of the deduced domain.
Q to: the termination position of the deduced domain.
Description: explanation of the deduced domain.

4. Amino acid sequence alignment

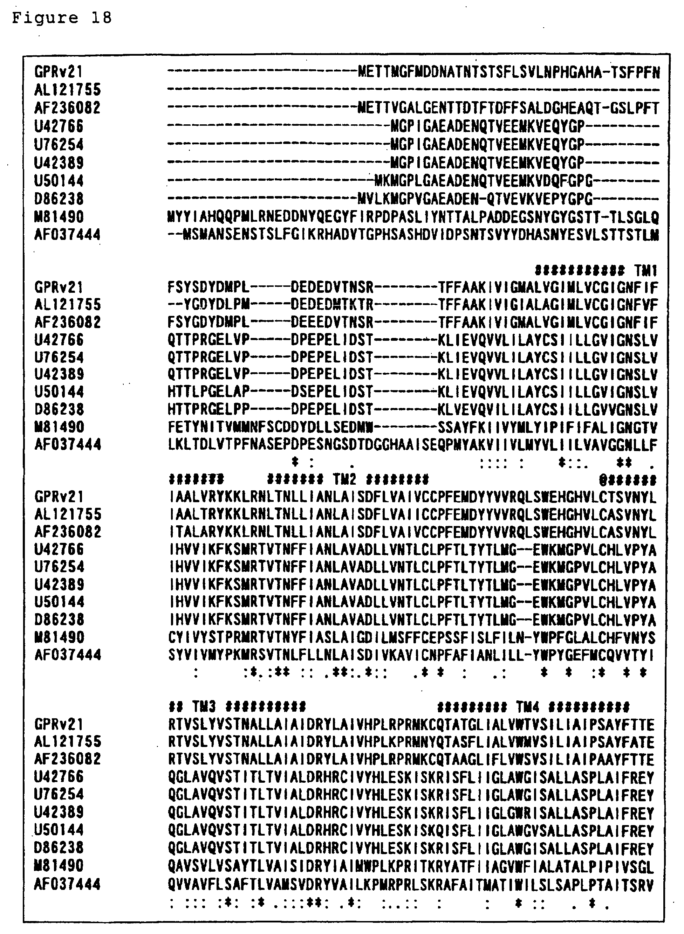

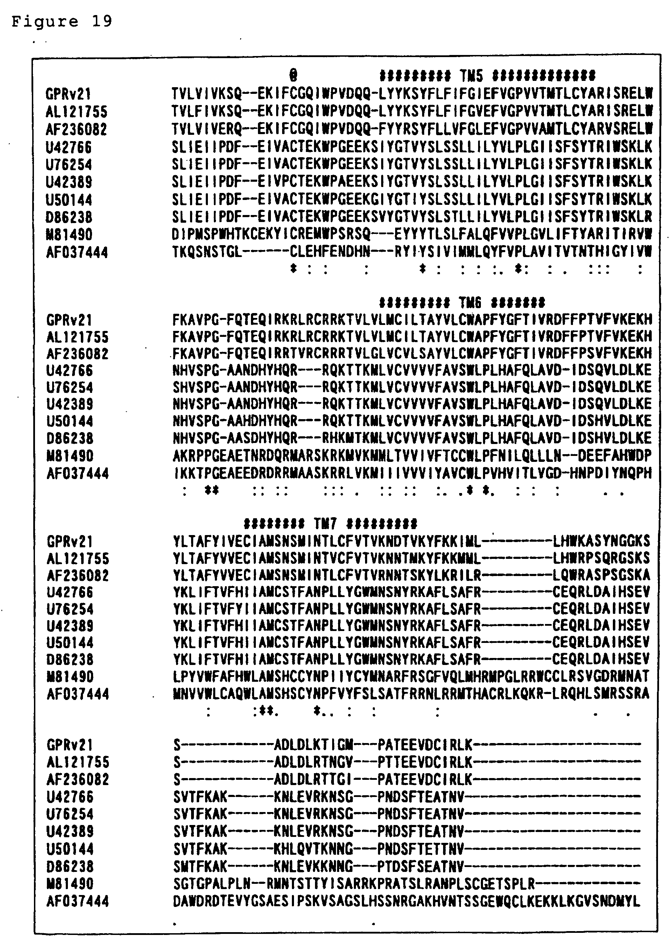

The amino acid sequences of GPRv21 and proteins shown in Table 8 were aligned together by using Clustalw 1.7 (Figures 18 and 19). The result showed that GPRv21 comprise seven transmembrane domains (### ###) and Cys (Cys marked with "@") participating in specific S-S bonding of GPCR.

[Example 8] Analysis of GPRv40 with bioinformatics

1. Homology search of GPRv40

The amino acid sequence of GPRv40 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res.* 25:3389-3402.). The result showed that GPRv40 had homology to the sequences shown in Table 10. Thus, GPRv40 was revealed to be a novel clone having homology to GPCR. The amino acid sequence of GPRv40 was analyzed by searching known sequences with an analysis program (BLAST 2.0); the result (data with the E-value lower than e-11) is shown in Table 10.

Table 10

| Hit (ID) | E-value | Identities % | Description |
|---|---|---|---|
| D86599 | 1e-13 | 23 | oxytocin receptor- Mus sp. |
| U15280 | 4e-13 | 23 | oxytocin 23 receptor-Rattus norvegicus |
| X76321 | 1e-12 | 22 | vasotocin receptor-white sucker |
| X64878 | 2e-12 | 21 | oxytocin receptor-H.sapiens |
| X87783 | 2e-12 | 21 | isotocin receptor-C.commersoni |
| D45400 | 3e-12 | 23 | vasopressin receptor V1b-rat |
| L37112 | 3e-12 | 24 | vasopressin receptor subtype 1b-Homo sapiens |

Table 10 (continued)

| Hit (ID) | E-value | Identities % | Description |
|---|---|---|---|
| U27322 | 6e-12 | 23 | arginine-vasopressin V1b receptor-Rattus norvegicus |
| U82440 | 6e-12 | 21 | oxytocin receptor-Macaca mulatta |

2. Prediction of transmembrane domain

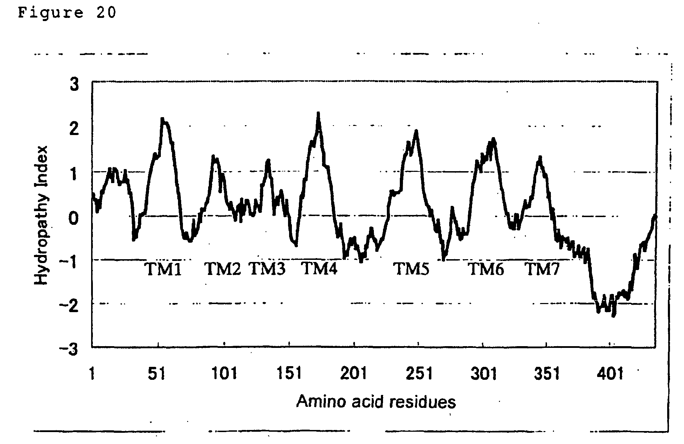

The amino acid sequence of GPRv40 was analyzed according to the method of Kyte-Doolittle (J. Kyte and R. F. Doolittle, (1982), *J. Mol. Biol.*, 157,105-132.), for obtaining a hydropathy plot and used to predict the transmembrane domain. The result showed that GPRv40 had seven transmembrane domains (TM1-TM7) (Figure 20).

3. HMMPfam search

Using the amino acid sequence of GPRv40 as the query, PFAM search based on the hidden Markov model (HMMPFAM (Sonnhammer EL, et al., *Nucleic Acids Res* 1998 Jan 1; 26 (1):320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv40 comprises tm7_1 (Rhodopsin family). The result of HMMPfam search is shown in Table 11.

Table 11

| Hit | Score | Expect | Q from | Q to | Description |
|---|---|---|---|---|---|
| 7tm_1 | 26.5 | 1.1e-07 | 228 | 352 | 7 transmembrane receptor (rhodopsin family) |
| 7tm_1 | 18.1 | 5e-05 | 59 | 181 | 7 transmembrane receptor (rhodopsin family) |

Hit: name of the domain deduced by the search.
Score: the higher the value, the higher the reliability.
Expext: as the value approaches 0, the reliability becomes higher.
Q from: the start position of the deduced domain.
Q to: the termination position of the deduced domain.
Description: explanation of the deduced domain.

4. Amino acid sequence alignment

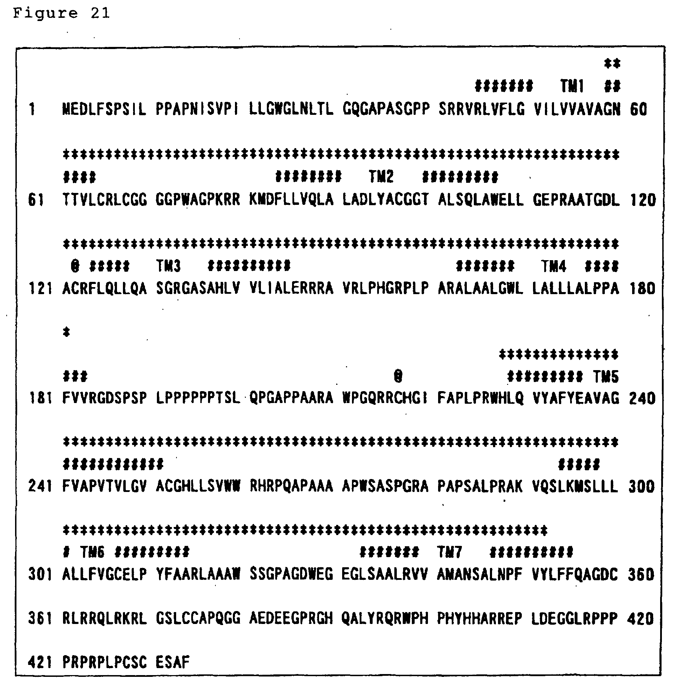

The result of section 3 and 4 are indicated in Figure 21. The result showed that GPRv40 comprise Cys (@) participating in specific S-S bonding of GPCR.

[Example 9] Analysis of GPRv47 with bioinformatics

1. Homology search of GPRv47

The amino acid sequence of GPRv47 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res*. 25:3389-3402.). The result showed that GPRv47 had homology to the sequences shown in Table 12. Thus, GPRv47 was revealed to be a novel clone having homology to GPCR. The amino acid sequence of GPRv47 was analyzed by searching known sequences with an analysis program (BLAST 2.0); the result (data with the E-value lower than e-11) is shown in Table 12.

Table 12

| Hit (ID) | E-value | Identities % | Description |
|---|---|---|---|
| D43633 | 1e-85 | 41 | G protein-coupled 7-transmembrane receptor-Medaka fish |

Table 12 (continued)

| Hit (ID) | E-value | Identities % | Description |
|---|---|---|---|
| X98133 | 2e-28 | 27 | histamine H2 receptor-H.sapiens |
| M32701 | 3e-28 | 28 | histamine H2 receptor-Canine histamine |
| L41147 | 6e-28 | 31 | 5-HT6 serotonin receptor-Homo sapiens |
| U25440 | 8e-28 | 26 | histamine H2 receptor-Cavia porcellus |
| D49783 | 1e-27 | 28 | histamine H2 receptor- Human |
| U64032 | 2e-27 | 27 | alpha 1d adrenoceptor-Oryctolagus cuniculus |
| S73473 | 3e-27 | 28 | beta 3-adrenergic receptor-rats |
| M74716 | 4e-27 | 28 | beta-adrenergic receptor- Rat |
| S57565 | 6e-27 | 27 | histamine H2-receptor- rats |

2. Prediction of transmembrane domain

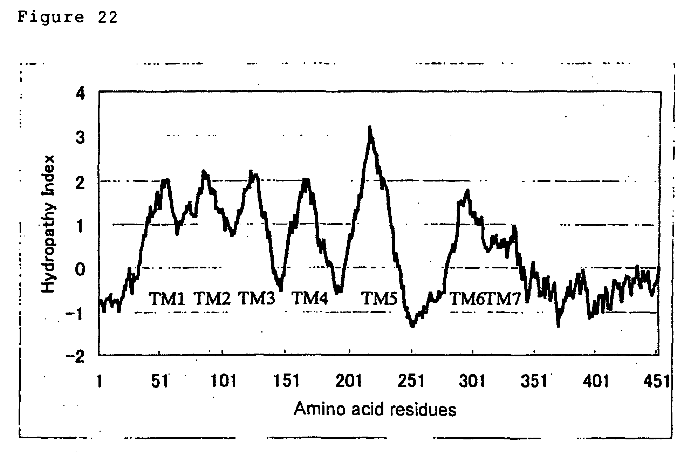

The amino acid sequence of GPRv47 was analyzed according to the method of Kyte-Doolittle (J. Kyte and R. F. Doolittle, (1982), *J. Mol. Biol.,* 157,105-132.), for obtaining a hydropathy plot and used to predict the transmembrane domain. The result showed that GPRv47 had seven transmembrane domains (TM1-TM7) (Figure 22).

3. HMMPfam search

Using the amino acid sequence of GPRv47 as the query, PFAM search based on the hidden Markov model (HMMPFAM (Sonnhammer EL, et al., *Nucleic Acids Res* 1998 Jan 1; 26 (1):320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv47 comprises tm7_1 (Rhodopsin family). The result of HMMPfam search is shown in Table 13.

Table 13

| Hit | Score | Expect | Q from | Q to | Description |
|---|---|---|---|---|---|
| 7tm_1 | 137.9 | 9.6e-43 | 59 | 341 | 7 transmembrane receptor (rhodopsin family) |

Hit: name of the domain deduced by the search.
Score: the higher the value, the higher the reliability.
Expext: as the value approaches 0, the reliability becomes higher.
Q from: the start position of the deduced domain.
Q to: the termination position of the deduced domain.
Description: explanation of the deduced domain.

4. Amino acid sequence alignment

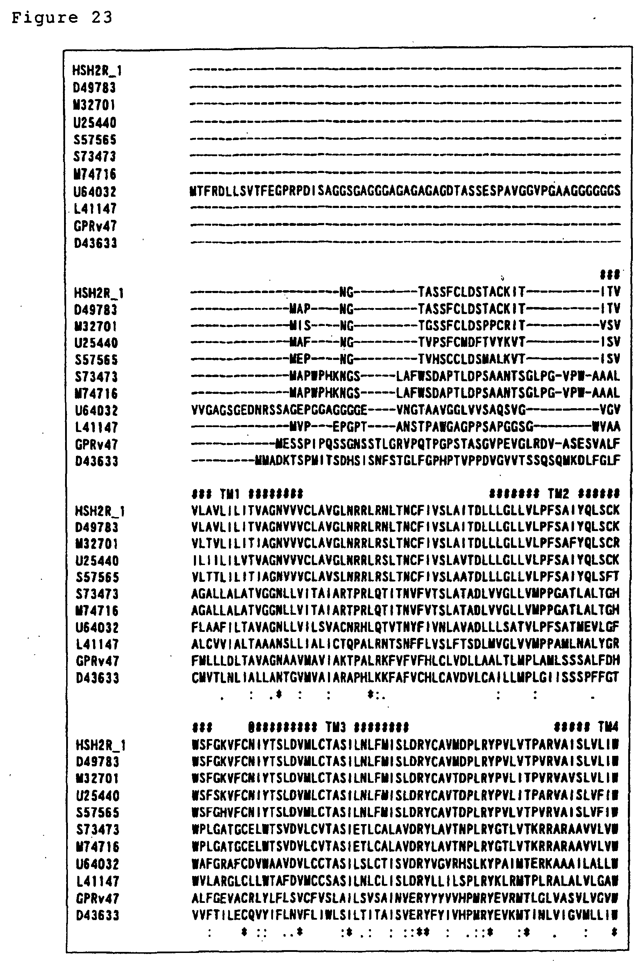

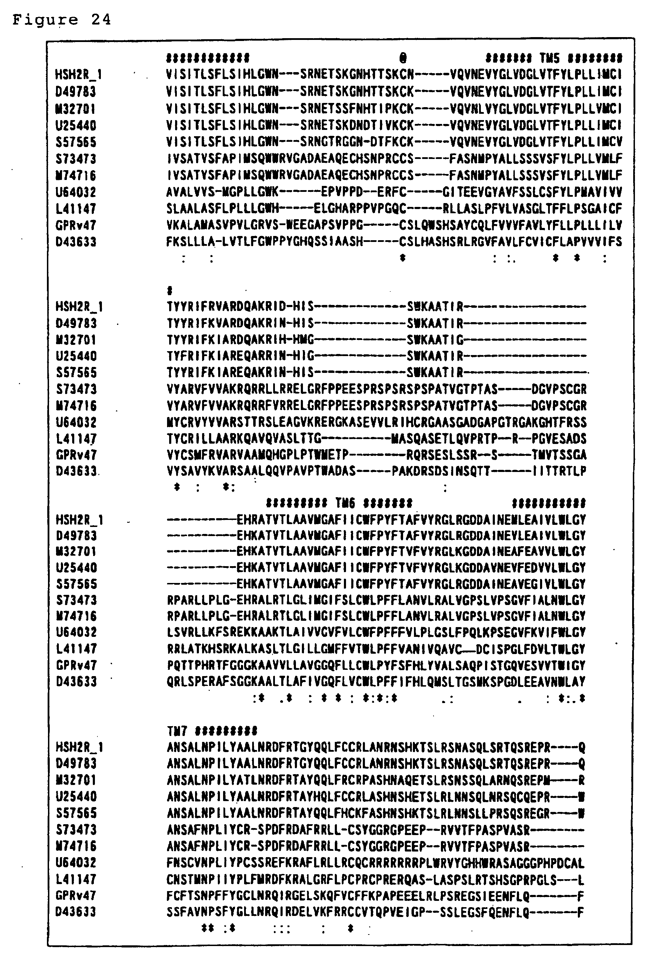

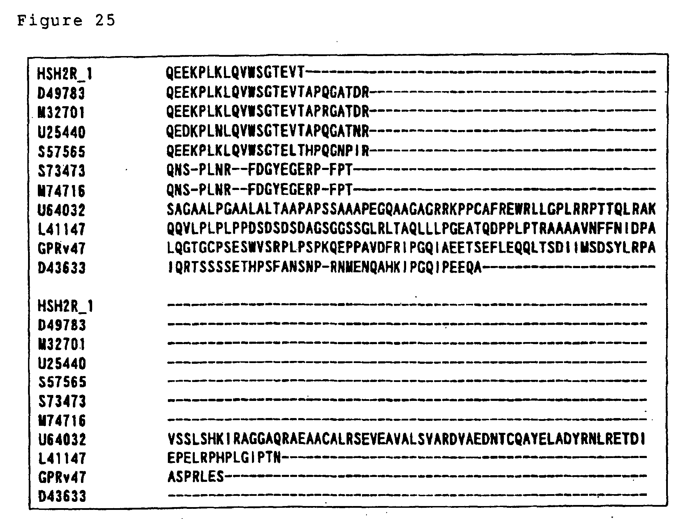

The amino acid sequences of GPRv47 and proteins shown in Table 2 were aligned together by using Clustalw 1.7 (Figures 23 to 25). The result showed that GPRv47 comprise seven transmembrane domains (### ###) and Cys (Cys marked with "@") participating in specific S-S bonding of GPCR.

[Example 10] Analysis of GPRv51 with bioinformatics

1. Homology search of GPRv51

The amino acid sequence of GPRv51 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res.* 25:3389-3402.). The result showed that GPRv51 had homology to the sequences shown in Table 14. Thus, GPRv51 was revealed to be a novel clone having homology to GPCR. The amino acid sequence of GPRv51 was analyzed by searching known sequences with an analysis program (BLAST 2.0); the result (data with the E-value lower than e-18) is shown in Table 14.

Table 14

| Hit (ID) | E-value | Identities % | Description |
|---|---|---|---|
| M35297 | 4e-43 | 36 | G-protein coupled receptor-Rat |
| J03823 | 1e-42 | 34 | Rat mas oncogene, complete cds. |
| M13150 | 3e-40 | 34 | mas proto-oncogene- Human |
| X67735 | 1e-39 | 35 | Mas proto-oncogene-M.musculus mas |
| AL035542 | 1e-35 | 36 | MAS-related Gprotein-coupled receptor MRG-Human |

2. Prediction of transmembrane domain

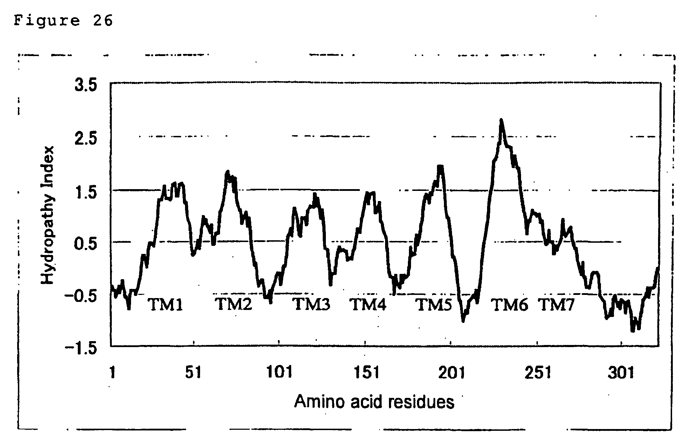

The amino acid sequence of GPRv51 was analyzed according to the method of Kyte-Doolittle (J. Kyte and R. F. Doolittle, (1982), *J. Mol. Biol.*, 157,105-132.), for obtaining a hydropathy plot and used to predict the transmembrane domain. The result showed that GPRv51 had seven transmembrane domains (TM1-TM7) (Figure 26).

3. HMMPfam search

Using the amino acid sequence of GPRv51 as the query, PFAM search based on the hidden Markov model (HMMPFAM (Sonnhammer EL, et al., *Nucleic Acids Res* 1998 Jan 1; 26 (1):320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv51 comprises tm7_1 (Rhodopsin family). The result of HMMPfam search is shown in Table 15.

Table 15

| Hit | Score | Expect | Q from | Q to | Description |
|---|---|---|---|---|---|
| 7tm_1 | 32.6 | 1.4e-09 | 44 | 78 | 7 transmembrane receptor (rhodopsin family) |
| 7tm_1 | 30.1 | 8.7e-09 | 104 | 276 | 7 transmembrane receptor (rhodopsin family) |
| Hit: name of the domain deduced by the search.<br>Score: the higher the value, the higher the reliability.<br>Expext: as the value approaches 0, the reliability becomes higher.<br>Q from: the start position of the deduced domain.<br>Q to: the termination position of the deduced domain.<br>Description: explanation of the deduced domain. | | | | | |

4. Amino acid sequence alignment

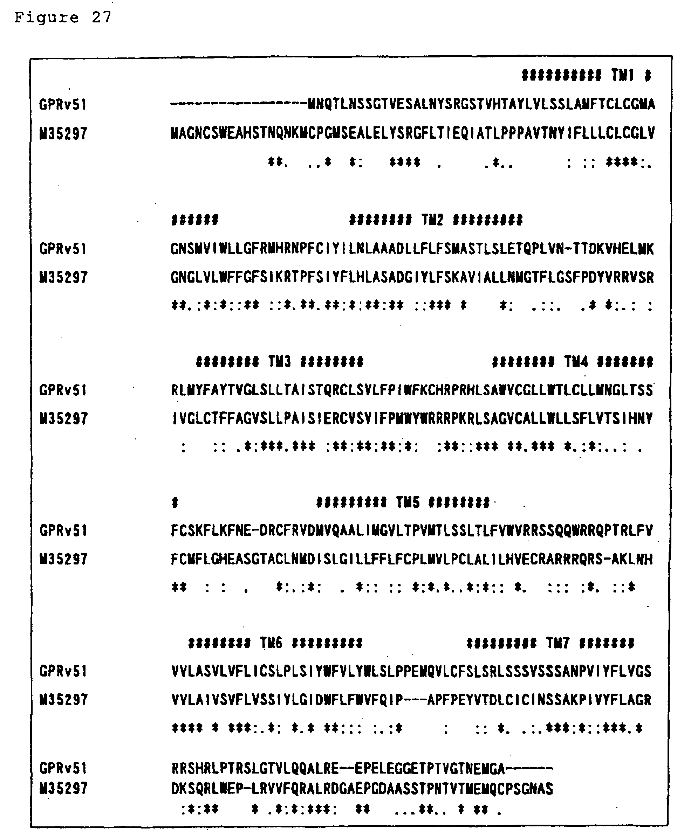

The amino acid sequences of GPRv51 and G-protein coupled receptor- Rat (M35297) were aligned together by using Clustalw 1.7 (Figure 27). The result showed that GPRv51 comprise seven transmembrane domains (### ###).

[Example 11] Analysis of GPRv71 with bioinformatics

1. Homology search of GPRv71

The amino acid sequence of GPRv71 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res.* 25:3389-3402.). The result showed that GPRv71 had homology to the sequences shown in Table 16. Thus, GPRv71 was revealed to be a novel clone having homology to GPCR. The amino acid sequence of GPRv71 was analyzed by searching known sequences with an analysis program (BLAST 2.0); the result (data with the E-value lower than e-35) is shown in Table 16.

Table 16

| Hit (ID) | E-value | Identities % | Description |
|---|---|---|---|
| AF069555 | 9e-44 | 44 | G protein-coupled receptor p2y3-Meleagris gallopavo |
| X98283 | 9e-44 | 45 | P2Y PURINOCEPTOR 3-G.domesticus |
| AF031897 | 6e-41 | 40 | P2Y nucleotide receptor-Meleagris gallopavo |
| X99953 | 1e-39 | 41 | P2Y PURINOCEPTOR 8- X.laevis |
| D63665 | 2e-37 | 41 | novel G protein-coupled P2 receptor-Rat |
| Y14705 | 1e-36 | 40 | P2Y4 receptor gene-Rattus norvegicus |
| AJ277752 | 2e-36 | 41 | P2Y4 receptor- Mus musculus |

2. Prediction of transmembrane domain

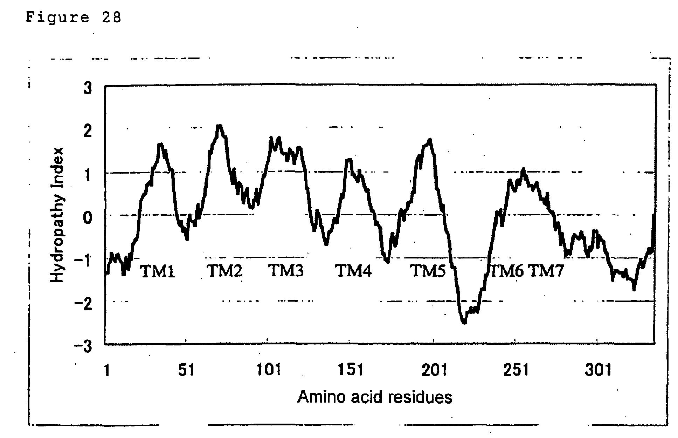

The amino acid sequence of GPRv71 was analyzed according to the method of Kyte-Doolittle (J. Kyte and R. F. Doolittle, (1982), *J. Mol. Biol.,* 157,105-132.), for obtaining a hydropathy plot and used to predict the transmembrane domain. The result showed that GPRv71 had seven transmembrane domains (TM1-TM7) (Figure 28).

3. HMMPfam search

Using the amino acid sequence of GPRv71 as the query, PFAM search based on the hidden Markov model (HMMPFAM (Sonnhammer EL, et al., *Nucleic Acids Res* 1998 Jan 1; 26 (1) : 320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv71 comprises tm7_1 (Rhodopsin family). The result of HMMPfam search is shown in Table 17.

Table 17

| Hit | Score | Expect | Q from | Q to | Description |
|---|---|---|---|---|---|
| 7tm_1 | 90.6 | 7.6e-28 | 40 | 161 | 7 transmembrane receptor (rhodopsin family) |
| Hit: name of the domain deduced by the search.<br>Score: the higher the value, the higher the reliability.<br>Expext: as the value approaches 0, the reliability becomes higher.<br>Q from: the start position of the deduced domain.<br>Q to: the termination position of the deduced domain.<br>Description: explanation of the deduced domain. | | | | | |

4. Amino acid sequence alignment

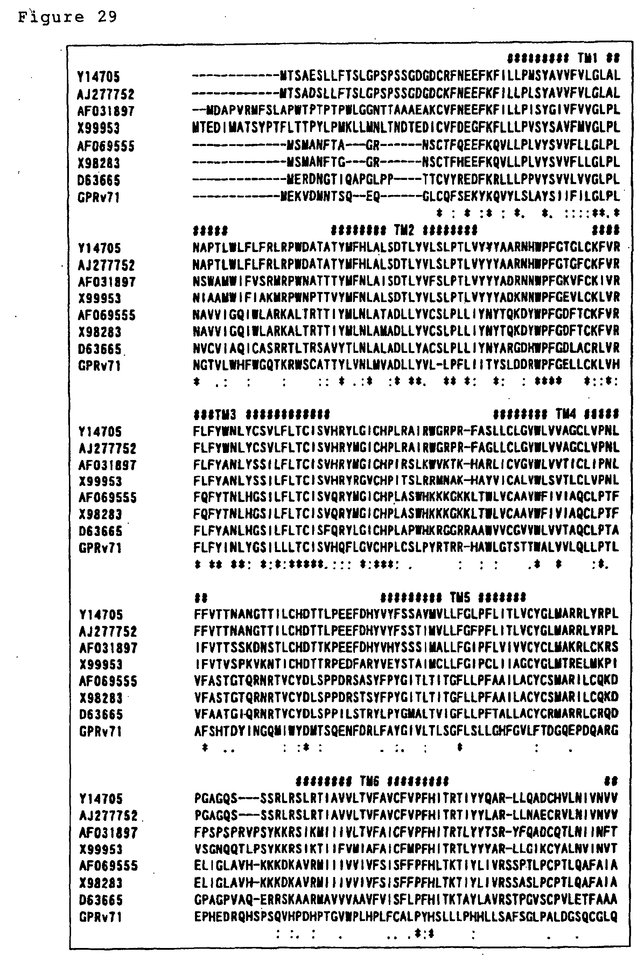

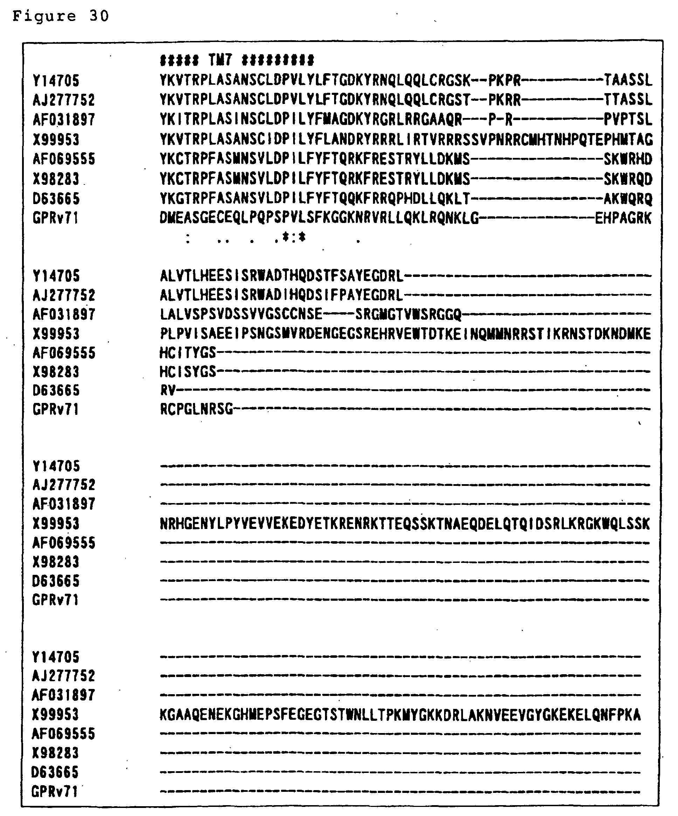

The amino acid sequences of GPRv71 and proteins shown in Table 2 were aligned together by using Clustalw 1.7 (Figures 29 and 30). The result showed that GPRv71 comprise seven transmembrane domains (### ###).

[Example 12] Analysis of GPRv72 with bioinformatics

1. Homology search of GPRv72

The amino acid sequence of GPRv72 was analyzed by searching known sequences (the known sequence databases are produced in EMBL (Release 64, http://www.ebi.ac.uk/), GENBANK (Release 120.0, http://www.ncbi.nlm.nih.gov/), and PIR (Release 66.00, http://www-nbrf.georgetown.edu/pir/)) with an analysis program (BLAST 2.0) (Altschul, Stephen F. et al. (1997) *Nucleic Acids Res.* 25:3389-3402.). The result showed that GPRv72 had homology to the sequences shown in Table 18. Thus, GPRv72 was revealed to be a novel clone having homology to GPCR. The amino acid sequence of GPRv72 was analyzed by searching known sequences with an analysis program (BLAST 2.0); the result (data with the E-value lower than e-24) is shown in Table 18.

Table 18

| Hit (ID) | E-value | Identities % | Description |
|---|---|---|---|
| AF091890 | 4e-29 | 32 | G-protein coupled receptor RE2-Homo sapiens |
| U81982 | 3e-25 | 30 | alpha 1a-adrenoceptor-Oryctolagus cuniculus |
| S71323 | 6e-25 | 32 | alpha-1A adrenergic receptor-Japanese medaka |
| D63859 | 6e-25 | 32 | alpha1A-adrenoceptor-Oryzias latipes |
| U07126 | 8e-25 | 29 | alpha1c adrenergic receptor-Rattus norvegicus |
| U03866 | 8e-25 | 30 | adrenergic alpha-1c receptor protein-Human |
| AF013261 | 8e-25 | 30 | alpha 1A adrenergic receptor isoform 4-Homo sapiens |
| L31774 | 8e-25 | 30 | alpha-1C-adrenergic receptor-Human |
| D32202 | 8e-25 | 30 | alpha 1C adrenergic receptor isoform 2-Human |
| D32201 | 8e-25 | 30 | alpha 1C adrenergic receptor isoform 3-Human |
| D25235 | 8e-25 | 30 | alpha1C adrenergic receptor |

2. Prediction of transmembrane domain

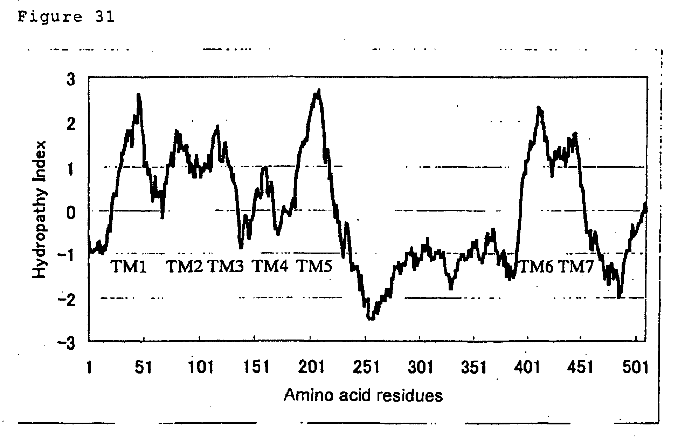

The amino acid sequence of GPRv72 was analyzed according to the method of Kyte-Doolittle (J. Kyte and R. F. Doolittle, (1982), *J. Mol. Biol.*, 157,105-132.), for obtaining a hydropathy plot and used to predict the transmembrane domain. The result showed that GPRv72 had seven transmembrane domains (TM1-TM7) (Figure 31).

3. HMMPfam search

Using the amino acid sequence of GPRv72 as the query, PFAM search based on the hidden Markov model (HMMPFAM (Sonnhammer EL, et al., *Nucleic Acids Res* 1998 Jan 1; 26 (1) :320-322)) was carried out. The search was carried out with the hidden Markov model of HMMER version 2.1 (http://hmmer.wustl.edu/) and the PFAM database of Pfam Version 5.5 (http://www.sanger.ac.uk/Software/Pfam/index.shtml).

The result indicated that GPRv72 comprises tm7_1 (Rhodopsin family). The result of HMMPfam search is shown in Table 19.

Table 19

| Hit | Score | Expect | Q from | Q to | Description |
|---|---|---|---|---|---|
| 7tm_1 | 196.1 | 4.7e-61 | 48 | 454 | 7 transmembrane receptor (rhodopsin family) |

Hit: name of the domain deduced by the search.
Score: the higher the value, the higher the reliability.
Expext: as the value approaches 0, the reliability becomes higher.
Q from: the start position of the deduced domain.
Q to: the termination position of the deduced domain.
Description: explanation of the deduced domain.

4. Amino acid sequence alignment

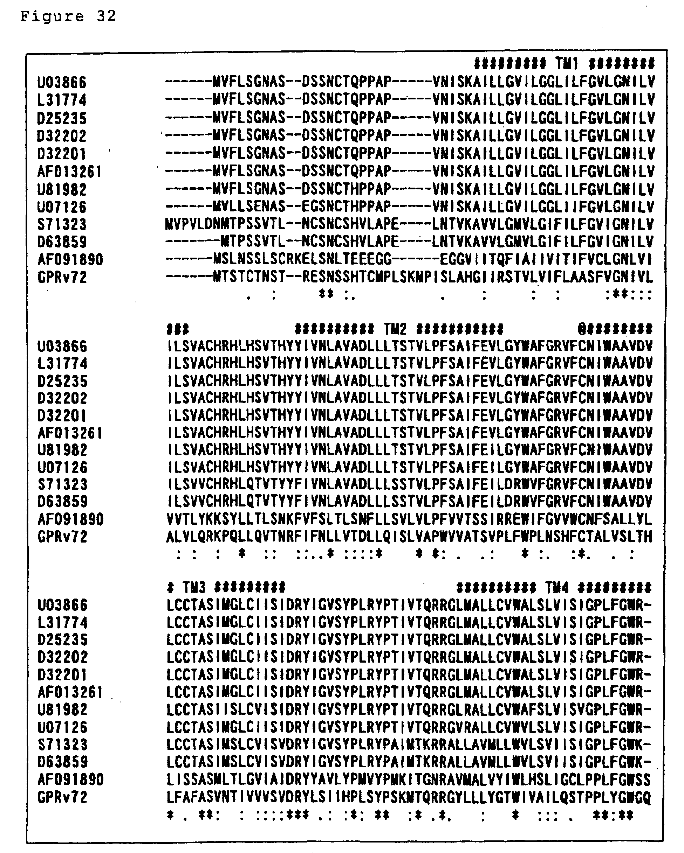

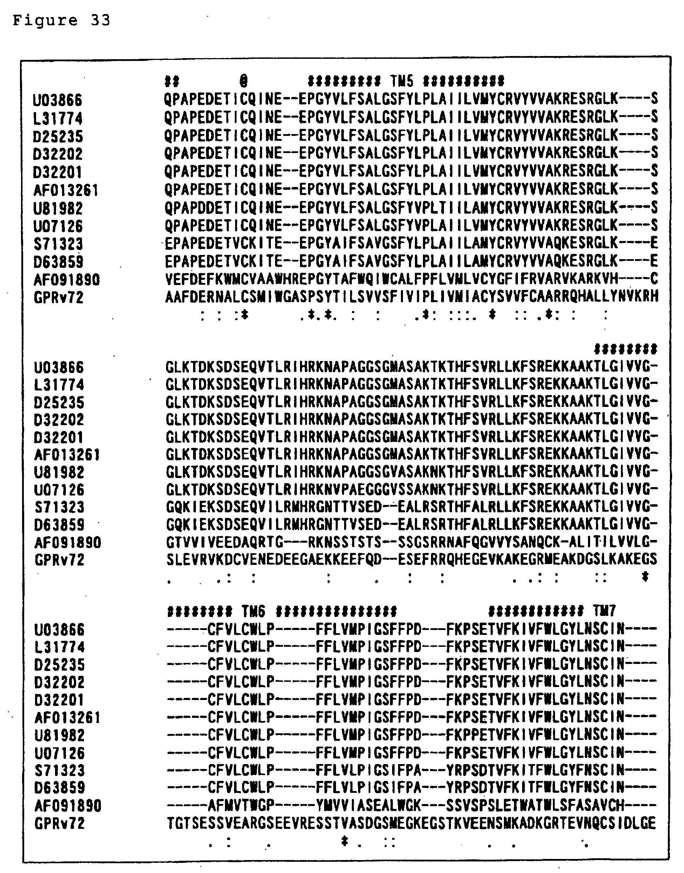

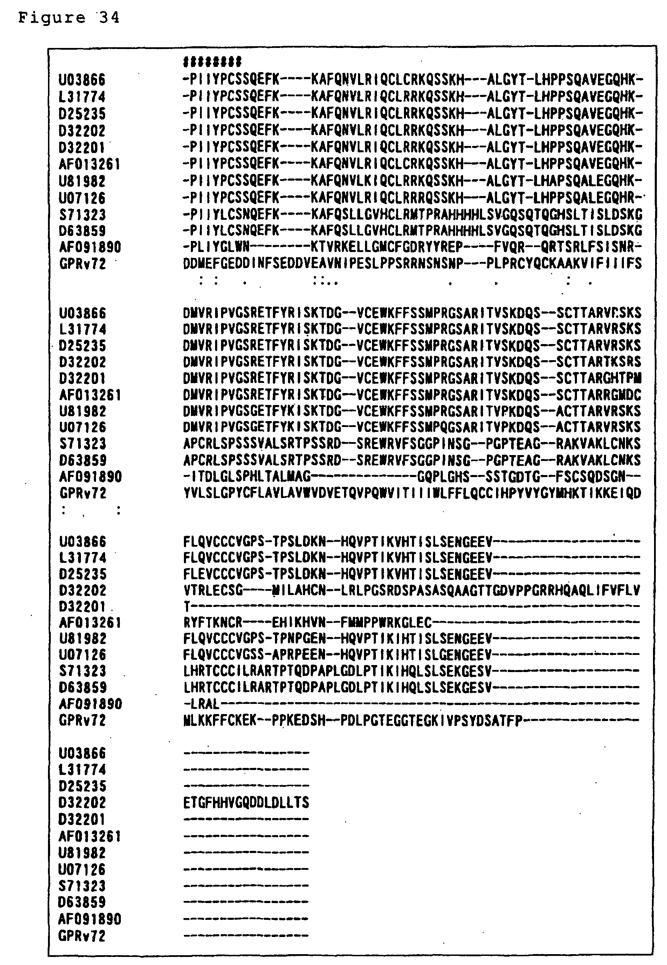

The amino acid sequences of GPRv72 and proteins shown in Table 18 were aligned together by using Clustalw 1.7 (Figures 32 to 34). The result showed that GPRv72 comprise seven transmembrane domains (### ###) and Cys (Cys marked with "@") participating in specific S-S bonding of GPCR.

Industrial Applicability

The present invention provided novel G protein-coupled receptors (GPRv8, GPRv12, GPRv16, GPRv21, GPRv40, GPRv47, GPRv51, GPRv71, and GPRv72), the genes encoding the proteins, vectors containing the genes, host cells containing the vectors, and a method for producing the proteins. Further, the present invention provided a screening method for compounds modifying the activities of the proteins. The proteins and genes of the present invention, and compounds modifying the activity of the proteins, are expected to be used for the development of new preventives and therapeutics for the diseases, with which the G protein-coupled receptors of the present invention are associated.

## Claims

1. A DNA that encodes a guanosine triphosphate-binding protein-coupled receptor, wherein said DNA is selected from the group consisting of the following (a) to (d):

   (a) a DNA encoding a protein comprising the amino acid sequence of any one of SEQ ID NOs: 1 to 4 and 17 to 21;
   (b) a DNA comprising a coding region of the nucleotide sequence of any one of SEQ ID NOs: 5 to 8 and 22 to 26;
   (c) a DNA encoding a protein comprising the amino acid sequence of any one of SEQ ID NOs: 1 to 4 and 17 to 21 in which one or more amino acids are substituted, deleted, added, and/or inserted; and
   (d) a DNA hybridizing under stringent conditions to the DNA comprising the nucleotide sequence of any one of SEQ ID NOs: 5 to 8 and 22 to 26.

2. A DNA encoding a partial peptide of a protein comprising the amino acid sequence of any one of SEQ ID NOs: 1 to 4 and 17 to 21.

3. A vector comprising the DNA of any one of claims 1 and 2.

4. A transformant carrying the DNA of any one of claims 1 and 2 or the vector of claim 3.

**5.** A protein or a peptide encoded by the DNA of any one of claims 1 and 2.

**6.** A method for producing the protein or the peptide of claim 5, said method comprising the steps of culturing the transformant of claim 4 and recovering an expressed protein or peptide from the transformant or culture supernatant thereof.

**7.** A method of screening for ligands that bind to the protein of claim 5, said method comprising the steps of:

(a) contacting a test sample with the protein or the peptide of claim 5; and
(b) selecting compounds that binds to said protein or said peptide.

**8.** A method of screening for compounds that have activity of inhibiting the binding between the protein of claim 5 and a ligand thereof, said method comprising the steps of:

(a) contacting the protein of claim 5 or a partial peptide thereof with the ligand in the presence of a test sample and detecting a binding activity of said protein or said partial peptide with said ligand; and
(b) selecting compounds that reduces the binding activity detected in step (a) as compared with a binding activity detected in the absence of the test sample;

**9.** A method of screening for compounds that inhibit or enhance activity of the protein of claim 5, said method comprising the steps of:

(a) contacting a ligand of said protein with cells expressing said protein in the presence of a test sample;
(b) detecting an alteration in the cells that results from binding of said ligand to said protein; and
(c) selecting compounds that suppress or enhance the alteration detected in step (b) as compared with an alteration detected in the cells in the absence of the test sample.

**10.** The method of claims 8 or 9, wherein the alteration in cells is a change in cAMP concentration or calcium concentration.

**11.** An antibody binding to the protein of claim 5.

**12.** A compound isolated by the method of any one of claim 7 to 10.

**13.** A pharmaceutical composition comprising the compound of claim 12 as an active ingredient.

**14.** The pharmaceutical composition of claim 13, wherein said pharmaceutical composition is formulated for the treatment of a disease selected from the group consisting of cancer, cirrhosis, and Alzheimer's disease.

**15.** A polynucleotide comprising at least 15 nucleotides, wherein said polynucleotide is complementary to the DNA comprising the nucleotide sequence of any one of SEQ ID NOs: 5 to 8 and 22 to 26 or a complementary strand thereof.

**16.** A method for diagnosing a disease selected from the group consisting of cancer, cirrhosis, and Alzheimer's disease, said method comprising the steps of detecting expression of the DNA of claim 1 in tissues related to the disease derived from a subject, or mutation in the DNA of claim 1 in the subject.

**17.** An agent for diagnosing a disease selected from the group consisting of cancer, cirrhosis, and Alzheimer's disease, said agent comprising the antibody of claim 11 or the nucleotide of claim 15.

Figure 1

```
>sp|P47901|V1BR_HUMAN VASOPRESSIN V1B RECEPTOR (AVPR V1B) (VASOPRESSIN V3
            RECEPTOR) (AVPR V3) (ANTIDIURETIC HORMONE RECEPTOR 1B).
            Length = 424

 Score = 316 (111.2 bits), Expect = 3.7e-41, Sum P(2) = 3.7e-41
 Identities = 70/194 (36%), Positives = 115/194 (59%)

Query:    56 LWVLFVFTIVGNSVVLFSTWRR-KKKSRMTFFVTQLAITDSFTGLVNILTDINWRFTGDF 114
             L  + V    GN  VL + + +K+SRM  FV  LA+TD    L  +L  + W  T  F
Sbjct:    41 LATVLVLATGGNLAVLLTLGQLGRKRSRMHLFVLHLALTDLAVALFQVLPQLLWDITYRF 100

Query:   115 TAPDLVCRVVRYLQVVLLYASTYVLVSLSIDRYHAIVYPMKFLQGEKQARVLIVIA-WSL 173
               PDL+CR V+YLQV+ ++ASTY+L+++++DRY A+ +P++ LQ   Q+  L++ A W L
Sbjct:   101 QGPDLLCRAVKYLQVLSMFASTYMLLAMTLDRYLAVCHPLRSLQQPGQSTYLLIAAPWLL 160

Query:   174 SFLFSIPTLIIFGKRTL--SNGEVQCWALWPDDSY-WTP--YMTIVAFLVYFIPLTIISI 228
             + +FS+P + IF  R +   +G + CWA   D  + W P  Y+T      ++ +P+T+++
Sbjct:   161 AAIFSLPQVFIFSLREVIQGSGVLDCWA---DFGFPWGPRAYLTWTTLAIFVLPVTMLTA 217

Query:   229 MYGIVIRTIW--IKSKT 243
              Y ++   I   +K KT
Sbjct:   218 CYSLICHEICKNLKVKT 234

 Score = 131 (46.1 bits), Expect = 3.7e-41, Sum P(2) = 3.7e-41
 Identities = 33/80 (41%), Positives = 47/80 (58%)

Query:   258 SSYNRGLISKAKIKAIKYSIIIILAFICCWSPYF---LFDILDNFNLLPDTQERFYASVI 314
             SS N   IS+AKI+ +K + +I+LA+I CW+P+F   ++ + D  N  PD      A  I
Sbjct:   267 SSINT--ISRAKIRTVKMTFVIVLAYIACWAPFFSVQMWSVWDK-NA-PDEDSTNVAFTI 322

Query:   315 IQNLPALNSAINPLIYCVFSSSI 337
                L  LNS  NP IY  F+S +
Sbjct:   323 SMLLGNLNSCCNPWIYMGFNSHL 345
```

Figure 2

```
>sp|P31388|5H6_RAT 5-HYDROXYTRYPTAMINE 6 RECEPTOR (5-HT-6) (SEROTONIN RECEPTOR)
           (ST-B17).
           Length = 436

 Score = 224 (78.9 bits), Expect = 6.7e-17, P = 6.7e-17
 Identities = 84/309 (27%), Positives = 144/309 (46%)

Query:     3 PGEA--LLAGLLVMVLAVALLSNALVLLCCAYSAELRTRASGVLLVNLSLGHLLLAALDM 60
             PG +  + A L V+++  A  ++ L++L C   A LR   S   LV+L    L++  + M
Sbjct:    23 PGGSGWVAAALCVVIVLTAAANSLLIVLICTQPA-LRN-TSNFFLVSLFTSDLMVGLVVM 80

Query:    61 PFTLLGVMRGRTPSAPGACQVIGFLDTFLASNAALSVAALSADQWLAVGFPLRYAGRLR- 119
             P  +L  + GR   A G C +    D    S + L++  +S D++L +  PLRY  R+
Sbjct:    81 PPAMLNALYGRWVLARGLCLLWTAFDVMCCSASILNLCLISLDRYLLILSPLRYKLRMTA 140

Query:   120 PRYAGLLLGCAWGQSLAFSGAALGCSWLGYSSAFASCSLRLPPEPERPRFAA---FTATL 176
             PR   L+LG AW  SLA    AL  S+L     +        P P + R  A   F
Sbjct:   141 PRALALILG-AW--SLA----ALA-SFLPLLLGWHELGKARTPAPGQCRLLASLPFVLVA 192

Query:   177 HAVGFVLPLAVLCLTSLQVHRVARRHCQRMDTVT-------MKALALLADLHPSVR---- 225
               V F LP   +C T  ++   AR+   ++ ++T       ++ L +     P +
Sbjct:   193 SGVTFFLPSGAICFTYCRILLAARKQAVQVASLTTGTAGQALETLQVPRTPRPGMESADS 252

Query:   226 QRCLIQQKRRRHRATRKIGIAIATFLICFAPYVMTRLAELVPFVTVNAQWGILSKCLTYS 285
             +R   +  R+  +A+  +GI +  F + + P+ +  +A+ V        + +L+  L Y
Sbjct:   253 RRLATKHSRKALKASLTLGILLGMFFVTWLPFFVANIAQAVCDCISPGLFDVLT-WLGYC 311

Query:   286 KAVADPFTYSLLRRPFRQVL 305
              +  +P  Y L  R F++ L
Sbjct:   312 NSTMNPIIYPLFMRDFKRAL 331
```

Figure 3

```
>sp|P56479|GALR_MOUSE GALANIN RECEPTOR TYPE 1 (GAL1-R) (GALR1).
            Length = 348

 Score = 269 (94.7 bits), Expect = 7.9e-24, P = 7.9e-24
 Identities = 82/289 (28%), Positives = 136/289 (47%)

Query:    49 VGFVGNLCVIGILLHNAWKGKP-SMIHSLILNLSLADLSLLLFSAPIRATAYSKSVWDLG 107
             +G +GN  VI +L  +   GKP S  + ILNLS+ADL+ LLF  P +AT Y+   W LG
Sbjct:    46 MGVLGNSLVITVLARSK-PGKPRSTTNLFILNLSIADLAYLLFCIPFQATVYALPTWVLG 104

Query:   108 WFVCKSSDWFIHTCMAAKSLTIVVVA--KVCFMYASDPAKQVSIHNYTIWSVLVAIWTVA 165
              F+CK   +F   M   T+ ++ +  + S + + +   + V  IW ++
Sbjct:   105 AFICKFIHYFFTVSMLVSIFTLAAMSVDRYVAIVHSRRSSSLRVSRNALLGVGF-IWALS 163

Query:   166 SLLPLPEWFFSTIRHHEGVE-MCLVDVPAVAEEFMSMFGKLYPL--LAFG--LPLFFASF 220
              + P +  + H + + C  P       + K Y +   FG LPL   F
Sbjct:   164 IAMASPVAYHQRLFHRDSNQTFCWEQWPN------KLHKKAYVVCTFVFGYLLPLLLICF 217

Query:   221 YFWRAYDQCKKRGTKTQNLRNQIRSKQVTVMLLSIAIISAVLWLPEWVAWLWVWHLKAAG 280
              + + +  K+ K + +++ K+  +L + ++ + WLP V LW   A
Sbjct:   218 CYAKVLNHLHKK-LKNMSKKSEASKKKTAQTVLVVVVVFGISWLPHHVVHLWAEF--GAF 274

Query:   281 PAPPQGFI--ALSQVLMFSISSANPLIFLVMSEEFREGLKGVWKWMITKKPPTVSESQE 337
             P P F  + L +S SS NP+I+ +SE FR+ K V+K + + P SE++E
Sbjct:   275 PLTPASFFFRITAHCLAYSNSSVNPIIYAFLSENFRKAYKQVFKCHVCDESPR-SETKE 332
```

Figure 4

```
>sp:NY2R_BOVIN NEUROPEPTIDE Y RECEPTOR TYPE 2 (NPY2-R).
          Length = 384

 Score =  153 bits (383), Expect = 5e-37
 Identities = 93/308 (30%), Positives = 164/308 (53%), Gaps = 7/308 (2%)

Query: 47   DEDEDVTNSRTFFAAKIVIGMALVGIMLVCGIGNFIFIAALVRYKKLRNLTNLLIANLAI 106
            D + ++ +S      ++V+ +A   I+L+  IGN + I  ++++K +R +TN  IANLA+
Sbjct: 38   DSEPELIDSTKLIEVQVVLILAYCSIILLGVIGNSLVIHVVIKFKSMRTVTNFFIANLAV 97

Query: 107  SDFLVAIVCCPFEMDYYVVRQLSWEHGHVLCTSVNYLRTVSLYVSTNALLAIAIDRYLAI 166
            +D LV  +C PF + Y ++ +  W+ G VLC  V Y + +++ VST  L  IA+DR+  I
Sbjct: 98   ADLLVNTLCLPFTLTYTLMGE--WKMGPVLCHLVPYAQGLAVQVSTITLTVIALDRHRCI 155

Query: 167  VHPLRPRMKCQTATGLIALVWTVSILIAIPSAYFTTETVLVIVKSQEKIFCGQIWPVDQQ 226
            V+ L  ++  Q +  +I L W VS L+A P A F   +++ I+   E + C + WP +++
Sbjct: 156  VYHLESKISKQISFLIIGLAWGVSALLASPLAIFREYSLIEIIPDFEIVACTEKWPGEEK 215

Query: 227  -LYYKSYFLFIFGIEFVGPVVTMTLCYARISRELWFKAVPGFQTEQIRKRLRCRRKTVLV 285
             +Y   Y L    I +V P+ ++   Y RI  +L     PG   +   +R   R+KT  +
Sbjct: 216  GIYGTIYSLSSLLILYVLPLGIISFSYTRIWSKLKNHVSPGAAHDHYHQR---RQKTTKM 272

Query: 286  LMCILTAYVLCWAPFYGFTIVRDFFPTVFVKEKHYLTAFYIVECIAMSNSMINTLCFVTV 345
            L+C++  + + W P + F +  D    V +  K Y   F +   IAM ++  N L +  +
Sbjct: 273  LVCVVVVFAVSWLPLHAFQLAVDIDSHV-LDLKEYKLIFTVFHIIAMCSTFANPLLYGWM 331

Query: 346  KNDTVKYF 353
             ++  K F
Sbjct: 332  NSNYRKAF 339
```

Figure 5

```
>sp|P97926|OXYR_MOUSE OXYTOCIN RECEPTOR (OT-R).
           Length = 388

 Score = 164 (57.7 bits), Expect = 8.9e-22, Sum P(2) = 8.9e-22
 Identities = 57/166 (34%), Positives = 84/166 (50%)

Query:    24 WGLNLTLGQGAP-------ASGPPSR-----RVRLVFLGVILVVAVAGNTTVLCRLCGGG 71
             W + L LG G P       +GPP R     RV +  L +IL +A++GN  VL  L
Sbjct:     9 WSIELDLGSGVPPGAEGNLTAGPPRRNEALARVEVAVLCLILFLALSGNACVLLAL---- 64

Query:    72 GPWAGPKRRKMDFLLVQLALADLYACGGTALSQLAWELLGEPRAATGDLACRFLQLLQAS 131
                    K  ++ F +  L++ADL        L QL W++     R    DL CR ++ LQ
Sbjct:    65 -RTTRHKHSRLFFFMKHLSIADLVVAVFQVLPQLLWDITF--RFYGPDLLCRLVKYLQVV 121

Query:   132 GRGASAHLVVLIALERRRAVRLPHGRPLPARA--LAALG-WLLALLLALPPAFV 182
             G  AS +L++L++L+R  A+  P  R L  R   LA L  WL  L+ ++P   +
Sbjct:   122 GMFASTYLLLLMSLDRCLAICQPL-RSLRRRTDRLAVLATWLGCLVASVPQVHI 174

 Score = 155 (54.6 bits), Expect = 8.9e-22, Sum P(2) = 8.9e-22
 Identities = 49/161 (30%), Positives = 85/161 (52%)

Query:   217 CHGIFAPLPRWHLQVYAFYEAVAGFVAPVTVLGVACGHLLS--VWW--RHRPQAPAAAAP 272
             C +F  +  W + Y  +  +A ++ PV VL  AC  L+S  +W   R +  A AAAA
Sbjct:   187 CWAVF--IQPWGPKAYVTWITLAVYIVPVIVLA-ACYGLISFKIWQNLRLKTAAAAAAAE 243

Query:   273 WSASPG--------RAPAPSALPRAKVQSLKMSLLLALLFVGCELPYFAARLAAAWS-SG 323
              S + G        R  +  + +AK++++KM+ ++ L F+ C  P+F  ++ + W  +
Sbjct:   244 GSDAAGGAGRAALARVSSVKLISKAKIRTVKMTFIIVLAFIVCWTPFFFVQMWSVWDVNA 303

Query:   324 PAGDWEGEGLSAALRVVAMANSALNPFVYLFFQAGDCRLRRQLRKRLGSLCCA 376
             P   E      A+ ++A  NS  NP++Y+ F        L +L +R   LCC+
Sbjct:   304 PK---EASAFIIAM-LLASLNSCCNPWIYMLFTG---HLFHELVQRF--LCCS 347
```

Figure 6

```
>sp|Q91178|GPRX_ORYLA PROBABLE G PROTEIN-COUPLED RECEPTOR (FRAGMENT).
            Length = 428

 Score = 823 (289.7 bits), Expect = 9.8e-83, P = 9.8e-83
 Identities = 182/422 (43%), Positives = 266/422 (63%)

Query:      2 ESSPIPQSSGNSSTLGRVPQTPGPSTASGVPEVGL----RDVASESVALFFMLLLDLTAV 57
              ++SP+  S  + S        P P+     P+VG+    +    +   LF M+ L+L A+
Sbjct:     5 KTSPMITSDHSISNFSTGLFGPHPTVP---PDVGVVTSSQSQMKDLFGLFCMVTLNLIAL 61

Query:     58 AGNAAVMAVIAKTPALRKFVFVFHLCLVDLLAALTLMPLAMLSSSALFDHALFGEVACRL 117
                N  VM  IA+ P L+KF FV HLC VD+L A+ LMPL ++SSS  F   +F  + C++
Sbjct:    62 LANTGVMVAIARAPHLKKFAFVCHLCAVDVLCAILLMPLGIISSSPFFGTVVFTILECQV 121

Query:    118 YLFLSVCFVSLAILSVSAINVERYYYVVHPMRYEVRMTLGLVASVLVGVWVKALAMASVP 177
              Y+FL+V  + L+IL+++AI+VERY+Y+VHPMRYEV+MT+ LV  V++ +W K+L +A V
Sbjct:   122 YIFLNVFLIWLSILTITAISVERYFYIVHPMRYEVKMTINLVIGVMLLIWFKSLLLALVT 181

Query:    178 VLGRVSWEEGAPSVPPGCSLQWSHSAYCQLFVVVFAVLYFLLPLLLILVVYCSMFRVARV 237
              + G   +   +       CSL  SHS    +F V+F V+ FL P+++I  VY ++++VAR
Sbjct:   182 LFGWPPYGHQSSIAASHCSLHASHSRLRGVFAVLFCVICFLAPVVVIFSVYSAVYKVARS 241

Query:    238 AAMQHGP-LPTWME-TP-RQRSESLSSRSTMVTSSGAPQT-TPHRTFGGGKAAVVLLAVG 293
              AA+Q  P +PTW + +P + RS+S++S++T++T+   PQ  +P R F GGKAA+ L  +
Sbjct:   242 AALQQVPAVPTWADASPAKDRSDSINSQTTIITTRTLPQRLSPERAFSGGKAALTLAFIV 301

Query:    294 GQFLLCWLPYFSFHLYVALSAQPISTGQVESVVTWIGYFCFTSNPFFYGCLNRQIRGELS 353
              GQFL+CWLP+F FHL ++L+    S G +E  V W+ Y  F  NP FYG LNRQIR EL
Sbjct:   302 GQFLVCWLPFFIFHLQMSLTGSMKSPGDLEEAVNWLAYSSFAVNPSFYGLLNRQIRDELV 361

Query:    354 K-QFVCFFKPAPEEELRLPSREGSIEENFLQFLQGTGCPSESWVSRPLPSPKQ-EPPAVD 411
              K +  C  +P    E+   S EGS +ENFLQF+Q T   SE+  S    +P+  E  A
Sbjct:   362 KFRRCCVTQPV---EIGPSSLEGSFQENFLQFIQRTSSSSETHPSFANSNPRNMENQA-- 416

Query:    412 FRIPGQIAEE 421
               +IPGQI EE
Sbjct:   417 HKIPGQIPEE 426
```

Figure 7

```
>sp|P23749|RTA_RAT PROBABLE G PROTEIN-COUPLED RECEPTOR RTA.
            Length = 343

 Score = 461 (162.3 bits), Expect = 2.3e-44, P = 2.3e-44
 Identities = 121/323 (37%), Positives = 178/323 (55%)

Query:     2 NQTLNSSGTVESALNYSRGS-TVHT-AYL----VLSSLAMFTCLCGMAGNSMVIWLLGFR 55
             NQ      G  E+    YSRG  T+    A L     V + + +  CLCG+ GN +V+W  GF
Sbjct:    13 NQNKMCPGMSEALELYSRGFLTIEQIATLPPPAVTNYIFLLLCLCGLVGNGLVLWFFGFS 72

Query:    56 MHRNPFCIYILNLAAADLLFLFSMASTLSLETQPLVNT-TDKVHELMKRLMYFAYTVGLS 114
             + R PF IY L+LA+AD ++LFS A     L      + +  D V  + + +      +  G+S
Sbjct:    73 IKRTPFSIYFLHLASADGIYLFSKAVIALLNMGTFLGSFPDYVRRVSRIVGLCTFFAGVS 132

Query:   115 LLTAISTQRCLSVLFPIWFKCHRPRHLSAWVCGLLWTLCLLMNGLTSSFCSKFL--KFNE 172
             LL AIS +RC+SV+FP+W+      RP+ LSA VC LLW L  L+  + + FC  FL  + +
Sbjct:   133 LLPAISIERCVSVIFPMWYWRRRPKRLSAGVCALLWLLSFLVTSIHNYFCM-FLGHEASG 191

Query:   173 DRCFRVDMVQAALIMGVLTPVMTLSSLTLFVWVRRSSQQWRRQPTRLFVVVLASVLVFLI 232
               C  +D+     L+  +  P+M L  L L + V    +++ R++  +L  VVLA V VFL+
Sbjct:   192 TACLNMDISLGILLFFLFCPLMVLPCLALILHVECRARR-RQRSAKLNHVVLAIVSVFLV 250

Query:   233 CSLPLSIYWFVLYWL-SLPPEMQVLCFSLSRLSSSVSSSANPVIYFLVGSRRSHRLPTRS 291
              S+ L I WF L+W+   +P           ++ L    ++SSA P++YFL G  +S RL
Sbjct:   251 SSIYLGIDWF-LFWVFQIPAPFPEY---VTDLCICINSSAKPIVYFLAGRDKSQRL-WEP 305

Query:   292 LGTVLQQALRE--EPELEGGETPTVGTNEM 319
             L  V Q+ALR+  EP       TP   T EM
Sbjct:   306 LRVVFQRALRDGAEPGDAASSTPNTVTMEM 335
```

Figure 8

```
>sp|Q98907|P2Y3_CHICK P2Y PURINOCEPTOR 3 (P2Y3) (NUCLEOSIDE DIPHOSPHATE
            RECEPTOR).
            Length = 328

 Score = 452 (159.1 bits), Expect = 2.0e-43, P = 2.0e-43
 Identities = 85/185 (45%), Positives = 116/185 (62%)

Query:    15 CQFSEKYKQVYLSLAYSIIFILGLPLNGTVLWHFWGQTKRWSCATTYLVNLMVADLLYVL 74
             C F E++KQV L L YS++F+LGLPLN  V+    W   K  +  T Y++NL +ADLLYV
Sbjct:    13 CTFHEEFKQVLLPLVYSVVFLLGLPLNAVVIGQIWLARKALTRTTIYMLNLAMADLLYVC 72

Query:    75 -LPFLIITYSLDDRWPFGELLCKLVHFLFYINLYGSILLLTCISVHQFLGVCHPLCSLPY 133
              LP LI  Y+  D WPFG+  CK V F FY NL+GSIL LTCISV +++G+CHPL S
Sbjct:    73 SLPLLIYNYTQKDYWPFGDFTCKFVRFQFYTNLHGSILFLTCISVQRYMGICHPLASWHK 132

Query:   134 RT-RRHAWLGTSTTWALVVLQLLPTLAFSHTDYINGQMIWYDMTSQENFDRLFAYGIVLT 192
             +  ++  WL   +  W +V+ Q LPT  F+ T      + + YD++   +      F YGI LT
Sbjct:   133 KKGKKLTWLVCAAVWFIVIAQCLPTFVFASTGTQRNRTVCYDLSPPDRSTSYFPYGITLT 192

Query:   193 LSGFL 197
             ++GFL
Sbjct:   193 ITGFL 197
```

Figure 9

```
>sp|O02824|A1AA_RABIT ALPHA-1A ADRENERGIC RECEPTOR (ALPHA 1A-ADRENOCEPTOR)
            (ALPHA-1C ADRENERGIC RECEPTOR).
            Length = 466

 Score = 295 (103.8 bits), Expect = 1.0e-31, Sum P(2) = 1.0e-31
 Identities = 66/215 (30%), Positives = 113/215 (52%)

Query:     8 STRESNSSHTCMPLSKMPISLAHGIIRSTVLVIFLAASFVGNIVLALVLQRKPQLLQVTN 67
             S   S+SS+   P +  P++++  I+   +L   +    +GNI++ L +     L  VT+
Sbjct:     5 SGNASDSSNCTHPPA--PVNISKAILLGVILGGLILFGVLGNILVILSVACHRHLHSVTH 62

Query:    68 RFIFNLLVTDLLQISLVAPWVVATSVPLFWPLNSHFCTALVSLTHLFAFASVNTIVVVSV 127
              +I NL V DLL  S V P+     +  +W     FC    ++  L   AS+ ++ V+S+
Sbjct:    63 YYIVNLAVADLLLTSTVLPFSAIFEILGYWAFGRVFCNIWAAVDVLCCTASIISLCVISI 122

Query:   128 DRYLSIIHPLSYPSKMTQRRGYLLLYGTWIVAILQSTPPLYGWGQAAFDERNALCSMIWG 187
             DRY+ + +PL YP+ +TQRRG   L   W  +++ S  PL+GW Q A D+   +C +
Sbjct:   123 DRYIGVSYPLRYPTIVTQRRGLRALLCVWAFSLVISVGPLFGWRQPAPDDET-ICQI--N 179

Query:   188 ASPSYTILSVVSFIVIPLIVMIACYSVVFCAARRQ 222
               P Y + S +    +PL +++A Y  V+  A+R+
Sbjct:   180 EEPGYVLFSALGSFYVPLTIILAMYCRVYVVAKRE 214

 Score = 106 (37.3 bits), Expect = 1.0e-31, Sum P(2) = 1.0e-31
 Identities = 23/75 (30%), Positives = 41/75 (54%)

Query:   396 KAAKVIFIIIFSYVLSLGPYCFLAVLAVWVDVETQVPQWVITIIIWLFFLQCCIHPYVYG 455
             KAAK + I++  +VL   P+  +  +  +   + + P+ V  I+ WL +L  CI+P +Y
Sbjct:   269 KAAKTLGIVVGCFVLCWLPFFLVMPIGSFFP-DFKPPETVFKIVFWLGYLNSCINPIIYP 327

Query:   456 YMHKTIKKEIQDMLK 470
                +  KK  Q++LK
Sbjct:   328 CSSQEFKKAFQNVLK 342
```

Figure 10

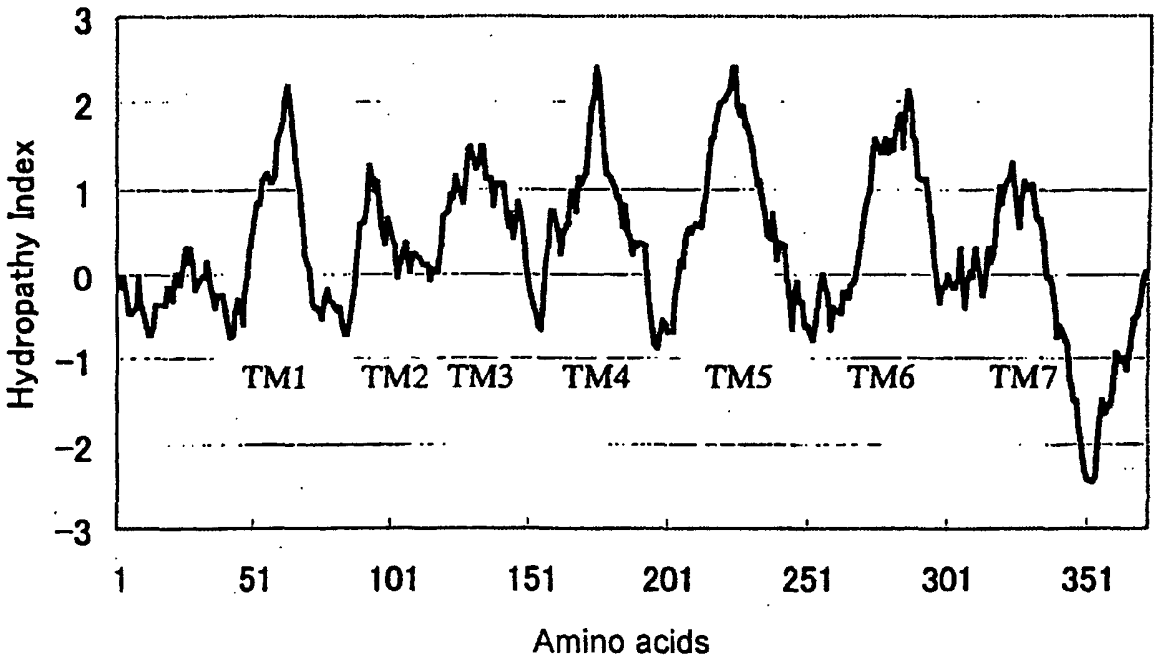
3
2
1
0
-1
-2
-3
Hydropathy Index
TM1
TM2
TM3
TM4
TM5
TM6
TM7
1
51
101
151
201
251
301
351
Amino acids

Figure 11

```
                                                                           ########
X64878             MEGALAAN---WSA-EAA-NASAAPPGAEG-----NRTAGPPRRNEALARVEVAVLCLIL
U82440             MEGELAAN---WST-EAV-NSSAAPPGAEG-----NCTAGPPRRNEALARVEVAVLCLIL
X93313             MEGLCLNL---DCS-ELP-NSSWVNSSMENQNHSSNSTRDPLKRNEEVAKVEVTVLALIL
X87783             MEEMFKEQDF-WSFNESSRNSTVGNETFGG-----NQTVNPLKRNEEVAKVEVTVLALVL
AF184966           -------------------MEKPGNITLHP------NGSDPFGRNEEVAQIEIMVLSITF
X76321             -------------------MGRIANQTTAS------NDTDPFGRNEEVAKMEITVLSVTF
AF147743           MKNFSFPMQD-STHQTESPPHRLLSLTNKS------DPVGRPERDEQLAQVEIAVLGVIF
GPRv8              MPANFTEGSFDSSGTGQTLDSSPVACTETVTFTEVVEGKEWGSFYYSFKTEQLITLWVLF
AE003754           ----------------------------------MKCDHTLFFALFQTEQFAVLWILF
                                                                  .    :. .* : :

                   TM1 ############            ######## TM2 ########
X64878             LLALSGNACVLLALRTTRQKHSRLFFFMKHLSIADLVVAVFQVLPQLLWDITFRFYGPDL
U82440             FLALSGNACVLLALRTTRHKHSRLFFFMKHLSIADLVVAVFQVLPQLLWDITFRFYGPDL
X93313             FLALAGNICVLLGIYINRHKHSRMYFFMKHLSIADLVVAIFQVLPQLIWDITFRFYAPDL
X87783             FLALAGNLCVLIAIYTAKHTQSRMYYLMKHLSIADLVVAVFQVLPQLIWDITFRFYGPDF
AF184966           VVAVIGNVSVLLAMYNTKKKMSRMHLFIKHLSLADLVVAFFQVLPQLCWEITYRFFGPDF
X76321             FVAVIGNLSVLLAMHNTKKKSSRMHLFIKHLSLADMVVAFFQVLPQLCWEITFRFYGPDF
AF147743           LTASVGNFILILVLWRRRKKLSRMYVFMLHLSIADLVVAFFQVLPQLIWDITDVFIGPDF
GPRv8              VFTIVGNSVVLFSTWR-RKKKSRMTFFVTQLAITDSFTGLVNILTDINWRFTGDFTAPDL
AE003754           TVIVLGNSAVLFVMFINKNRKSRMNYFIKQLALADLCVGLLNVLTDIIWRITISWRAGNL
                        **  :::      ::  **:  ::  :*:::*  ....::*.:: * :*  :  . ::

                    @########### TM3 ############              ########## TM4 #
X64878             LCRLVKYLQVVGMFASTYLLLLMSLDRCLAICQPLRSLRRRT--DRLAVLATWLGCLVAS
U82440             LCRLVKYLQVVGMFASTYLLLLMSLDRCLAICQPLRSLRRRT--DRLAVLATWLGCLVAS
X93313             VCRLVTYLQVVGMFASTYMLLLMSLDRCLAICQPLRSLHRRS--DCVYVLFTWILSFLLS
X87783             LCRLVKYLQTVGMFASTYMLVLMSIDRCIAICQPLRSLHKRK--DRCYVIVSWALSLVFS
AF184966           LCRIVKHLQVTGMFASTYMMVMMTLDRYIAICHPLKTLQQPTQRSYIMIVSTWMCSLVFS
X76321             LCRIVKHLQVLGMFASTYMMVMMTLDRYIAICHPLKTLQQPTQRAYIMIGSTWLCSLLLS
AF147743           LCRIIKYLQLLGMFASTYMIVVMTVDRYQAVCYPMVTFQKKRALWNIPICTSWSISLILS
GPRv8              VCRVVRYLQVVLLYASTYVLVSLSIDRYHAIVYPMKFLQGEKQ-ARVLIVIAWSLSFLFS
AE003754           ACKAIRFSQVCVTYSSTYVLVAMSIDRYDAITHPMNFSKSWKR-ARHLVAGAWLISALFS
                    *: : . *    ::***::: :::**  *:  *:   :         :  :*  . : *

                   ##########          @           ########## TM5 #############
X64878             APQVHIFSLREVADG--VFDCWAVFIQP--WGPKAYITWITLAVYIVPVIVLATCYGLIS
U82440             APQVHIFSLREVADG--VFDCWAVFIQP--WGPKAYITWITLAVYIVPVIVLAACYGLIS
X93313             TPQTVIFSLTEVGNG--VYDCRADFIQP--WGPKAYITWITLAVYIIPVMILSVCYGLIS
X87783             VPQVYIFSLREIGNG--VYDCWGDFVQP--WGAKAYITWISLTIYIIPVAILGGCYGLIS
AF184966           TPQYFIFSLSEVKNGSTVKDCWAHFIEP--WGARAYITWITGGIFLVPVVILVMCYGFIC
X76321             TPQYFIFSLSEIQNGSYVYDCWGHFIEP--WGIRAYITWITVGIFLIPVIILMICYGFIC
AF147743           LPQVFIFSKIEISPG--IFECWAEFIQP--WGPRAYVTWILVVIFFIPSTILITCQVKIC
GPRv8              IPTLIIFGKRTLSNG--EVQCWALWPDDSYWTP--YMTIVAFLVYFIPLTIISIMYGIVI
AE003754           LPILVLYEEKLIQGH---PQCWIELGSPIAWQV--YMSLVSATLFAIPALIISACYAIIV
                    *   ::    :       :*     .   *    *:: :   :: :*  ::      :
```

Figure 12

```
                                                                ######## TM6
X64878          FKIWQNLRLKTAAAAAAEAPEGAAAGDGGRVALARVSSVKLISKAKIRTVKMTFIIVLAF
U82440          FKIWQNLRLKTAAAAAAEAPEGAAAGDGGRMALARVSSVKLISKAKIRTVKMTFIIVLAF
X93313          YKIWQNIRLKTVCESNLRLST------SRRATLSRVSSVRLISKAKIRTVKMTFIIVLAY
X87783          FKIWQNFKRKTKKDQCITLTTAA----SKANALARVSSVKLVSKAKITTVKMTFVIVLAY
AF184966        HTIWKNIKYKKRKTIPGAAS------KNGLIGKNSVSSVTTISRAKLRTVKMTFVIVLAY
X76321          HSIWKNIKCK---TMRGTRNT-----KDGMIGKVSVSSVTIISRAKLRTVKMTLVIVLAY
AF147743        KIIKRNIYVKKQNEYQVTNQ--------KQVLPSRASSVNCISKAMIKTVKMTIVTVVAY
GPRv8           RTIWIKSKTYETVISNCSDG-----------KLCSSYNRGLISKAKIKAIKYSIIIILAF
AE003754        KTIWAKCSIFVPTERAGFGA----------APARRASSRGIIPRAKVKTVKMTLTIVFVF
                  *  :                               .   :.:* : ::* ::  :..:

                ################          ############ TM7 #########
X64878          IVCWTPFFFVQMWSVWDANAPK---EASAFIIVMLLASLNSCCNPWIYMLFTGHLFHELV
U82440          IVCWTPFFFVQMWSVWDANAPK---EASAFIIVMLLASLNSCCNPWIYMLFTGHLFHELV
X93313          IVCWTPFFFVQMWSVWDPNPPK---EASLFIIAMLLGSLNSCCNPWIYMLFTGHLFHDLL
X87783          IVCWTPFFFVQMWSAWDPEAPR---EAMPFIISMLLASLNSCCNPWIYMFFAGHLFHDLK
AF184966        IICWAPFFTVQMWSVWDENFQYADSENTAVTISALLASLNSCCNPWIYMIFSGHLLQDFM
X76321          IVCWAPFFIVQMWSVWDENFSWDDSENAAVTLSALLASLNSCCNPWIYMLFSGHLLYDFL
AF147743        VLCWSPFFIAQLWSVWFPSGIT---EGSAFTIIMLLGNLNSCTNPWIYMYFCGHIPY---
GPRv8           ICCWSPYFLFDILDNFNLLPDT-QERFYASVIIQNLPALNSAINPLIYCVFSSSISFP--
AE003754        IICWSPYIIFDLLQVFGQIPHS-QTNIAIATFIQSLAPLNSAANPLIYCLFSSQVFRTLS
                : **:*::  :: . :   .     .     :   *  ***. ** **  * . :

X64878          QR-----FLCCSASYLKGRRLG--ETSASKKSN-------SSSFVLSHRSSSQRSCSQPS
U82440          QR-----FLCCSASYLKGNRLG--ETSTSKKSN-------SSSFVLSHRSSSQRSCSQPS
X93313          QS-----FLCCSARYLKTQQQGS-DLSASRKSN-------SSTFVLSRKSSSQKSITQPS
X87783          QS-----LLCCSTLYLKSSQCRCDQEHDSRKSN-------CSTYVIKSTSS-QRSITQSS
AF184966        NC-----FAWCRRANADFKKED--SDSSIRRTT-------LLTKMTN-RSPTGSTGNWRD
X76321          RC-----FPCCKKPRNMLQKED--SDSSIRRNT-------LLTKLAAGRMTNDGFGSWRD
AF147743        ----------CTNKQLENTSAQ--EDSVVTGS----------IHLVD-RDPEENSTCA--
GPRv8           ----------CREQRSQDSRMT----FRERTER-------HEMQILS--KP-EF------
AE003754        RFPPFKWFTCCCKSYRNNSQQNRCHTVGRRLHNSCDSMRTLTTSLTVSRRSTNKTNARVV
                          *                                 :     .

X64878          TA----------------------------------------------------------
U82440          TA----------------------------------------------------------
X93313          TA----------------------------------------------------------
X87783          IT----------------------------------------------------------
AF184966        LDNSPK--TSIQME----------------------------------------------
X76321          PCNSRKSSQSIGLDCFCKSSQCLEHDCSRKSSQCIPLDCSRKSSQCIPLDCSRKSSQCMS
AF147743        ------------------------------------------------------------
GPRv8           ------------------------------------------------------------
AE003754        ICERPTKVVTVPAMSERRGVSLKGNTDIL-------------------------------

X64878          ---
U82440          ---
X93313          ---
X87783          ---
AF184966        ---
X76321          KES
AF147743        ---
GPRv8           ---
AE003754        ---
```

Figure 13

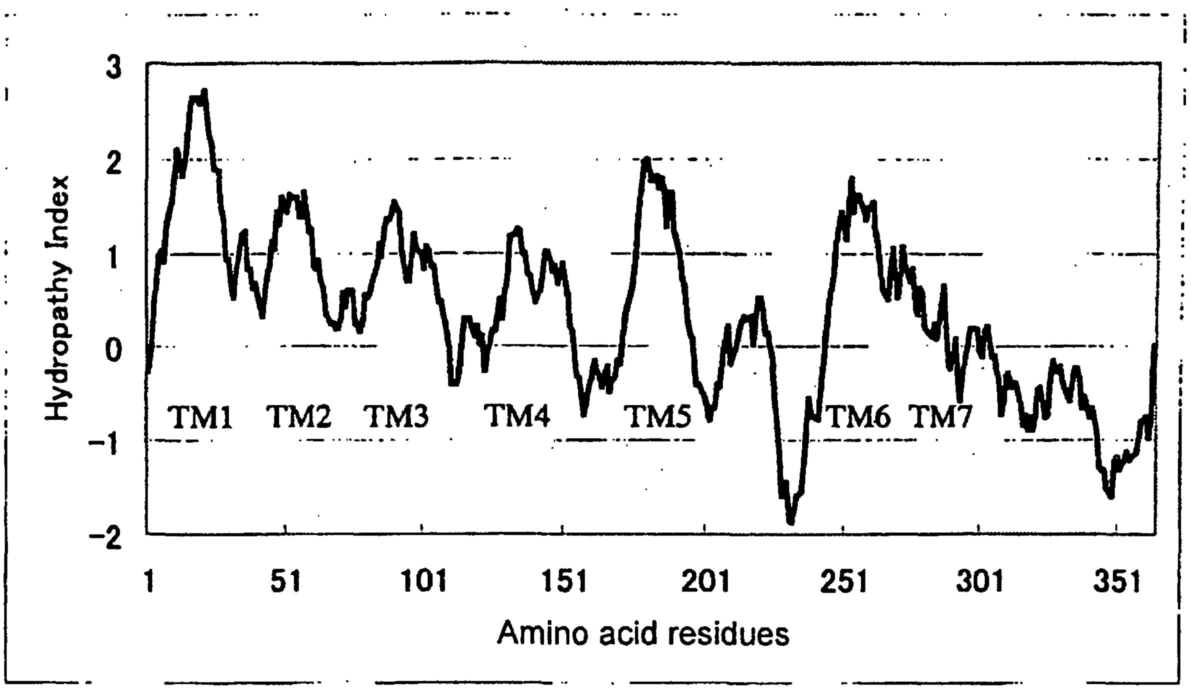
3
2
1
0
-1
-2
Hydropathy Index
TM1 TM2 TM3 TM4 TM5 TM6 TM7
1 51 101 151 201 251 301 351
Amino acid residues

Figure 14

```
                    ############ TM1 ############          ########### TM2 ###
GPRv12_ORF    MGPGEALLAGLLVMVLAVALLSNALVLLCCAYSAELRTRASGVLLVNLSLGHLLLAALDM
AF208288      MNSWDAGLAGLLVGTIGVSLLSNGLVLLCLLHSADIRRQAPALFTLNLTCGNLLCTVVNM
              *.. :* ****** .:.*:****.*****  :**::* :*..:: :**: *:** :.::*

              #########          @########### TM3 ###########
GPRv12_ORF    PFTLLGVMRGRTPSAPGACQVIGFLDTFLASNAALSVAALSADQWLAVGFPLRYAGRLRP
AF208288      PLTLAGVVAQRQPAGDRLCRLAAFLDTFLAANSMLSMAALSIDRWVAVVFPLSYRAKMRL
              *:** **:  * *:.   *:: .*******:*: **:**** *:*:** *** * .::*

                ########## TM4 ########          @            #########
GPRv12_ORF    RYAGLLLGCAWGQSLAFSGAALGCSWLGYSSAFASCSLRLPPEPERPRFAAFTATLHAVG
AF208288      RDAAFMVAYTWLHALTFPATALALSWLGFHQLYASCTLCSRRPDERLRFAVFTSAFHALS
              * *.:::. :* ::*:*..:**. ****: . :***:*      ** ***.**:::**:.

              TM5 ###########                                            ##
GPRv12_ORF    FVLPLAVLCLTSLQVHRVARRHCQRMDTVTMKALALLADLHPSVRQRCLIQQKRRRHRAT
AF208288      FLLSFIVLCFTYLKVLKVARFHCKRIDVITMQTLVLLVDIHPSVRERCLEEQKRRRQRAT
              *:*.: ***:* *:* :*** **:*:*.:**::*.**.*:*****:*** :*****:***

              ###### TM6 ###########           ########## TM7 ##########
GPRv12_ORF    RKIGIAIATFLICFAPYVMTRLAELVPFVTVNAQWGILSKCLTYSKAVADPFTYSLLRRP
AF208288      KKISTFIGTFLVCFAPYVITRLVELFSTAPIDSHWGVLSKCLAYSKAASDPFVYSLLRHQ
              :**.  *.***:******:***.**.. ..::::**:*****:****.:***.*****:


GPRv12_ORF    FRQVLAGMVHRLLKRTPRPASTHDSSLDVAGMVHQLLKRTPRPASTHNGSVDTENDSCLQ
AF208288      YRRSCKELLNRIFNRR----SIHSVGLTGDSHSQNILPVSE-------------------
              :*:     :::*:::*     * *. .*   .  :::*  :


GPRv12_ORF    QTH
AF208288      ---
```

Figure 15

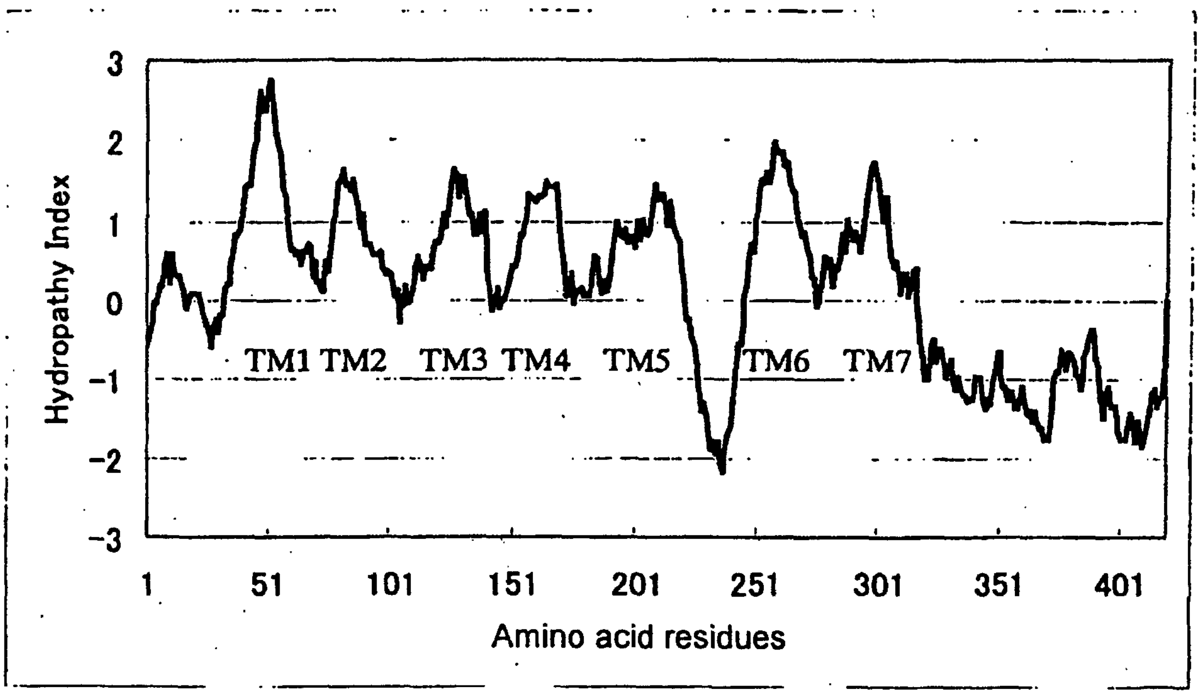
3
2
1
0
-1
-2
-3
Hydropathy Index
TM1 TM2 TM3 TM4 TM5 TM6 TM7
1
51
101
151
201
251
301
351
401
Amino acid residues

Figure 16

```
                                                        #######
                                           ########     TM1   ######
1   MLAAAFADSN SSSMNVSFAH LHFAGGYLPS DSQDWRTIIP ALLVAVCLVG FVGNLCVIGI 60

    #################################################################
    ##              #####   TM2   #####
61  LLHNAWKGKP SMIHSLILNL SLADLSLLLF SAPIRATAYS KSVWDLGWFV CKSSDWFIHT 120

    ##############                       ############################
    @##### TM3 ##########               #####    TM4   #########
121 CMAAKSLTIV VVAKVCFMYA SDPAKQVSIH NYTIWSVLVA IWTVASLLPL PEWFFSTIRH 180

    #################################################################
          @           ########   TM5   ############
181 HEGVEMCLVD VPAVAEEFMS MFGKLYPLLA FGLPLFFASF YFWRAYDQCK KRGTKTQNLR 240

    #################################################################
           ##########   TM6    ##########             ######## TM7 ##
241 NQIRSKQVTV MLLSIAIISA VLWLPEWVAW LWVWHLKAAG PAPPQGFIAL SQVLMFSISS 300

    ######
    #########
301 ANPLIFLVMS EEFREGLKGV WKWMITKKPP TVSESQETPA GNSEGLPDKV PSPESPASIP 360

361 EKEKPSSPSS GKGKTEKAEI PILPDVEQFW HERDTVPSVQ DNDPIPWEHE DQETGEGVK  419
```

Figure 17

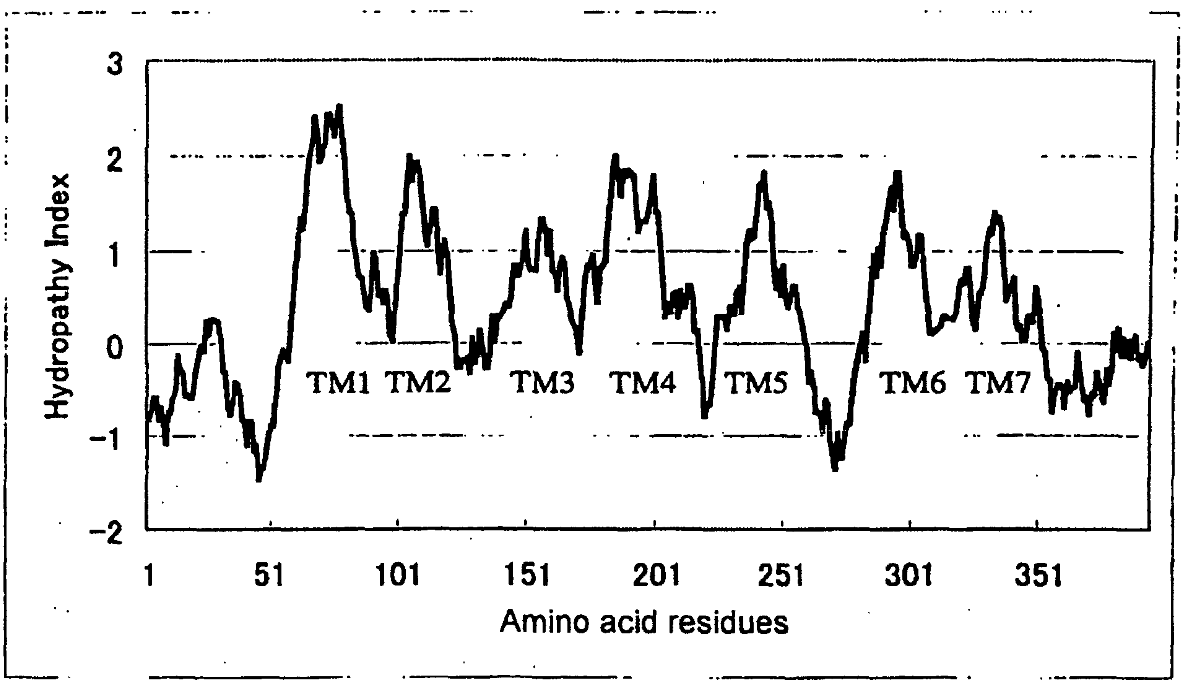
3
2
1
0
-1
-2
Hydropathy Index
TM1 TM2
TM3
TM4
TM5
TM6 TM7
1
51
101
151
201
251
301
351
Amino acid residues

Figure 18

```
GPRv21          -----------------------METTMGFMDDNATNTSTSFLSVLNPHGAHA-TSFPFN
AL121755        ------------------------------------------------------------
AF236082        -----------------------METTVGALGENTTDTFTDFFSALDGHEAQT-GSLPFT
U42766          ---------------------------MGPIGAEADENQTVEEMKVEQYGP---------
U76254          ---------------------------MGPIGAEADENQTVEEMKVEQYGP---------
U42389          ---------------------------MGPIGAEADENQTVEEMKVEQYGP---------
U50144          -------------------------MKMGPLGAEADENQTVEEMKVDQFGPG--------
D86238          -----------------------MVLKMGPVGAEADEN-QTVEVKVEPYGPG--------
M81490          MYYIAHQQPMLRNEDDNYQEGYFIRPDPASLIYNTTALPADDEGSNYGYGSTT-TLSGLQ
AF037444        --MSMANSENSTSLFGIKRHADVTGPHSASHDVIDPSNTSVYYDHASNYESVLSTTSTLM

                                                             ########### TM1
GPRv21          FSYSDYDMPL-----DEDEDVTNSR--------TFFAAKIVIGMALVGIMLVCGIGNFIF
AL121755        --YGDYDLPM-----DEDEDMTKTR--------TFFAAKIVIGIALAGIMLVCGIGNFVF
AF236082        FSYGDYDMPL-----DEEEDVTNSR--------TFFAAKIVIGMALVGIMLVCGIGNFIF
U42766          QTTPRGELVP-----DPEPELIDST--------KLIEVQVVLILAYCSIILLGVIGNSLV
U76254          QTTPRGELVP-----DPEPELIDST--------KLIEVQVVLILAYCSIILLGVIGNSLV
U42389          QTTPRGELVP-----DPEPELIDST--------KLIEVQVVLILAYCSIILLGVIGNSLV
U50144          HTTLPGELAP-----DSEPELIDST--------KLIEVQVVLILAYCSIILLGVIGNSLV
D86238          HTTPRGELPP-----DPEPELIDST--------KLVEVQVILILAYCSIILLGVVGNSLV
M81490          FETYNITVMMNFSCDDYDLLSEDMW--------SSAYFKIIVYMLYIPIFIFALIGNGTV
AF037444        LKLTDLVTPFNASEPDPESNGSDTDGGHAAISEQPMYAKVIIVLMYVLIILVAVGGNLLF
                               * :     .                 :::: :    *::.   **  .
                #######     ####### TM2 ########                         @######
GPRv21          IAALVRYKKLRNLTNLLIANLAISDFLVAIVCCPFEMDYYVVRQLSWEHGHVLCTSVNYL
AL121755        IAALTRYKKLRNLTNLLIANLAISDFLVAIICCPFEMDYYVVRQLSWEHGHVLCASVNYL
AF236082        ITALARYKKLRNLTNLLIANLAISDFLVAIVCCPFEMDYYVVRQLSWEHGHVLCASVNYL
U42766          IHVVIKFKSMRTVTNFFIANLAVADLLVNTLCLPFTLTYTLMG--EWKMGPVLCHLVPYA
U76254          IHVVIKFKSMRTVTNFFIANLAVADLLVNTLCLPFTLTYTLMG--EWKMGPVLCHLVPYA
U42389          IHVVIKFKSMRTVTNFFIANLAVADLLVNTLCLPFTLTYTLMG--EWKMGPVLCHLVPYA
U50144          IHVVIKFKSMRTVTNFFIANLAVADLLVNTLCLPFTLTYTLMG--EWKMGPVLCHLVPYA
D86238          IHVVIKFKSMRTVTNFFIANLAVADLLVNTLCLPFTLTYTLMG--EWKMGPVLCHLVPYA
M81490          CYIVYSTPRMRTVTNYFIASLAIGDILMSFFCEPSSFISLFILN-YWPFGLALCHFVNYS
AF037444        SYVIVMYPKMRSVTNLFLLNLAISDIVKAVICNPFAFIANLILL-YWPYGEFMCQVVTYI
                   :     :*.:** :: .**:.*::   .* *  :   .:     *  *  :*  * *

                ## TM3 ##########                ######### TM4 ##########
GPRv21          RTVSLYVSTNALLAIAIDRYLAIVHPLRPRMKCQTATGLIALVWTVSILIAIPSAYFTTE
AL121755        RTVSLYVSTNALLAIAIDRYLAIVHPLKPRMNYQTASFLIALVWMVSILIAIPSAYFATE
AF236082        RTVSLYVSTNALLAIAIDRYLAIVHPLRPRMKCQTAAGLIFLVWSVSILIAIPAAYFTTE
U42766          QGLAVQVSTITLTVIALDRHRCIVYHLESKISKRISFLIIGLAWGISALLASPLAIFREY
U76254          QGLAVQVSTITLTVIALDRHRCIVYHLESKISKRISFLIIGLAWGISALLASPLAIFREY
U42389          QGLAVQVSTITLTVIALDRHRCIVYHLESKISKRISFLIIGLGWRISALLASPLAIFREY
U50144          QGLAVQVSTITLTVIALDRHRCIVYHLESKISKQISFLIIGLAWGVSALLASPLAIFREY
D86238          QGLAVQVSTITLTVIALDRHRCIVYHLESKISKRISFLIIGLAWGISALLASPLAIFREY
M81490          QAVSVLVSAYTLVAISIDRYIAIMWPLKPRITKRYATFIIAGVWFIALATALPIPIVSGL
AF037444        QVVAVFLSAFTLVAMSVDRYVAILKPMRPRLSKRAFAITMATIWILSLSAPLPTAITSRV
                : ::: :*: :* .:::**: .*:  :..::  :        :   * ::    . * .
```

Figure 19

```
                         @          ######### TM5 #############
GPRv21      TVLVIVKSQ--EKIFCGQIWPVDQQ-LYYKSYFLFIFGIEFVGPVVTMTLCYARISRELW
AL121755    TVLFIVKSQ--EKIFCGQIWPVDQQ-LYYKSYFLFIFGVEFVGPVVTMTLCYARISRELW
AF236082    TVLVIVERQ--EKIFCGQIWPVDQQ-FYYRSYFLLVFGLEFVGPVVAMTLCYARVSRELW
U42766      SLIEIIPDF--EIVACTEKWPGEEKSIYGTVYSLSSLLILYVLPLGIISFSYTRIWSKLK
U76254      SLIEIIPDF--EIVACTEKWPGEEKSIYGTVYSLSSLLILYVLPLGIISFSYTRIWSKLK
U42389      SLIEIIPDF--EIVPCTEKWPAEEKSIYGTVYSLSSLLILYVLPLGIISFSYTRIWSKLK
U50144      SLIEIIPDF--EIVACTEKWPGEEKGIYGTIYSLSSLLILYVLPLGIISFSYTRIWSKLK
D86238      SLIEIIPDF--EIVACTEKWPGEEKSVYGTVYSLSTLLILYVLPLGIISFSYTRIWSKLR
M81490      DIPMSPWHTKCEKYICREMWPSRSQ---EYYYTLSLFALQFVVPLGVLIFTYARITIRVW
AF037444    TKQSNSTGL------CLEHFENDHN---RYIYSIVIMMLQYFVPLAVITVTNTHIGYIVW
                         * : :    :      * :  : : :. *:  : .  :::   :

                                    ######### TM6 #######
GPRv21      FKAVPG-FQTEQIRKRLRCRRKTVLVLMCILTAYVLCWAPFYGFTIVRDFFPTVFVKEKH
AL121755    FKAVPG-FQTEQIRKRLRCRRKTVLVLMCILTAYVLCWAPFYGFTIVRDFFPTVFVKEKH
AF236082    FKAVPG-FQTEQIRRTVRCRRRTVLGLVCVLSAYVLCWAPFYGFTIVRDFFPSVFVKEKH
U42766      NHVSPG-AANDHYHQR---RQKTTKMLVCVVVVFAVSWLPLHAFQLAVD-IDSQVLDLKE
U76254      SHVSPG-AANDHYHQR---RQKTTKMLVCVVVVFAVSWLPLHAFQLAVD-IDSQVLDLKE
U42389      NHVSPG-AANDHYHQR---RQKTTKMLVCVVVVFAVSWLPLHAFQLAVD-IDSQVLDLKE
U50144      NHVSPG-AAHDHYHQR---RQKTTKMLVCVVVVFAVSWLPLHAFQLAVD-IDSHVLDLKE
D86238      NHVSPG-AASDHYHQR---RHKMTKMLVCVVVVFAVSWLPLHAFQLAVD-IDSHVLDLKE
M81490      AKRPPGEAETNRDQRMARSKRKMVKMMLTVVIVFTCCWLPFNILQLLLN--DEEFAHWDP
AF037444    IKKTPGEAEEDRDRRMAASKRRLVKMIIIVVVIYAVCWLPVHVITLVGD-HNPDIYNQPH
             :   **   :: ::    ::: .  :: ::  :. .* *.  : :  :      . .

                ######## TM7 #########
GPRv21      YLTAFYIVECIAMSNSMINTLCFVTVKNDTVKYFKKIML---------LHWKASYNGGKS
AL121755    YLTAFYVVECIAMSNSMINTVCFVTVKNNTMKYFKKMML---------LHWRPSQRGSKS
AF236082    YLTAFYVVECIAMSNSMINTLCFVTVRNNTSKYLKRILR---------LQWRASPSGSKA
U42766      YKLIFTVFHIIAMCSTFANPLLYGWMNSNYRKAFLSAFR---------CEQRLDAIHSEV
U76254      YKLIFTVFYIIAMCSTFANPLLYGWMNSNYRKAFLSAFR---------CEQRLDAIHSEV
U42389      YKLIFTVFHIIAMCSTFANPLLYGWMNSNYRKAFLSAFR---------CEQRLDAIHSEV
U50144      YKLIFTVFHIIAMCSTFANPLLYGWMNSNYRKAFLSAFR---------CEQRLDAIHSEV
D86238      YKLIFTVFHIIAMCSTFANPLLYGWMNSNYRKAFLSAFR---------CEQRLDAIHSEV
M81490      LPYVWFAFHWLAMSHCCYNPIIYCYMNARFRSGFVQLMHRMPGLRRWCCLRSVGDRMNAT
AF037444    MNVVWLCAQWLAMSHSCYNPFVYFSLSATFRRNLRRMTHACRLKQKR-LRQHLSMRSSRA
                :     :**.    *.. :  :       :                    .   .

GPRv21      S-----------ADLDLKTIGM---PATEEVDCIRLK-------------------
AL121755    S-----------ADLDLRTNGV---PTTEEVDCIRLK-------------------
AF236082    S-----------ADLDLRTTGI---PATEEVDCIRLK-------------------
U42766      SVTFKAK-----KNLEVRKNSG---PNDSFTEATNV--------------------
U76254      SVTFKAK-----KNLEVRKNSG---PNDSFTEATNV--------------------
U42389      SVTFKAK-----KNLEVRKNSG---PNDSFTEATNV--------------------
U50144      SVTFKAK-----KHLQVTKNNG---PNDSFTETTNV--------------------
D86238      SMTFKAK-----KNLEVKKNNG---PTDSFSEATNV--------------------
M81490      SGTGPALPLN--RMNTSTTYISARRKPRATSLRANPLSCGETSPLR----------
AF037444    DAWDRDTEVYGSAESIPSKVSAGSLHSSNRGAKHVNTSSGEWQCLKEKKLKGVSNDMYL
            .                         .
```

Figure 20

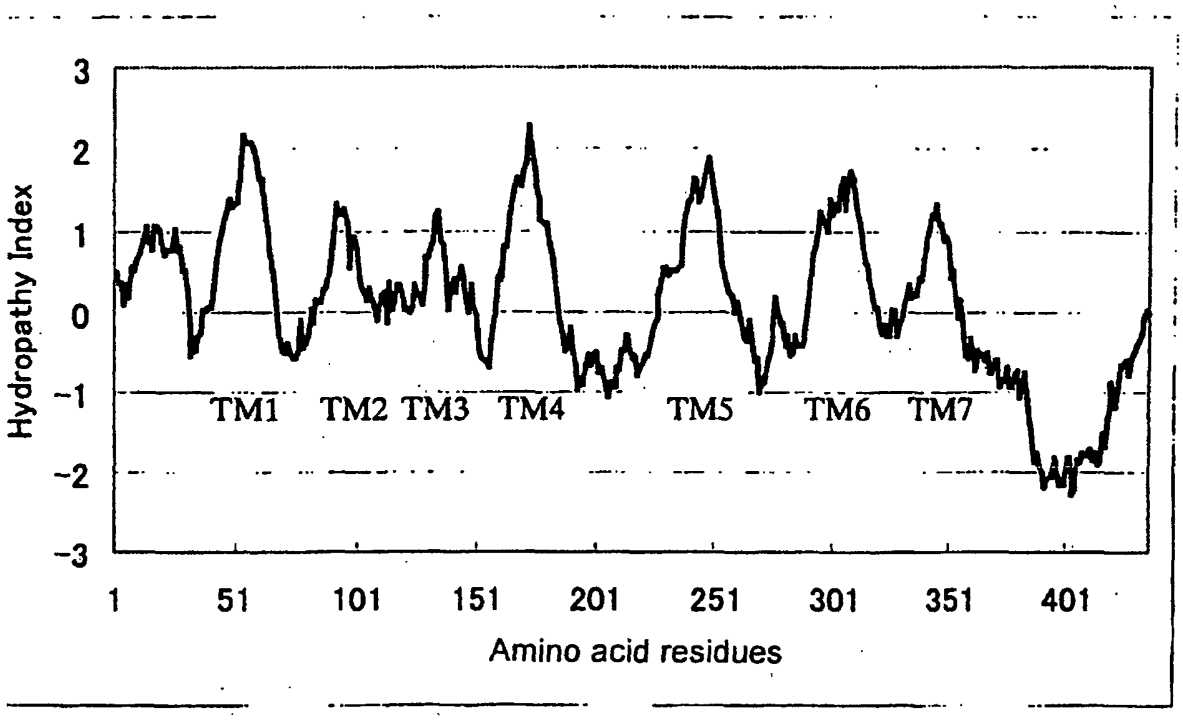
3
2
1
0
-1
-2
-3
Hydropathy Index
TM1
TM2
TM3
TM4
TM5
TM6
TM7
1
51
101
151
201
251
301
351
401
Amino acid residues

Figure 21

```
                                                               **
                                                   #######  TM1 ##
1   MEDLFSPSIL PPAPNISVPI LLGWGLNLTL GQGAPASGPP SRRVRLVFLG VILVVAVAGN 60

    ****************************************************************
    ####                   ########  TM2  #########
61  TTVLCRLCGG GGPWAGPKRR KMDFLLVQLA LADLYACGGT ALSQLAWELL GEPRAATGDL 120

    ****************************************************************
     @ #####   TM3   ##########                        #######  TM4  ####
121 ACRFLQLLQA SGRGASAHLV VLIALERRRA VRLPHGRPLP ARALAALGWL LALLLALPPA 180

    *
                                                      **************
    ###                                     @           ######### TM5
181 FVVRGDSPSP LPPPPPPTSL QPGAPPAARA WPGQRRCHGI FAPLPRWHLQ VYAFYEAVAG 240

    ****************************************************************
    ############                                            #####
241 FVAPVTVLGV ACGHLLSVWW RHRPQAPAAA APWSASPGRA PAPSALPRAK VQSLKMSLLL 300

    ********************************************************
    # TM6 #########                    #######  TM7   ##########
301 ALLFVGCELP YFAARLAAAW SSGPAGDWEG EGLSAALRVV AMANSALNPF VYLFFQAGDC 360

361 RLRRQLRKRL GSLCCAPQGG AEDEEGPRGH QALYRQRWPH PHYHHARREP LDEGGLRPPP 420

421 PRPRPLPCSC ESAF
```

Figure 22

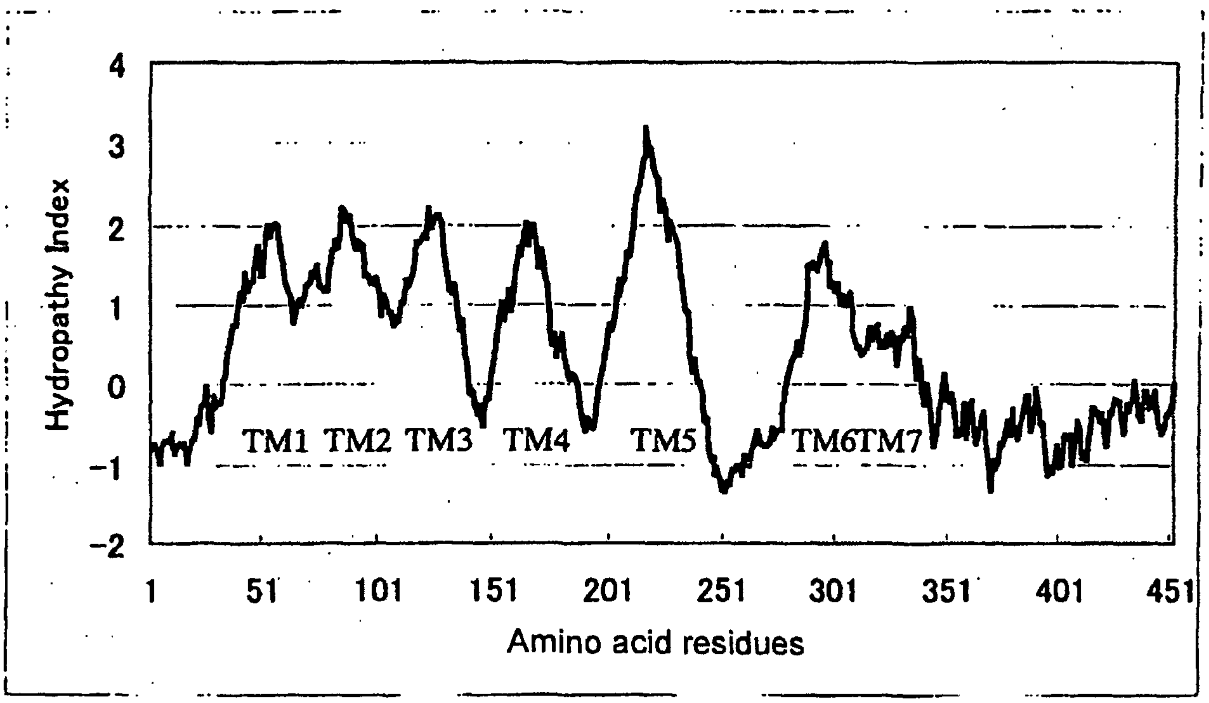
4
3
2
1
0
-1
-2
Hydropathy Index
TM1 TM2 TM3 TM4 TM5 TM6TM7
1 51 101 151 201 251 301 351 401 451
Amino acid residues

Figure 23

```
HSH2R_1    ------------------------------------------------------------
D49783     ------------------------------------------------------------
M32701     ------------------------------------------------------------
U25440     ------------------------------------------------------------
S57565     ------------------------------------------------------------
S73473     ------------------------------------------------------------
M74716     ------------------------------------------------------------
U64032     MTFRDLLSVTFEGPRPDISAGGSGAGGGAGAGAGAGDTASSESPAVGGVPGAAGGGGGGS
L41147     ------------------------------------------------------------
GPRv47     ------------------------------------------------------------
D43633     ------------------------------------------------------------

                                                                    ###
HSH2R_1    ---------------------NG---------TASSFCLDSTACKIT----------ITV
D49783     --------------MAP----NG---------TASSFCLDSTACKIT----------ITV
M32701     --------------MIS----NG---------TGSSFCLDSPPCRIT----------VSV
U25440     --------------MAF----NG---------TVPSFCMDFTVYKVT----------ISV
S57565     --------------MEP----NG---------TVHSCCLDSMALKVT----------ISV
S73473     --------------MAPWPHKNGS-----LAFWSDAPTLDPSAANTSGLPG-VPW-AAAL
M74716     --------------MAPWPHKNGS-----LAFWSDAPTLDPSAANTSGLPG-VPW-AAAL
U64032     VVGAGSGEDNRSSAGEPGGAGGGGE----VNGTAAVGGLVVSAQSVG----------VGV
L41147     --------------MVP---EPGPT----ANSTPAWGAGPPSAPGGSG--------WVAA
GPRv47     ------------MESSPIPQSSGNSSTLGRVPQTPGPSTASGVPEVGLRDV-ASESVALF
D43633     ---------MMADKTSPMITSDHSISNFSTGLFGPHPTVPPDVGVVTSSQSQMKDLFGLF

           ### TM1 ########                          ####### TM2 ######
HSH2R_1    VLAVLILITVAGNVVVCLAVGLNRRLRNLTNCFIVSLAITDLLLGLLVLPFSAIYQLSCK
D49783     VLAVLILITVAGNVVVCLAVGLNRRLRNLTNCFIVSLAITDLLLGLLVLPFSAIYQLSCK
M32701     VLTVLILITIAGNVVVCLAVGLNRRLRSLTNCFIVSLAITDLLLGLLVLPFSAFYQLSCR
U25440     ILIILILVTVAGNVVVCLAVGLNRRLRSLTNCFIVSLAVTDLLLGLLVLPFSAIYQLSCK
S57565     VLTTLILITIAGNVVVCLAVSLNRRLRSLTNCFIVSLAATDLLLGLLVLPFSAIYQLSFT
S73473     AGALLALATVGGNLLVITAIARTPRLQTITNVFVTSLATADLVVGLLVMPPGATLALTGH
M74716     AGALLALATVGGNLLVITAIARTPRLQTITNVFVTSLATADLVVGLLVMPPGATLALTGH
U64032     FLAAFILTAVAGNLLVILSVACNRHLQTVTNYFIVNLAVADLLLSATVLPFSATMEVLGF
L41147     ALCVVIALTAAANSLLIALICTQPALRNTSNFFLVSLFTSDLMVGLVVMPPAMLNALYGR
GPRv47     FMLLLDLTAVAGNAAVMAVIAKTPALRKFVFVFHLCLVDLLAALTLMPLAMLSSSALFDH
D43633     CMVTLNLIALLANTGVMVAIARAPHLKKFAFVCHLCAVDVLCAILLMPLGIISSSPFFGT
              .   :  .*  :   :     *:.                  :   :    .

           ###     @######### TM3 ########                     ##### TM4
HSH2R_1    WSFGKVFCNIYTSLDVMLCTASILNLFMISLDRYCAVMDPLRYPVLVTPARVAISLVLIW
D49783     WSFGKVFCNIYTSLDVMLCTASILNLFMISLDRYCAVMDPLRYPVLVTPVRVAISLVLIW
M32701     WSFGKVFCNIYTSLDVMLCTASILNLFMISLDRYCAVTDPLRYPVLITPVRVAVSLVLIW
U25440     WSFSKVFCNIYTSLDVMLCTASILNLFMISLDRYCAVTDPLRYPVLITPARVAISLVFIW
S57565     WSFGHVFCNIYTSLDVMLCTASILNLFMISLDRYCAVTDPLRYPVLVTPVRVAISLVF1W
S73473     WPLGATGCELWTSVDVLCVTASIETLCALAVDRYLAVTNPLRYGTLVTKRRARAAVVLVW
M74716     WPLGATGCELWTSVDVLCVTASIETLCALAVDRYLAVTNPLRYGTLVTKRRARAAVVLVW
U64032     WAFGRAFCDVWAAVDVLCCTASILSLCTISVDRYVGVRHSLKYPAIMTERKAAAILALLW
L41147     WVLARGLCLLWTAFDVMCCSASILNLCLISLDRYLLILSPLRYKLRMTPLRALALVLGAW
GPRv47     ALFGEVACRLYLFLSVCFVSLAILSVSAINVERYYYVVHPMRYEVRMTLGLVASVLVGVW
D43633     VVFTILECQVYIFLNVFLIWLSILTITAISVERYFYIVHPMRYEVKMTINLVIGVMLLIW
             :    * ::  ..*    :* .:  : ::**  :  .::*   :*   .   :   *
```

Figure 24

```
                #############                     @           ####### TM5 ########
HSH2R_1         VISITLSFLSIHLGWN---SRNETSKGNHTTSKCN-----VQVNEVYGLVDGLVTFYLPLLIMCI
D49783          VISITLSFLSIHLGWN---SRNETSKGNHTTSKCK-----VQVNEVYGLVDGLVTFYLPLLIMCI
M32701          VISITLSFLSIHLGWN---SRNETSSFNHTIPKCK-----VQVNLVYGLVDGLVTFYLPLLVMCI
U25440          VISITLSFLSIHLGWN---SRNETSKDNDTIVKCK-----VQVNEVYGLVDGLVTFYLPLLIMCI
S57565          VISITLSFLSIHLGWN---SRNGTRGGN-DTFKCK-----VQVNEVYGLVDGLVTFYLPLLIMCV
S73473          IVSATVSFAPIMSQWWRVGADAEAQECHSNPRCCS-----FASNMPYALLSSSVSFYLPLLVMLF
M74716          IVSATVSFAPIMSQWWRVGADAEAQECHSNPRCCS-----FASNMPYALLSSSVSFYLPLLVMLF
U64032          AVALVVS-MGPLLGWK------EPVPPD--ERFC-----GITEEVGYAVFSSLCSFYLPMAVIVV
L41147          SLAALASFLPLLLGWH-----ELGHARPPVPGQC-----RLLASLPFVLVASGLTFFLPSGAICF
GPRv47          VKALAMASVPVLGRVS-WEEGAPSVPPG-----CSLQWSHSAYCQLFVVVFAVLYFLLPLLLILV
D43633          FKSLLLA-LVTLFGWPPYGHQSSIAASH-----CSLHASHSRLRGVFAVLFCVICFLAPVVVIFS
                  :   :                           *           : :.      *  *   :

                #
HSH2R_1         TYYRIFRVARDQAKRID-HIS------------SWKAATIR----------------
D49783          TYYRIFKVARDQAKRIN-HIS------------SWKAATIR----------------
M32701          TYYRIFKIARDQAKRIH-HMG------------SWKAATIG----------------
U25440          TYFRIFKIAREQARRIN-HIG------------SWKAATIR----------------
S57565          TYYRIFKIAREQAKRIN-HIS------------SWKAATIR----------------
S73473          VYARVFVVAKRQRRLLRRELGRFPPEESPRSPSRSPSPATVGTPTAS-----DGVPSCGR
M74716          VYARVFVVAKRQRRFVRRELGRFPPEESPRSPSRSPSPATVGTPTAS-----DGVPSCGR
U64032          MYCRVYVVARSTTRSLEAGVKRERGKASEVVLRIHCRGAASGADGAPGTRGAKGHTFRSS
L41147          TYCRILLAARKQAVQVASLTTG---------MASQASETLQVPRTP--R--PGVESADS
GPRv47          VYCSMFRVARVAAMQHGPLPTWMETP--------RQRSESLSSR--S-----TMVTSSGA
D43633          VYSAVYKVARSAALQQVPAVPTWADAS-----PAKDRSDSINSQTT------IITTRTLP
                 *  :   *:                              :
                              ######### TM6 #######          ############
HSH2R_1         ----------EHRATVTLAAVMGAFIICWFPYFTAFVYRGLRGDDAINEMLEAIVLWLGY
D49783          ----------EHKATVTLAAVMGAFIICWFPYFTAFVYRGLRGDDAINEVLEAIVLWLGY
M32701          ----------EHKATVTLAAVMGAFIICWFPYFTVFVYRGLKGDDAINEAFEAVVLWLGY
U25440          ----------EHKATVTLAAVMGAFIICWFPYFTVFVYRGLKGDDAVNEVFEDVVLWLGY
S57565          ----------EHKATVTLAAVMGAFIICWFPYFTAFVYRGLRGDDAINEAVEGIVLWLGY
S73473          RPARLLPLG-EHRALRTLGLIMGIFSLCWLPFFLANVLRALVGPSLVPSGVFIALNWLGY
M74716          RPARLLPLG-EHRALRTLGLIMGIFSLCWLPFFLANVLRALVGPSLVPSGVFIALNWLGY
U64032          LSVRLLKFSREKKAAKTLAIVVGVFVLCWFPFFFVLPLGSLFPQLKPSEGVFKVIFWLGY
L41147          RRLATKHSRKALKASLTLGILLGMFFVTWLPFFVANIVQAVC—DCISPGLFDVLTWLGY
GPRv47          PQTTPHRTFGGGKAAVVLLAVGGQFLLCWLPYFSFHLYVALSAQPISTGQVESVVTWIGY
D43633          QRLSPERAFSGGKAALTLAFIVGQFLVCWLPFFIFHLQMSLTGSMKSPGDLEEAVNWLAY
                            :*  .*  : * * : *:*:*      .:          .   : *:.*

                TM7 #########
HSH2R_1         ANSALNPILYAALNRDFRTGYQQLFCCRLANRNSHKTSLRSNASQLSRTQSREPR----Q
D49783          ANSALNPILYAALNRDFRTGYQQLFCCRLANRNSHKTSLRSNASQLSRTQSREPR----Q
M32701          ANSALNPILYATLNRDFRTAYQQLFRCRPASHNAQETSLRSNSSQLARNQSREPM----R
U25440          ANSALNPILYAALNRDFRTAYHQLFCCRLASHNSHETSLRLNNSQLNRSQCQEPR----W
S57565          ANSALNPILYAALNRDFRTAYQQLFHCKFASHNSHKTSLRLNNSLLPRSQSREGR----W
S73473          ANSAFNPLIYCR-SPDFRDAFRRLL-CSYGGRGPEEP--RVVTFPASPVASR--------
M74716          ANSAFNPLIYCR-SPDFRDAFRRLL-CSYGGRGPEEP--RVVTFPASPVASR--------
U64032          FNSCVNPLIYPCSSREFKRAFLRLLRCQCRRRRRRRPLWRVYGHHWRASAGGGPHPDCAL
L41147          CNSTMNPIIYPLFMRDFKRALGRFLPCPRCPRERQAS-LASPSLRTSHSGPRPGLS---L
GPRv47          FCFTSNPFFYGCLNRQIRGELSKQFVCFFKPAPEEELRLPSREGSIEENFLQ-------F
D43633          SSFAVNPSFYGLLNRQIRDELVKFRRCCVTQPVEIGP--SSLEGSFQENFLQ-------F
                     ** :*      :::      :   *  :
```

Figure 25

```
HSH2R_1     QEEKPLKLQVWSGTEVT-------------------------------------------
D49783      QEEKPLKLQVWSGTEVTAPQGATDR-----------------------------------
M32701      QEEKPLKLQVWSGTEVTAPRGATDR-----------------------------------
U25440      QEDKPLNLQVWSGTEVTAPQGATNR-----------------------------------
S57565      QEEKPLKLQVWSGTELTHPQGNPIR-----------------------------------
S73473      QNS-PLNR--FDGYEGERP-FPT-------------------------------------
M74716      QNS-PLNR--FDGYEGERP-FPT-------------------------------------
U64032      SAGAALPGAALALTAAPAPSSAAAPEGQAAGAGRRKPPCAFREWRLLGPLRRPTTQLRAK
L41147      QQVLPLPLPPDSDSDSDAGSGGSSGLRLTAQLLLPGEATQDPPLPTRAAAAVNFFNIDPA
GPRv47      LQGTGCPSESWVSRPLPSPKQEPPAVDFRIPGQIAEETSEFLEQQLTSDIIMSDSYLRPA
D43633      IQRTSSSSETHPSFANSNP-RNMENQAHKIPGQIPEEQA---------------------

HSH2R_1     -----------------------------------------------------------
D49783      -----------------------------------------------------------
M32701      -----------------------------------------------------------
U25440      -----------------------------------------------------------
S57565      -----------------------------------------------------------
S73473      -----------------------------------------------------------
M74716      -----------------------------------------------------------
U64032      VSSLSHKIRAGGAQRAEAACALRSEVEAVALSVARDVAEDNTCQAYELADYRNLRETDI
L41147      EPELRPHPLGIPTN---------------------------------------------
GPRv47      ASPRLES----------------------------------------------------
D43633      -----------------------------------------------------------
```

Figure 26

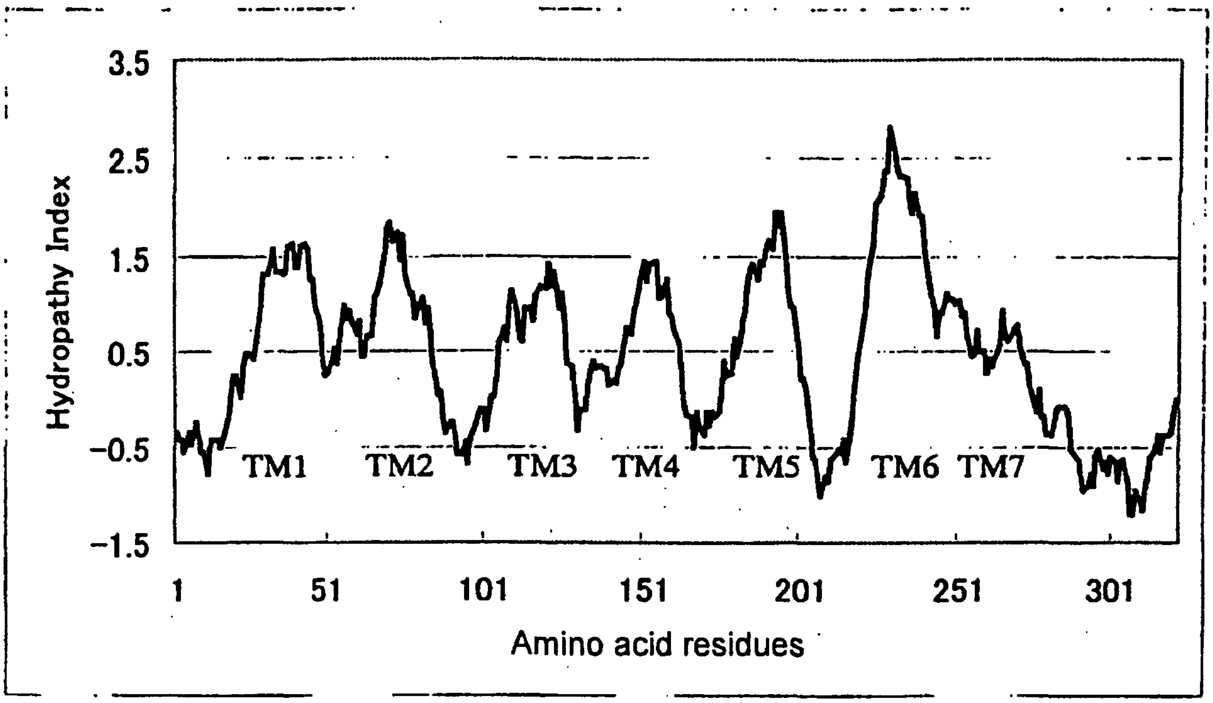
3.5
2.5
1.5
0.5
-0.5
-1.5
Hydropathy Index
TM1
TM2
TM3
TM4
TM5
TM6
TM7
1
51
101
151
201
251
301
Amino acid residues

Figure 27

```
                                                                    ########## TM1 #
GPRv51          -----------------MNQTLNSSGTVESALNYSRGSTVHTAYLVLSSLAMFTCLCGMA
M35297          MAGNCSWEAHSTNQNKMCPGMSEALELYSRGFLTIEQIATLPPPAVTNYIFLLLCLCGLV
                            **.  ..*  *:   ****  .     .*..      : :: ****:.

                ######                ######## TM2 #########
GPRv51          GNSMVIWLLGFRMHRNPFCIYILNLAAADLLFLFSMASTLSLETQPLVN-TTDKVHELMK
M35297          GNGLVLWFFGFSIKRTPFSIYFLHLASADGIYLFSKAVIALLNMGTFLGSFPDYVRRVSR
                **.:*:*::** ::*.**.**:*:**:** ::*** *    *:  .::.  .* *:.: :

                   ######## TM3 ########                ######## TM4 #######
GPRv51          RLMYFAYTVGLSLLTAISTQRCLSVLFPIWFKCHRPRHLSAWVCGLLWTLCLLMNGLTSS
M35297          IVGLCTFFAGVSLLPAISIERCVSVIFPMWYWRRRPKRLSAGVCALLWLLSFLVTSIHNY
                 :   :: .*:***.*** :**:**:**:*:  :**::*** **.*** *.:*:..: .

                #                 ######### TM5 ########
GPRv51          FCSKFLKFNE-DRCFRVDMVQAALIMGVLTPVMTLSSLTLFVWVRRSSQQWRRQPTRLFV
M35297          FCMFLGHEASGTACLNMDISLGILLFFLFCPLMVLPCLALILHVECRARRRQRS-AKLNH
                **  : :  .   *:.:*:  . *:: :: *:*.*..*:*:: *.  ::: :*. ::*

                  ######## TM6 #########             ######### TM7 #######
GPRv51          VVLASVLVFLICSLPLSIYWFVLYWLSLPPEMQVLCFSLSRLSSSVSSSANPVIYFLVGS
M35297          VVLAIVSVFLVSSIYLGIDWFLFWVFQIP---APFPEYVTDLCICINSSAKPIVYFLAGR
                **** * ***:.*: *.* **::: :.:*     :   :: *. .:.***:*::***.*

GPRv51          RRSHRLPTRSLGTVLQQALRE--EPELEGGETPTVGTNEMGA------
M35297          DKSQRLWEP-LRVVFQRALRDGAEPGDAASSTPNTVTMEMQCPSGNAS
                 :*:**    * .*:*:***: **   ...**.. * ** .
```

Figure 28

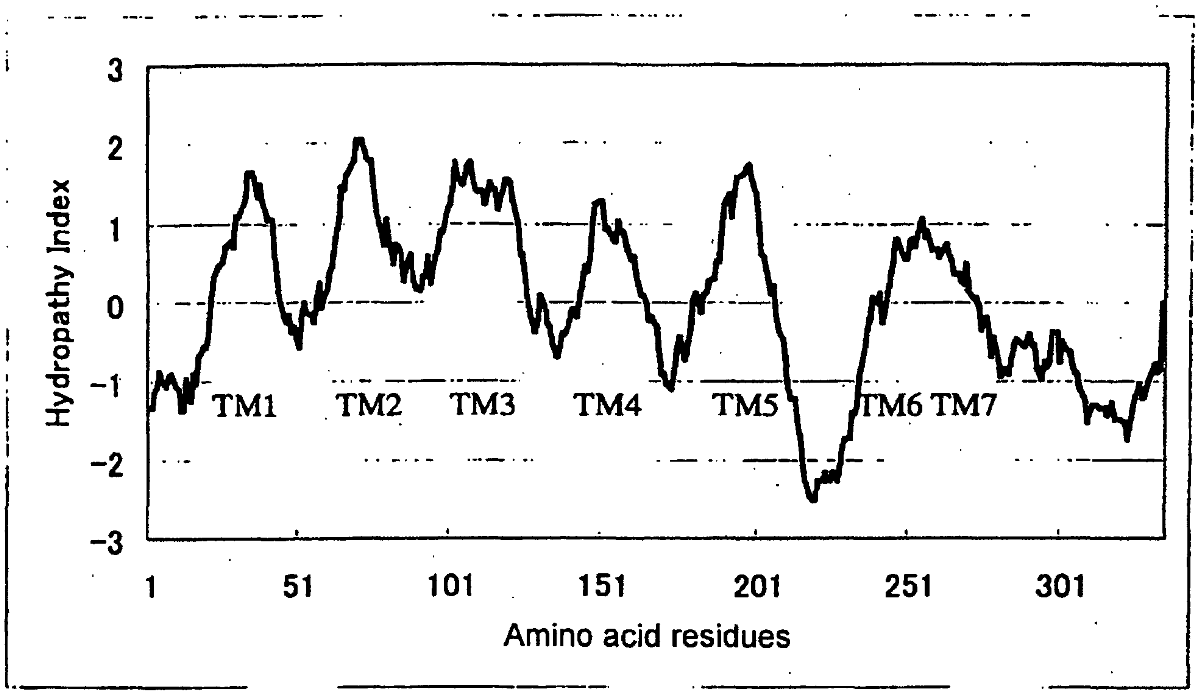
3
2
1
0
-1
-2
-3
Hydropathy Index
TM1
TM2
TM3
TM4
TM5
TM6 TM7
1
51
101
151
201
251
301
Amino acid residues

Figure 29

```
                                                  ######### TM1 ##
Y14705      ------------MTSAESLLFTSLGPSPSSGDGDCRFNEEFKFILLPMSYAVVFVLGLAL
AJ277752    ------------MTSADSLLFTSLGPSPSSGDGDCKFNEEFKFILLPLSYAVVFVLGLAL
AF031897    --MDAPVRMFSLAPWTPTPTPWLGGNTTAAAEAKCVFNEEFKFILLPISYGIVFVVGLPL
X99953      MTEDIMATSYPTFLTTPYLPMKLLMNLTNDTEDICVFDEGFKFLLLPVSYSAVFMVGLPL
AF069555    -------------MSMANFTA---GR------NSCTFQEEFKQVLLPLVYSVVFLLGLPL
X98283      -------------MSMANFTG---GR------NSCTFHEEFKQVLLPLVYSVVFLLGLPL
D63665      -------------MERDNGTIQAPGLPP----TTCVYREDFKRLLLPPVYSVVLVVGLPL
GPRv71      ------------MEKVDMNTSQ--EQ------GLCQFSEKYKQVYLSLAYSIIFILGLPL
                                             * : * :* : *.  *. ::::**.*

            #####               ######## TM2 ########               ####
Y14705      NAPTLWLFLFRLRPWDATATYMFHLALSDTLYVLSLPTLVYYYAARNHWPFGTGLCKFVR
AJ277752    NAPTLWLFLFRLRPWDATATYMFHLALSDTLYVLSLPTLVYYYAARNHWPFGTGFCKFVR
AF031897    NSWAMWIFVSRMRPWNATTTYMFNLAISDTLYVFSLPTLVYYYADRNNWPFGKVFCKIVR
X99953      NIAAMWIFIAKMRPWNPTTVYMFNLALSDTLYVLSLPTLVYYYADKNNWPFGEVLCKLVR
AF069555    NAVVIGQIWLARKALTRTTIYMLNLATADLLYVCSLPLLIYNYTQKDYWPFGDFTCKFVR
X98283      NAVVIGQIWLARKALTRTTIYMLNLAMADLLYVCSLPLLIYNYTQKDYWPFGDFTCKFVR
D63665      NVCVIAQICASRRTLTRSAVYTLNLALADLLYACSLPLLIYNYARGDHWPFGDLACRLVR
GPRv71      NGTVLWHFWGQTKRWSCATTYLVNLMVADLLYVL-LPFLIITYSLDDRWPFGELLCKLVH
            *  .:  :     :    :: * .:*  :* **.  ** *:  *:  : ****   *::*:

            ###TM3 #############                         ######## TM4 #####
Y14705      FLFYWNLYCSVLFLTCISVHRYLGICHPLRAIRWGRPR-FASLLCLGVWLVVAGCLVPNL
AJ277752    FLFYWNLYCSVLFLTCISVHRYMGICHPLRAIRWGRPR-FAGLLCLGVWLVVAGCLVPNL
AF031897    FLFYANLYSSILFLTCISVHRYMGICHPIRSLKWVKTK-HARLICVGVWLVVTICLIPNL
X99953      FLFYANLYSSILFLTCISVHRYRGVCHPITSLRRMNAK-HAYVICALVWLSVTLCLVPNL
AF069555    FQFYTNLHGSILFLTCISVQRYMGICHPLASWHKKKGKKLTWLVCAAVWFIVIAQCLPTF
X98283      FQFYTNLHGSILFLTCISVQRYMGICHPLASWHKKKGKKLTWLVCAAVWFIVIAQCLPTF
D63665      FLFYANLHGSILFLTCISFQRYLGICHPLAPWHKRGGRRAAWVVCGVVWLVVTAQCLPTA
GPRv71      FLFYINLYGSILLLTCISVHQFLGVCHPLCSLPYRTRR-HAWLGTSTTWALVVLQLLPTL
            * ** **: *:*:*****.::: *:***: .        :  : :     .*  *    :*.

            ##                                 ######### TM5 #######
Y14705      FFVTTNANGTTILCHDTTLPEEFDHYVYFSSAVMVLLFGLPFLITLVCYGLMARRLYRPL
AJ277752    FFVTTNANGTTILCHDTTLPEEFDHYVYFSSTIMVLLFGFPFLITLVCYGLMARRLYRPL
AF031897    IFVTTSSKDNSTLCHDTTKPEEFDHYVHYSSSIMALLFGIPFLVIVVCYCLMAKRLCKRS
X99953      IFVTVSPKVKNTICHDTTRPEDFARYVEYSTAIMCLLFGIPCLIIAGCYGLMTRELMKPI
AF069555    VFASTGTQRNRTVCYDLSPPDRSASYFPYGITLTITGFLLPFAAILACYCSMARILCQKD
X98283      VFASTGTQRNRTVCYDLSPPDRSTSYFPYGITLTITGFLLPFAAILACYCSMARILCQKD
D63665      VFAATGI-QRNRTVCYDLSPPILSTRYLPYGMALTVIGFLLPFTALLACYCRMARRLCRQD
GPRv71      AFSHTDYINGQMIWYDMTSQENFDRLFAYGIVLTLSGFLSLLGHFGVLFTDGQEPDQARG
              *  ..        : :* :         . :.   :     *             :   .

                               ######## TM6 #########                   ##
Y14705      PGAGQS---SSRLRSLRTIAVVLTVFAVCFVPFHITRTIYYQAR-LLQADCHVLNIVNVV
AJ277752    PGAGQS---SSRLRSLRTIAVVLTVFAVCFVPFHITRTIYYLAR-LLNAECRVLNIVNVV
AF031897    FPSPSPRVPSYKKRSIKMIIIVLTVFAICFVPFHITRTLYYTSR-YFQADCQTLNIINFT
X99953      VSGNQQTLPSYKKRSIKTIIFVMIAFAICFMPFHITRTLYYYAR-LLGIKCYALNVINVT
AF069555    ELIGLAVH-KKKDKAVRMIIIVVIVFSISFFPFHLTKTIYLIVRSSPTLPCPTLQAFAIA
X98283      ELIGLAVH-KKKDKAVRMIIIVVIVFSISFFPFHLTKTIYLIVRSSASLPCPTLQAFAIA
D63665      GPAGPVAQ-ERRSKAARMAVVVAAVFVISFLPFHITKTAYLAVRSTPGVSCPVLETFAAA
GPRv71      EPHEDRQHSPSQVHPDHPTGVWPLHPLFCALPYHSLLLPHHLLSAFSGLPALDGSQCGLQ
                           : :. :   .      .. .*:*     :            .  .
```

Figure 30

```
                 ##### TM7 #########
Y14705           YKVTRPLASANSCLDPVLYLFTGDKYRNQLQQLCRGSK--PKPR----------TAASSL
AJ277752         YKVTRPLASANSCLDPVLYLFTGDKYRNQLQQLCRGST--PKRR----------TTASSL
AF031897         YKITRPLASINSCLDPILYFMAGDKYRGRLRRGAAQR---P-R-----------PVPTSL
X99953           YKVTRPLASANSCIDPILYFLANDRYRRRLIRTVRRRSSVPNRRCMHTNHPQTEPHMTAG
AF069555         YKCTRPFASMNSVLDPILFYFTQRKFRESTRYLLDKMS----------------SKWRHD
X98283           YKCTRPFASMNSVLDPILFYFTQRKFRESTRYLLDKMS----------------SKWRQD
D63665           YKGTRPFASANSVLDPILFYFTQQKFRRQPHDLLQKLT----------------AKWQRQ
GPRv71           DMEASGECEQLPQPSPVLSFKGGKNRVRLLQKLRQNKLG--------------EHPAGRK
                    :   ..  .  .*:*      .

Y14705           ALVTLHEESISRWADTHQDSTFSAYEGDRL------------------------------
AJ277752         ALVTLHEESISRWADIHQDSIFPAYEGDRL------------------------------
AF031897         LALVSPSVDSSVVGSCCNSE----SRGMGTVWSRGGQ-----------------------
X99953           PLPVISAEEIPSNGSMVRDENGEGSREHRVEWTDTKEINQMMNRRSTIKRNSTDKNDMKE
AF069555         HCITYGS-----------------------------------------------------
X98283           HCISYGS-----------------------------------------------------
D63665           RV----------------------------------------------------------
GPRv71           RCPGLNRSG---------------------------------------------------

Y14705           ------------------------------------------------------------
AJ277752         ------------------------------------------------------------
AF031897         ------------------------------------------------------------
X99953           NRHGENYLPYVEVVEKEDYETKRENRKTTEQSSKTNAEQDELQTQIDSRLKRGKWQLSSK
AF069555         ------------------------------------------------------------
X98283           ------------------------------------------------------------
D63665           ------------------------------------------------------------
GPRv71           ------------------------------------------------------------

Y14705           -----------------------------------------------------------
AJ277752         -----------------------------------------------------------
AF031897         -----------------------------------------------------------
X99953           KGAAQENEKGHMEPSFEGEGTSTWNLLTPKMYGKKDRLAKNVEEVGYGKEKELQNFPKA
AF069555         -----------------------------------------------------------
X98283           -----------------------------------------------------------
D63665           -----------------------------------------------------------
GPRv71           -----------------------------------------------------------
```

Figure 31

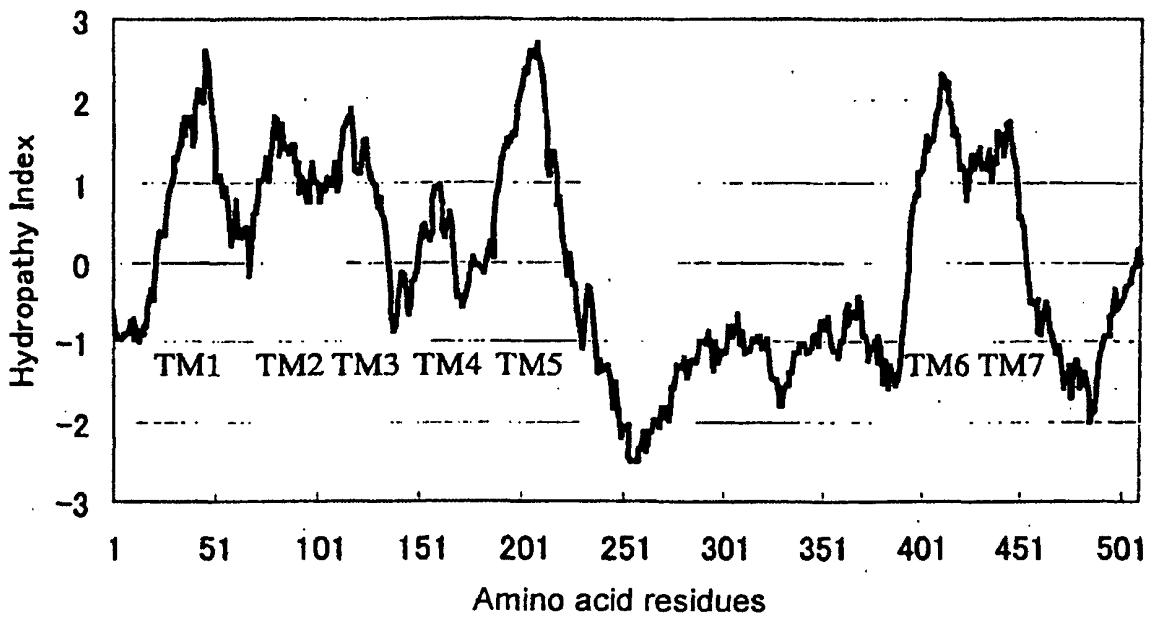
3
2
1
0
-1
-2
-3
Hydropathy Index
TM1
TM2
TM3
TM4
TM5
TM6
TM7
1
51
101
151
201
251
301
351
401
451
501
Amino acid residues

Figure 32

```
                                                      ######### TM1 ########
U03866       ------MVFLSGNAS--DSSNCTQPPAP-----VNISKAILLGVILGGLILFGVLGNILV
L31774       ------MVFLSGNAS--DSSNCTQPPAP-----VNISKAILLGVILGGLILFGVLGNILV
D25235       ------MVFLSGNAS--DSSNCTQPPAP-----VNISKAILLGVILGGLILFGVLGNILV
D32202       ------MVFLSGNAS--DSSNCTQPPAP-----VNISKAILLGVILGGLILFGVLGNILV
D32201       ------MVFLSGNAS--DSSNCTQPPAP-----VNISKAILLGVILGGLILFGVLGNILV
AF013261     ------MVFLSGNAS--DSSNCTQPPAP-----VNISKAILLGVILGGLILFGVLGNILV
U81982       ------MVFLSGNAS--DSSNCTHPPAP-----VNISKAILLGVILGGLILFGVLGNILV
U07126       ------MVLLSENAS--EGSNCTHPPAP-----VNISKAILLGVILGGLIIFGVLGNILV
S71323       MVPVLDNMTPSSVTL--NCSNCSHVLAPE----LNTVKAVVLGMVLGIFILFGVIGNILV
D63859       -------MTPSSVTL--NCSNCSHVLAPE----LNTVKAVVLGMVLGIFILFGVIGNILV
AF091890     ------MSLNSSLSCRKELSNLTEEEGG------EGGVIITQFIAIIVITIFVCLGNLVI
GPRv72       ------MTSTCTNST--RESNSSHTCMPLSKMPISLAHGIIRSTVLVIFLAASFVGNIVL
                       .  :     ** :.          .    :     :  :     :**:::

             ###             ########## TM2 ###########         @########
U03866       ILSVACHRHLHSVTHYYIVNLAVADLLLTSTVLPFSAIFEVLGYWAFGRVFCNIWAAVDV
L31774       ILSVACHRHLHSVTHYYIVNLAVADLLLTSTVLPFSAIFEVLGYWAFGRVFCNIWAAVDV
D25235       ILSVACHRHLHSVTHYYIVNLAVADLLLTSTVLPFSAIFEVLGYWAFGRVFCNIWAAVDV
D32202       ILSVACHRHLHSVTHYYIVNLAVADLLLTSTVLPFSAIFEVLGYWAFGRVFCNIWAAVDV
D32201       ILSVACHRHLHSVTHYYIVNLAVADLLLTSTVLPFSAIFEVLGYWAFGRVFCNIWAAVDV
AF013261     ILSVACHRHLHSVTHYYIVNLAVADLLLTSTVLPFSAIFEVLGYWAFGRVFCNIWAAVDV
U81982       ILSVACHRHLHSVTHYYIVNLAVADLLLTSTVLPFSAIFEILGYWAFGRVFCNIWAAVDV
U07126       ILSVACHRHLHSVTHYYIVNLAVADLLLTSTVLPFSAIFEILGYWAFGRVFCNIWAAVDV
S71323       ILSVVCHRHLQTVTYYFIVNLAVADLLLSSTVLPFSAIFEILDRWVFGRVFCNIWAAVDV
D63859       ILSVVCHRHLQTVTYYFIVNLAVADLLLSSTVLPFSAIFEILDRWVFGRVFCNIWAAVDV
AF091890     VVTLYKKSYLLTLSNKFVFSLTLSNFLLSVLVLPFVVTSSIRREWIFGVVWCNFSALLYL
GPRv72       ALVLQRKPQLLQVTNRFIFNLLVTDLLQISLVAPWVVATSVPLFWPLNSHFCTALVSLTH
              : :  :  *  ::  ::..* ::::*    * *: .  .:   * :.  :*.  . :

             # TM3 #########                     ########## TM4 #########
U03866       LCCTASIMGLCIISIDRYIGVSYPLRYPTIVTQRRGLMALLCVWALSLVISIGPLFGWR-
L31774       LCCTASIMGLCIISIDRYIGVSYPLRYPTIVTQRRGLMALLCVWALSLVISIGPLFGWR-
D25235       LCCTASIMGLCIISIDRYIGVSYPLRYPTIVTQRRGLMALLCVWALSLVISIGPLFGWR-
D32202       LCCTASIMGLCIISIDRYIGVSYPLRYPTIVTQRRGLMALLCVWALSLVISIGPLFGWR-
D32201       LCCTASIMGLCIISIDRYIGVSYPLRYPTIVTQRRGLMALLCVWALSLVISIGPLFGWR-
AF013261     LCCTASIMGLCIISIDRYIGVSYPLRYPTIVTQRRGLMALLCVWALSLVISIGPLFGWR-
U81982       LCCTASIISLCVISIDRYIGVSYPLRYPTIVTQRRGLRALLCVWAFSLVISVGPLFGWR-
U07126       LCCTASIMGLCIISIDRYIGVSYPLRYPTIVTQRRGVRALLCVWVLSLVISIGPLFGWR-
S71323       LCCTASIMSLCVISVDRYIGVSYPLRYPAIMTKRRALLAVMLLWVLSVIISIGPLFGWK-
D63859       LCCTASIMSLCVISVDRYIGVSYPLRYPAIMTKRRALLAVMLLWVLSVIISIGPLFGWK-
AF091890     LISSASMLTLGVIAIDRYYAVLYPMVYPMKITGNRAVMALVYIWLHSLIGCLPPLFGWSS
GPRv72       LFAFASVNTIVVVSVDRYLSIIHPLSYPSKMTQRRGYLLLYGTWIVAILQSTPPLYGWGQ
             * . **:  : ::::*** .: :*: **  :* .*.   :   *  ::: .  **:**
```

Figure 33

```
          ##       @       ######### TM5 ##########
U03866    QPAPEDETICQINE--EPGYVLFSALGSFYLPLAIILVMYCRVYVVAKRESRGLK----S
L31774    QPAPEDETICQINE--EPGYVLFSALGSFYLPLAIILVMYCRVYVVAKRESRGLK----S
D25235    QPAPEDETICQINE--EPGYVLFSALGSFYLPLAIILVMYCRVYVVAKRESRGLK----S
D32202    QPAPEDETICQINE--EPGYVLFSALGSFYLPLAIILVMYCRVYVVAKRESRGLK----S
D32201    QPAPEDETICQINE--EPGYVLFSALGSFYLPLAIILVMYCRVYVVAKRESRGLK----S
AF013261  QPAPEDETICQINE--EPGYVLFSALGSFYLPLAIILVMYCRVYVVAKRESRGLK----S
U81982    QPAPDDETICQINE--EPGYVLFSALGSFYVPLTIILAMYCRVYVVAKRESRGLK----S
U07126    QPAPEDETICQINE--EPGYVLFSALGSFYVPLAIILVMYCRVYVVAKRESRGLK----S
S71323    EPAPEDETVCKITE--EPGYAIFSAVGSFYLPLAIILAMYCRVYVVAQKESRGLK----E
D63859    EPAPEDETVCKITE--EPGYAIFSAVGSFYLPLAIILAMYCRVYVVAQKESRGLK----E
AF091890  VEFDEFKWMCVAAWHREPGYTAFWQIWCALFPFLVMLVCYGFIFRVARVKARKVH----C
GPRv72    AAFDERNALCSMIWGASPSYTILSVVSFIVIPLIVMIACYSVVFCAARRQHALLYNVKRH
              : : :*      .*.*. :  :    .*: :::. *  :: .*: :   :

                                                              ########
U03866    GLKTDKSDSEQVTLRIHRKNAPAGGSGMASAKTKTHFSVRLLKFSREKKAAKTLGIVVG-
L31774    GLKTDKSDSEQVTLRIHRKNAPAGGSGMASAKTKTHFSVRLLKFSREKKAAKTLGIVVG-
D25235    GLKTDKSDSEQVTLRIHRKNAPAGGSGMASAKTKTHFSVRLLKFSREKKAAKTLGIVVG-
D32202    GLKTDKSDSEQVTLRIHRKNAPAGGSGMASAKTKTHFSVRLLKFSREKKAAKTLGIVVG-
D32201    GLKTDKSDSEQVTLRIHRKNAPAGGSGMASAKTKTHFSVRLLKFSREKKAAKTLGIVVG-
AF013261  GLKTDKSDSEQVTLRIHRKNAPAGGSGMASAKTKTHFSVRLLKFSREKKAAKTLGIVVG-
U81982    GLKTDKSDSEQVTLRIHRKNAPAGGSGVASAKNKTHFSVRLLKFSREKKAAKTLGIVVG-
U07126    GLKTDKSDSEQVTLRIHRKNVPAEGGGVSSAKNKTHFSVRLLKFSREKKAAKTLGIVVG-
S71323    GQKIEKSDSEQVILRMHRGNTTVSED--EALRSRTHFALRLLKFSREKKAAKTLGIVVG-
D63859    GQKIEKSDSEQVILRMHRGNTTVSED--EALRSRTHFALRLLKFSREKKAAKTLGIVVG-
AF091890  GTVVIVEEDAQRTG---RKNSSTSTS--SSGSRRNAFQGVVYSANQCK-ALITILVVLG-
GPRv72    SLEVRVKDCVENEDEEGAEKKEEFQD--ESEFRRQHEGEVKAKEGRMEAKDGSLKAKEGS
          .   . .:  :        :     .   :   :        . .: :    ::    *

          ######## TM6 ###############           ############ TM7
U03866    -----CFVLCWLP-----FFLVMPIGSFFPD---FKPSETVFKIVFWLGYLNSCIN----
L31774    -----CFVLCWLP-----FFLVMPIGSFFPD---FKPSETVFKIVFWLGYLNSCIN----
D25235    -----CFVLCWLP-----FFLVMPIGSFFPD---FKPSETVFKIVFWLGYLNSCIN----
D32202    -----CFVLCWLP-----FFLVMPIGSFFPD---FKPSETVFKIVFWLGYLNSCIN----
D32201    -----CFVLCWLP-----FFLVMPIGSFFPD---FKPSETVFKIVFWLGYLNSCIN----
AF013261  -----CFVLCWLP-----FFLVMPIGSFFPD---FKPSETVFKIVFWLGYLNSCIN----
U81982    -----CFVLCWLP-----FFLVMPIGSFFPD---FKPPETVFKIVFWLGYLNSCIN----
U07126    -----CFVLCWLP-----FFLVMPIGSFFPD---FKPSETVFKIVFWLGYLNSCIN----
S71323    -----CFVLCWLP-----FFLVLPIGSIFPA---YRPSDTVFKITFWLGYFNSCIN----
D63859    -----CFVLCWLP-----FFLVLPIGSIFPA---YRPSDTVFKITFWLGYFNSCIN----
AF091890  -----AFMVTWGP-----YMVVIASEALWGK---SSVSPSLETWATWLSFASAVCH----
GPRv72    TGTSESSVEARGSEEVRESSTVASDGSMEGKEGSTKVEENSMKADKGRTEVNQCSIDLGE
               . :    .        * .  ::        .  .  .       .
```

Figure 34

```
                ########
U03866          -PIIYPCSSQEFK----KAFQNVLRIQCLCRKQSSKH---ALGYT-LHPPSQAVEGQHK-
L31774          -PIIYPCSSQEFK----KAFQNVLRIQCLRRKQSSKH---ALGYT-LHPPSQAVEGQHK-
D25235          -PIIYPCSSQEFK----KAFQNVLRIQCLRRKQSSKH---ALGYT-LHPPSQAVEGQHK-
D32202          -PIIYPCSSQEFK----KAFQNVLRIQCLRRKQSSKH---ALGYT-LHPPSQAVEGQHK-
D32201          -PIIYPCSSQEFK----KAFQNVLRIQCLRRKQSSKH---ALGYT-LHPPSQAVEGQHK-
AF013261        -PIIYPCSSQEFK----KAFQNVLRIQCLCRKQSSKH---ALGYT-LHPPSQAVEGQHK-
U81982          -PIIYPCSSQEFK----KAFQNVLKIQCLRRKQSSKH---ALGYT-LHAPSQALEGQHK-
U07126          -PIIYPCSSQEFK----KAFQNVLRIQCLRRRQSSKH---ALGYT-LHPPSQALEGQHR-
S71323          -PIIYLCSNQEFK----KAFQSLLGVHCLRMTPRAHHHHLSVGQSQTQGHSLTISLDSKG
D63859          -PIIYLCSNQEFK----KAFQSLLGVHCLRMTPRAHHHHLSVGQSQTQGHSLTISLDSKG
AF091890        -PLIYGLWN--------KTVRKELLGMCFGDRYYREP----FVQR--QRTSRLFSISNR-
GPRv72          DDMEFGEDDINFSEDDVEAVNIPESLPPSRRNSNSNP---PLPRCYQCKAAKVIFIIIFS
                 : :  .     ::..           .     .        :  .

U03866          DMVRIPVGSRETFYRISKTDG--VCEWKFFSSMPRGSARITVSKDQS--SCTTARVRSKS
L31774          DMVRIPVGSRETFYRISKTDG--VCEWKFFSSMPRGSARITVSKDQS--SCTTARVRSKS
D25235          DMVRIPVGSRETFYRISKTDG--VCEWKFFSSMPRGSARITVSKDQS--SCTTARVRSKS
D32202          DMVRIPVGSRETFYRISKTDG--VCEWKFFSSMPRGSARITVSKDQS--SCTTARTKSRS
D32201          DMVRIPVGSRETFYRISKTDG--VCEWKFFSSMPRGSARITVSKDQS--SCTTARGHTPM
AF013261        DMVRIPVGSRETFYRISKTDG--VCEWKFFSSMPRGSARITVSKDQS--SCTTARRGMDC
U81982          DMVRIPVGSGETFYKISKTDG--VCEWKFFSSMPRGSARITVPKDQS--ACTTARVRSKS
U07126          DMVRIPVGSGETFYKISKTDG--VCEWKFFSSMPQGSARITVPKDQS--ACTTARVRSKS
S71323          APCRLSPSSSVALSRTPSSRD--SREWRVFSGGPINSG--PGPTEAG--RAKVAKLCNKS
D63859          APCRLSPSSSVALSRTPSSRD--SREWRVFSGGPINSG--PGPTEAG--RAKVAKLCNKS
AF091890        -ITDLGLSPHLTALMAG-------------GQPLGHS--SSTGDTG--FSCSQDSGN--
GPRv72          YVLSLGPYCFLAVLAVWVDVETQVPQWVITIIIWLFFLQCCIHPYVYGYMHKTIKKEIQD
:  .  :

U03866          FLQVCCCVGPS-TPSLDKN--HQVPTIKVHTISLSENGEEV-------------------
L31774          FLQVCCCVGPS-TPSLDKN--HQVPTIKVHTISLSENGEEV-------------------
D25235          FLEVCCCVGPS-TPSLDKN--HQVPTIKVHTISLSENGEEV-------------------
D32202          VTRLECSG----MILAHCN--LRLPGSRDSPASASQAAGTTGDVPPGRRHQAQLIFVFLV
D32201          T-----------------------------------------------------------
AF013261        RYFTKNCR----EHIKHVN--FMMPPWRKGLEC---------------------------
U81982          FLQVCCCVGPS-TPNPGEN--HQVPTIKIHTISLSENGEEV-------------------
U07126          FLQVCCCVGSS-APRPEEN--HQVPTIKIHTISLGENGEEV-------------------
S71323          LHRTCCCILRARTPTQDPAPLGDLPTIKIHQLSLSEKGESV-------------------
D63859          LHRTCCCILRARTPTQDPAPLGDLPTIKIHQLSLSEKGESV-------------------
AF091890        -LRAL-------------------------------------------------------
GPRv72          MLKKFFCKEK--PPKEDSH--PDLPGTEGGTEGKIVPSYDSATFP---------------

U03866          ----------------
L31774          ----------------
D25235          ----------------
D32202          ETGFHHVGQDDLDLLTS
D32201          ----------------
AF013261        ----------------
U81982          ----------------
U07126          ----------------
S71323          ----------------
D63859          ----------------
AF091890        ----------------
GPRv72          ----------------
```

## INTERNATIONAL SEARCH REPORT

International application No.
PCT/JP00/09408

A. CLASSIFICATION OF SUBJECT MATTER
Int.Cl[7] C12N15/09, C12N1/15, C12N1/19, C12N1/21, C12N5/10, C07K14/705, C07K16/28, C12P21/02, C12Q1/02, C12Q1/68, A61K31/711, A61K48/00, A61P43/00, G01N33/15, G01N33/50
According to International Patent Classification (IPC) or to both national classification and IPC

B. FIELDS SEARCHED
Minimum documentation searched (classification system followed by classification symbols)
Int.Cl[7] C12N15/00-15/09, C07K14/705

Documentation searched other than minimum documentation to the extent that such documents are included in the fields searched

Electronic data base consulted during the international search (name of data base and, where practicable, search terms used)
GeneBank/EMBL/DDBJ/GeneSeq
SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| A | JP, 8-245697, A (Takeda Chemical Industries, Ltd.), 24 September, 1996 (24.09.96) (Family: none) | 1-15,17 |
| A | WO, 98/46620, A1 (MILLENNIUM PHARM INC), 22 January, 1998 (22.01.98) & AU, 9869736, A & US, 5891720, A & EP, 1007536, A1 | 1-15,17 |
| A | WO, 99/37679, A1 (MILLENNIUM PHARM INC), 29 July, 1999 (29.07.99) & US, 5945307, A & AU, 9922369, A & EP, 1056777, A1 | 1-15,17 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 March, 2001 (27.03.01) | 10 April, 2001 (10.04.01) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP00/09408

**Box I Observations where certain claims were found unsearchable (Continuation of item 1 of first sheet)**

This international search report has not been established in respect of certain claims under Article 17(2)(a) for the following reasons:

1. ☒ Claims Nos.: 16
because they relate to subject matter not required to be searched by this Authority, namely:

The invention as set forth in claim 16 pertains to methods for diagnosis of diseases and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The inventions as set forth in claims 1 to 15 and 17 are divided into groups of 9 individual inventions, i.e., inventions relating to DNAs encoding the amino acids of SEQ ID NOS:1 to 4 and 17 to 21, and DNAs having the sequences of SEQ ID NOS:5 to 8 and 22 to 26. These groups of inventions are not considered as relating to a group of inventions so linked as to form a single general inventive concept.

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:

Claims 1 to 15 and 17 (inventions relating to the DNA encoding the amino acid sequence of SEQ ID NO:1 and the DNA having the sequence of SEQ ID NO:5)

**Remark on Protest** ☐ The additional search fees were accompanied by the applicant's protest.
☐ No protest accompanied the payment of additional search fees.

Form PCT/ISA/210 (continuation of first sheet (1)) (July 1992)

SEQUENCE LISTING

<110> HELIX RESEARCH INSTITUTE

<120> NOVEL GUANOSINE TRIPHOSPHATE-BINDING PROTEIN-COUPLED RECEPTORS AND GENES THEREOF, AND PRODUCTION AND USES THEREOF

<130> H1-113DP1PCT

<140>
<141>

<150> JP 1999-375152
<151> 1999-12-28

<150> JP 2000-101339
<151> 2000-03-31

<160> 63

<170> PatentIn Ver. 2.1

<210> 1
<211> 371
<212> PRT
<213> Homo sapiens

<400> 1
Met Pro Ala Asn Phe Thr Glu Gly Ser Phe Asp Ser Ser Gly Thr Gly
1               5                   10                  15

Gln Thr Leu Asp Ser Ser Pro Val Ala Cys Thr Glu Thr Val Thr Phe
            20                  25                  30

Thr Glu Val Val Glu Gly Lys Glu Trp Gly Ser Phe Tyr Tyr Ser Phe

35 40 45

Lys Thr Glu Gln Leu Ile Thr Leu Trp Val Leu Phe Val Phe Thr Ile
50 55 60

Val Gly Asn Ser Val Val Leu Phe Ser Thr Trp Arg Arg Lys Lys Lys
65 70 75 80

Ser Arg Met Thr Phe Phe Val Thr Gln Leu Ala Ile Thr Asp Ser Phe
85 90 95

Thr Gly Leu Val Asn Ile Leu Thr Asp Ile Asn Trp Arg Phe Thr Gly
100 105 110

Asp Phe Thr Ala Pro Asp Leu Val Cys Arg Val Val Arg Tyr Leu Gln
115 120 125

Val Val Leu Leu Tyr Ala Ser Thr Tyr Val Leu Val Ser Leu Ser Ile
130 135 140

Asp Arg Tyr His Ala Ile Val Tyr Pro Met Lys Phe Leu Gln Gly Glu
145 150 155 160

Lys Gln Ala Arg Val Leu Ile Val Ile Ala Trp Ser Leu Ser Phe Leu
165 170 175

Phe Ser Ile Pro Thr Leu Ile Ile Phe Gly Lys Arg Thr Leu Ser Asn
180 185 190

Gly Glu Val Gln Cys Trp Ala Leu Trp Pro Asp Asp Ser Tyr Trp Thr
195 200 205

Pro Tyr Met Thr Ile Val Ala Phe Leu Val Tyr Phe Ile Pro Leu Thr
210 215 220

Ile Ile Ser Ile Met Tyr Gly Ile Val Ile Arg Thr Ile Trp Ile Lys

225 230 235 240

Ser Lys Thr Tyr Glu Thr Val Ile Ser Asn Cys Ser Asp Gly Lys Leu
245 250 255

Cys Ser Ser Tyr Asn Arg Gly Leu Ile Ser Lys Ala Lys Ile Lys Ala
260 265 270

Ile Lys Tyr Ser Ile Ile Ile Ile Leu Ala Phe Ile Cys Cys Trp Ser
275 280 285

Pro Tyr Phe Leu Phe Asp Ile Leu Asp Asn Phe Asn Leu Leu Pro Asp
290 295 300

Thr Gln Glu Arg Phe Tyr Ala Ser Val Ile Ile Gln Asn Leu Pro Ala
305 310 315 320

Leu Asn Ser Ala Ile Asn Pro Leu Ile Tyr Cys Val Phe Ser Ser Ser
325 330 335

Ile Ser Phe Pro Cys Arg Glu Gln Arg Ser Gln Asp Ser Arg Met Thr
340 345 350

Phe Arg Glu Arg Thr Glu Arg His Glu Met Gln Ile Leu Ser Lys Pro
355 360 365

Glu Phe Ile
370

<210> 2
<211> 363
<212> PRT
<213> Homo sapiens

<400> 2

Met Gly Pro Gly Glu Ala Leu Leu Ala Gly Leu Leu Val Met Val Leu
1 5 10 15

Ala Val Ala Leu Leu Ser Asn Ala Leu Val Leu Leu Cys Cys Ala Tyr
20 25 30

Ser Ala Glu Leu Arg Thr Arg Ala Ser Gly Val Leu Leu Val Asn Leu
35 40 45

Ser Leu Gly His Leu Leu Leu Ala Ala Leu Asp Met Pro Phe Thr Leu
50 55 60

Leu Gly Val Met Arg Gly Arg Thr Pro Ser Ala Pro Gly Ala Cys Gln
65 70 75 80

Val Ile Gly Phe Leu Asp Thr Phe Leu Ala Ser Asn Ala Ala Leu Ser
85 90 95

Val Ala Ala Leu Ser Ala Asp Gln Trp Leu Ala Val Gly Phe Pro Leu
100 105 110

Arg Tyr Ala Gly Arg Leu Arg Pro Arg Tyr Ala Gly Leu Leu Leu Gly
115 120 125

Cys Ala Trp Gly Gln Ser Leu Ala Phe Ser Gly Ala Ala Leu Gly Cys
130 135 140

Ser Trp Leu Gly Tyr Ser Ser Ala Phe Ala Ser Cys Ser Leu Arg Leu
145 150 155 160

Pro Pro Glu Pro Glu Arg Pro Arg Phe Ala Ala Phe Thr Ala Thr Leu
165 170 175

His Ala Val Gly Phe Val Leu Pro Leu Ala Val Leu Cys Leu Thr Ser
180 185 190

Leu Gln Val His Arg Val Ala Arg Arg His Cys Gln Arg Met Asp Thr
195 200 205

Val Thr Met Lys Ala Leu Ala Leu Leu Ala Asp Leu His Pro Ser Val
210 215 220

Arg Gln Arg Cys Leu Ile Gln Gln Lys Arg Arg Arg His Arg Ala Thr
225 230 235 240

Arg Lys Ile Gly Ile Ala Ile Ala Thr Phe Leu Ile Cys Phe Ala Pro
245 250 255

Tyr Val Met Thr Arg Leu Ala Glu Leu Val Pro Phe Val Thr Val Asn
260 265 270

Ala Gln Trp Gly Ile Leu Ser Lys Cys Leu Thr Tyr Ser Lys Ala Val
275 280 285

Ala Asp Pro Phe Thr Tyr Ser Leu Leu Arg Arg Pro Phe Arg Gln Val
290 295 300

Leu Ala Gly Met Val His Arg Leu Leu Lys Arg Thr Pro Arg Pro Ala
305 310 315 320

Ser Thr His Asp Ser Ser Leu Asp Val Ala Gly Met Val His Gln Leu
325 330 335

Leu Lys Arg Thr Pro Arg Pro Ala Ser Thr His Asn Gly Ser Val Asp
340 345 350

Thr Glu Asn Asp Ser Cys Leu Gln Gln Thr His
355 360

<210> 3
<211> 419

<212> PRT

<213> Homo sapiens

<400> 3

Met Leu Ala Ala Ala Phe Ala Asp Ser Asn Ser Ser Ser Met Asn Val
1               5                   10                  15

Ser Phe Ala His Leu His Phe Ala Gly Gly Tyr Leu Pro Ser Asp Ser
            20                  25                  30

Gln Asp Trp Arg Thr Ile Ile Pro Ala Leu Leu Val Ala Val Cys Leu
        35                  40                  45

Val Gly Phe Val Gly Asn Leu Cys Val Ile Gly Ile Leu Leu His Asn
    50                  55                  60

Ala Trp Lys Gly Lys Pro Ser Met Ile His Ser Leu Ile Leu Asn Leu
65                  70                  75                  80

Ser Leu Ala Asp Leu Ser Leu Leu Leu Phe Ser Ala Pro Ile Arg Ala
                85                  90                  95

Thr Ala Tyr Ser Lys Ser Val Trp Asp Leu Gly Trp Phe Val Cys Lys
            100                 105                 110

Ser Ser Asp Trp Phe Ile His Thr Cys Met Ala Ala Lys Ser Leu Thr
        115                 120                 125

Ile Val Val Val Ala Lys Val Cys Phe Met Tyr Ala Ser Asp Pro Ala
    130                 135                 140

Lys Gln Val Ser Ile His Asn Tyr Thr Ile Trp Ser Val Leu Val Ala
145                 150                 155                 160

Ile Trp Thr Val Ala Ser Leu Leu Pro Leu Pro Glu Trp Phe Phe Ser
                165                 170                 175

Thr Ile Arg His His Glu Gly Val Glu Met Cys Leu Val Asp Val Pro
180 185 190

Ala Val Ala Glu Glu Phe Met Ser Met Phe Gly Lys Leu Tyr Pro Leu
195 200 205

Leu Ala Phe Gly Leu Pro Leu Phe Phe Ala Ser Phe Tyr Phe Trp Arg
210 215 220

Ala Tyr Asp Gln Cys Lys Lys Arg Gly Thr Lys Thr Gln Asn Leu Arg
225 230 235 240

Asn Gln Ile Arg Ser Lys Gln Val Thr Val Met Leu Leu Ser Ile Ala
245 250 255

Ile Ile Ser Ala Val Leu Trp Leu Pro Glu Trp Val Ala Trp Leu Trp
260 265 270

Val Trp His Leu Lys Ala Ala Gly Pro Ala Pro Pro Gln Gly Phe Ile
275 280 285

Ala Leu Ser Gln Val Leu Met Phe Ser Ile Ser Ser Ala Asn Pro Leu
290 295 300

Ile Phe Leu Val Met Ser Glu Glu Phe Arg Glu Gly Leu Lys Gly Val
305 310 315 320

Trp Lys Trp Met Ile Thr Lys Lys Pro Pro Thr Val Ser Glu Ser Gln
325 330 335

Glu Thr Pro Ala Gly Asn Ser Glu Gly Leu Pro Asp Lys Val Pro Ser
340 345 350

Pro Glu Ser Pro Ala Ser Ile Pro Glu Lys Glu Lys Pro Ser Ser Pro
355 360 365

Ser Ser Gly Lys Gly Lys Thr Glu Lys Ala Glu Ile Pro Ile Leu Pro
370 375 380

Asp Val Glu Gln Phe Trp His Glu Arg Asp Thr Val Pro Ser Val Gln
385 390 395 400

Asp Asn Asp Pro Ile Pro Trp Glu His Glu Asp Gln Glu Thr Gly Glu
405 410 415

Gly Val Lys

<210> 4
<211> 393
<212> PRT
<213> Homo sapiens

<400> 4
Met Glu Thr Thr Met Gly Phe Met Asp Asp Asn Ala Thr Asn Thr Ser
1 5 10 15

Thr Ser Phe Leu Ser Val Leu Asn Pro His Gly Ala His Ala Thr Ser
20 25 30

Phe Pro Phe Asn Phe Ser Tyr Ser Asp Tyr Asp Met Pro Leu Asp Glu
35 40 45

Asp Glu Asp Val Thr Asn Ser Arg Thr Phe Phe Ala Ala Lys Ile Val
50 55 60

Ile Gly Met Ala Leu Val Gly Ile Met Leu Val Cys Gly Ile Gly Asn
65 70 75 80

Phe Ile Phe Ile Ala Ala Leu Val Arg Tyr Lys Lys Leu Arg Asn Leu

85 90 95

Thr Asn Leu Leu Ile Ala Asn Leu Ala Ile Ser Asp Phe Leu Val Ala
100 105 110

Ile Val Cys Cys Pro Phe Glu Met Asp Tyr Tyr Val Val Arg Gln Leu
115 120 125

Ser Trp Glu His Gly His Val Leu Cys Thr Ser Val Asn Tyr Leu Arg
130 135 140

Thr Val Ser Leu Tyr Val Ser Thr Asn Ala Leu Leu Ala Ile Ala Ile
145 150 155 160

Asp Arg Tyr Leu Ala Ile Val His Pro Leu Arg Pro Arg Met Lys Cys
165 170 175

Gln Thr Ala Thr Gly Leu Ile Ala Leu Val Trp Thr Val Ser Ile Leu
180 185 190

Ile Ala Ile Pro Ser Ala Tyr Phe Thr Thr Glu Thr Val Leu Val Ile
195 200 205

Val Lys Ser Gln Glu Lys Ile Phe Cys Gly Gln Ile Trp Pro Val Asp
210 215 220

Gln Gln Leu Tyr Tyr Lys Ser Tyr Phe Leu Phe Ile Phe Gly Ile Glu
225 230 235 240

Phe Val Gly Pro Val Val Thr Met Thr Leu Cys Tyr Ala Arg Ile Ser
245 250 255

Arg Glu Leu Trp Phe Lys Ala Val Pro Gly Phe Gln Thr Glu Gln Ile
260 265 270

Arg Lys Arg Leu Arg Cys Arg Arg Lys Thr Val Leu Val Leu Met Cys

275 280 285

Ile Leu Thr Ala Tyr Val Leu Cys Trp Ala Pro Phe Tyr Gly Phe Thr
290 295 300

Ile Val Arg Asp Phe Phe Pro Thr Val Phe Val Lys Glu Lys His Tyr
305 310 315 320

Leu Thr Ala Phe Tyr Ile Val Glu Cys Ile Ala Met Ser Asn Ser Met
325 330 335

Ile Asn Thr Leu Cys Phe Val Thr Val Lys Asn Asp Thr Val Lys Tyr
340 345 350

Phe Lys Lys Ile Met Leu Leu His Trp Lys Ala Ser Tyr Asn Gly Gly
355 360 365

Lys Ser Ser Ala Asp Leu Asp Leu Lys Thr Ile Gly Met Pro Ala Thr
370 375 380

Glu Glu Val Asp Cys Ile Arg Leu Lys
385 390

<210> 5
<211> 1116
<212> DNA
<213> Homo sapiens

<400> 5
atgccagcca acttcacaga gggcagcttc gattccagtg ggaccgggca gacgctggat 60
tcttccccag tggcttgcac tgaaacagtg acttttactg aagtggtgga aggaaaggaa 120
tggggttcct tctactactc ctttaagact gagcaattga taactctgtg ggtcctcttt 180
gtttttacca ttgttggaaa ctccgttgtg ctttttttcca catggaggag aaagaagaag 240
tcaagaatga ccttctttgt gactcagctg gccatcacag attctttcac aggactggtc 300
aacatcttga cagatattaa ttggcgattc actggagact tcacggcacc tgacctggtt 360

tgccgagtgg tccgctattt gcaggttgtg ctgctctacg cctctaccta cgtcctggtg 420
tccctcagca tagacagata ccatgccatc gtctacccca tgaagttcct tcaaggagaa 480
aagcaagcca gggtcctcat tgtgatcgcc tggagcctgt cttttctgtt ctccattccc 540
accctgatca tatttgggaa gaggacactg tccaacggtg aagtgcagtg ctgggccctg 600
tggcctgacg actcctactg gaccccatac atgaccatcg tggccttcct ggtgtacttc 660
atccctctga caatcatcag catcatgtat ggcattgtga tccgaactat ttggattaaa 720
agcaaaacct acgaaacagt gatttccaac tgctcagatg ggaaactgtg cagcagctat 780
aaccgaggac tcatctcaaa ggcaaaaatc aaggctatca agtatagcat catcatcatt 840
cttgccttca tctgctgttg gagtccatac ttcctgtttg acattttgga caatttcaac 900
ctccttccag acacccagga gcgtttctat gcctctgtga tcattcagaa cctgccagca 960
ttgaatagtg ccatcaaccc cctcatctac tgtgtcttca gcagctccat ctctttcccc 1020
tgcagggagc aaagatcaca ggattccaga atgacgttcc gggagagaac tgagaggcat 1080
gagatgcaga ttctgtccaa gccagaattc atctag 1116

<210> 6
<211> 1092
<212> DNA
<213> Homo sapiens

<400> 6
atgggccccg gcgaggcgct gctggcgggt ctcctggtga tggtactggc cgtggcgctg 60
ctatccaacg cactggtgct gctttgttgc gcctacagcg ctgagctccg cactcgagcc 120
tcaggcgtcc tcctggtgaa tctgtctctg ggccacctgc tgctggcggc gctggacatg 180
cccttcacgc tgctcggtgt gatgcgcggg cggacaccgt cggcgcccgg cgcatgccaa 240
gtcattggct tcctggacac cttcctggcg tccaacgcgg cgctgagcgt ggcggcgctg 300
agcgcagacc agtggctggc agtgggcttc ccactgcgct acgccggacg cctgcgaccg 360
cgctatgccg gcctgctgct gggctgtgcc tggggacagt cgctggcctt ctcaggcgct 420
gcacttggct gctcgtggct tggctacagc agcgccttcg cgtcctgttc gctgcgcctg 480
ccgcccgagc ctgagcgtcc gcgcttcgca gccttcaccg ccacgctcca tgccgtgggc 540
ttcgtgctgc cgctggcggt gctctgcctc acctcgctcc aggtgcaccg ggtggcacgc 600
agacactgcc agcgcatgga caccgtcacc atgaaggcgc tcgcgctgct cgccgacctg 660
cacccccagtg tgcggcagcg ctgcctcatc cagcagaagc ggcgccgcca ccgcgccacc 720
aggaagattg gcattgctat tgcgaccttc ctcatctgct ttgccccgta tgtcatgacc 780
aggctggcgg agctcgtgcc cttcgtcacc gtgaacgccc agtggggcat cctcagcaag 840
tgcctgacct acagcaaggc ggtggccgac ccgttcacgt actctctgct ccgccggccg 900

ttccgccaag tcctggccgg catggtgcac cggctgctga agagaacccc gcgcccagca 960
tccacccatg acagctctct ggatgtggcc ggcatggtgc accagctgct gaagagaacc 1020
ccgcgcccag cgtccaccca caacggctct gtggacacag agaatgattc ctgcctgcag 1080
cagacacact ga 1092

<210> 7
<211> 1260
<212> DNA
<213> Homo sapiens

<400> 7
atgctggcag ctgcctttgc agactctaac tccagcagca tgaatgtgtc ctttgctcac 60
ctccactttg ccggagggta cctgccctct gattcccagg actggagaac catcatcccg 120
gctctcttgg tggctgtctg cctggtgggc ttcgtgggaa acctgtgtgt gattggcatc 180
ctccttcaca atgcttggaa aggaaagcca tccatgatcc actccctgat tctgaatctc 240
agcctggctg atctctccct cctgctgttt tctgcaccta tccgagctac ggcgtactcc 300
aaaagtgttt gggatctagg ctggtttgtc tgcaagtcct ctgactggtt tatccacaca 360
tgcatggcag ccaagagcct gacaatcgtt gtggtggcca aagtatgctt catgtatgca 420
agtgacccag ccaagcaagt gagtatccac aactacacca tctggtcagt gctggtggcc 480
atctggactg tggctagcct gttacccctg ccggaatggt tctttagcac catcaggcat 540
catgaaggtg tggaaatgtg cctcgtggat gtaccagctg tggctgaaga gtttatgtcg 600
atgtttggta agctctaccc actcctggca tttggccttc cattattttt tgccagcttt 660
tatttctgga gagcttatga ccaatgtaaa aaacgaggaa ctaagactca aaatcttaga 720
aaccagatac gctcaaagca agtcacagtg atgctgctga gcattgccat catctctgct 780
gtcttgtggc tccccgaatg ggtagcttgg ctgtgggtat ggcatctgaa ggctgcaggc 840
ccggccccac cacaaggttt catagccctg tctcaagtct tgatgttttc catctcttca 900
gcaaatcctc tcattttct tgtgatgtcg gaagagttca gggaaggctt gaaaggtgta 960
tggaaatgga tgataaccaa aaaacctcca actgtctcag agtctcagga aacaccagct 1020
ggcaactcag agggtcttcc tgacaaggtt ccatctccag aatccccagc atccatacca 1080
gaaaaagaga aacccagctc tccctcctct ggcaaaggga aaactgagaa ggcagagatt 1140
cccatccttc ctgacgtaga gcagttttgg catgagaggg acacagtccc ttctgtacag 1200
gacaatgacc ctatcccctg ggaacatgaa gatcaagaga caggggaagg tgttaaatag 1260

<210> 8

<211> 1182
<212> DNA
<213> Homo sapiens

<400> 8
atggagacca ccatggggtt catggatgac aatgccacca acacttccac cagcttcctt 60
tctgtgctca accctcatgg agcccatgcc acttccttcc cattcaactt cagctacagc 120
gactatgata tgcctttgga tgaagatgag gatgtgacca attccaggac gttctttgct 180
gccaagattg tcattgggat ggccctggtg ggcatcatgc tggtctgcgg cattggaaac 240
ttcatcttta tcgctgccct ggtccgctac aagaaactgc gcaacctcac caacctgctc 300
atcgccaacc tggccatctc tgacttcctg gtggccattg tctgctgccc ctttgagatg 360
gactactatg tggtgcgcca gctctcctgg gagcacggcc acgtcctgtg cacctctgtc 420
aactacctgc gcactgtctc tctctatgtc tccaccaatg ccctgctggc catcgccatt 480
gacaggtatc tggctattgt ccatccgctg agaccacgga tgaagtgcca aacagccact 540
ggcctgattg ccttggtgtg gacggtgtcc atcctgatcg ccatcccttc cgcctacttc 600
accaccgaga cggtcctcgt cattgtcaag agccaggaaa agatcttctg cggccagatc 660
tggcctgtgg accagcagct ctactacaag tcctacttcc tctttatctt tggcatagaa 720
ttcgtgggcc ccgtggtcac catgaccctg tgctatgcca ggatctcccg ggagctctgg 780
ttcaaggcgg tccctggatt ccagacagag cagatccgca agaggctgcg ctgccgcagg 840
aagacggtcc tggtgctcat gtgcatcctc accgcctacg tgctatgctg ggcgcccttc 900
tacggcttca ccatcgtgcg cgacttcttc cccaccgtgt ttgtgaagga gaagcactac 960
ctcactgcct tctacatcgt cgagtgcatc gccatgagca acagcatgat caacactctg 1020
tgcttcgtga ccgtcaagaa cgacaccgtc aagtacttca aaaagatcat gttgctccac 1080
tggaaggctt cttacaatgg cggtaagtcc agtgcagacc tggacctcaa gacaattggg 1140
atgcctgcca ccgaagaggt ggactgcatc agactaaaat aa 1182

<210> 9
<211> 28
<212> DNA
<213> Artificial Sequence

<220>
<223> Description of Artificial Sequence:an artificially synthesized primer sequence

<400> 9
atgccagcca acttcacaga gggcagct 28

<210> 10
<211> 28
<212> DNA
<213> Artificial Sequence

<220>
<223> Description of Artificial Sequence:an artificially synthesized primer sequence

<400> 10
ctagatgaat tctggcttgg acagaatc 28

<210> 11
<211> 28
<212> DNA
<213> Artificial Sequence

<220>
<223> Description of Artificial Sequence:an artificially synthesized primer sequence

<400> 11
atgggccccg gcgaggcgct gctggcgg 28

<210> 12
<211> 28
<212> DNA
<213> Artificial Sequence

<220>

<223> Description of Artificial Sequence:an artificially synthesized primer sequence

<400> 12
tcagtgtgtc tgctgcaggc aggaatca 28

<210> 13
<211> 30
<212> DNA
<213> Artificial Sequence

<220>
<223> Description of Artificial Sequence:an artificially synthesized primer sequence

<400> 13
atgctggcag ctgcctttgc agactctaac 30

<210> 14
<211> 30
<212> DNA
<213> Artificial Sequence

<220>
<223> Description of Artificial Sequence:an artificially synthesized primer sequence

<400> 14
ctatttaaca ccttcccctg tctcttgatc 30

<210> 15
<211> 28
<212> DNA

<213> Artificial Sequence

<220>
<223> Description of Artificial Sequence:an artificially synthesized primer sequence

<400> 15
atggagacca ccatggggtt catggatg 28

<210> 16
<211> 30
<212> DNA
<213> Artificial Sequence

<220>
<223> Description of Artificial Sequence:an artificially synthesized primer sequence

<400> 16
ttattttagt ctgatgcagt ccacctcttc 30

<210> 17
<211> 434
<212> PRT
<213> Homo sapiens

<400> 17
Met Glu Asp Leu Phe Ser Pro Ser Ile Leu Pro Pro Ala Pro Asn Ile
1 5 10 15

Ser Val Pro Ile Leu Leu Gly Trp Gly Leu Asn Leu Thr Leu Gly Gln
20 25 30

Gly Ala Pro Ala Ser Gly Pro Pro Ser Arg Arg Val Arg Leu Val Phe

35 40 45

Leu Gly Val Ile Leu Val Val Ala Val Ala Gly Asn Thr Thr Val Leu
50 55 60

Cys Arg Leu Cys Gly Gly Gly Gly Pro Trp Ala Gly Pro Lys Arg Arg
65 70 75 80

Lys Met Asp Phe Leu Leu Val Gln Leu Ala Leu Ala Asp Leu Tyr Ala
85 90 95

Cys Gly Gly Thr Ala Leu Ser Gln Leu Ala Trp Glu Leu Leu Gly Glu
100 105 110

Pro Arg Ala Ala Thr Gly Asp Leu Ala Cys Arg Phe Leu Gln Leu Leu
115 120 125

Gln Ala Ser Gly Arg Gly Ala Ser Ala His Leu Val Val Leu Ile Ala
130 135 140

Leu Glu Arg Arg Arg Ala Val Arg Leu Pro His Gly Arg Pro Leu Pro
145 150 155 160

Ala Arg Ala Leu Ala Ala Leu Gly Trp Leu Leu Ala Leu Leu Leu Ala
165 170 175

Leu Pro Pro Ala Phe Val Val Arg Gly Asp Ser Pro Ser Pro Leu Pro
180 185 190

Pro Pro Pro Pro Pro Thr Ser Leu Gln Pro Gly Ala Pro Pro Ala Ala
195 200 205

Arg Ala Trp Pro Gly Gln Arg Arg Cys His Gly Ile Phe Ala Pro Leu
210 215 220

Pro Arg Trp His Leu Gln Val Tyr Ala Phe Tyr Glu Ala Val Ala Gly

225 230 235 240

Phe Val Ala Pro Val Thr Val Leu Gly Val Ala Cys Gly His Leu Leu
245 250 255

Ser Val Trp Trp Arg His Arg Pro Gln Ala Pro Ala Ala Ala Ala Pro
260 265 270

Trp Ser Ala Ser Pro Gly Arg Ala Pro Ala Pro Ser Ala Leu Pro Arg
275 280 285

Ala Lys Val Gln Ser Leu Lys Met Ser Leu Leu Leu Ala Leu Leu Phe
290 295 300

Val Gly Cys Glu Leu Pro Tyr Phe Ala Ala Arg Leu Ala Ala Ala Trp
305 310 315 320

Ser Ser Gly Pro Ala Gly Asp Trp Glu Gly Glu Gly Leu Ser Ala Ala
325 330 335

Leu Arg Val Val Ala Met Ala Asn Ser Ala Leu Asn Pro Phe Val Tyr
340 345 350

Leu Phe Phe Gln Ala Gly Asp Cys Arg Leu Arg Arg Gln Leu Arg Lys
355 360 365

Arg Leu Gly Ser Leu Cys Cys Ala Pro Gln Gly Gly Ala Glu Asp Glu
370 375 380

Glu Gly Pro Arg Gly His Gln Ala Leu Tyr Arg Gln Arg Trp Pro His
385 390 395 400

Pro His Tyr His His Ala Arg Arg Glu Pro Leu Asp Glu Gly Gly Leu
405 410 415

Arg Pro Pro Pro Pro Arg Pro Arg Pro Leu Pro Cys Ser Cys Glu Ser

420 425 430

Ala Phe

<210> 18
<211> 451
<212> PRT
<213> Homo sapiens

<400> 18
Met Glu Ser Ser Pro Ile Pro Gln Ser Ser Gly Asn Ser Ser Thr Leu
1 5 10 15

Gly Arg Val Pro Gln Thr Pro Gly Pro Ser Thr Ala Ser Gly Val Pro
20 25 30

Glu Val Gly Leu Arg Asp Val Ala Ser Glu Ser Val Ala Leu Phe Phe
35 40 45

Met Leu Leu Leu Asp Leu Thr Ala Val Ala Gly Asn Ala Ala Val Met
50 55 60

Ala Val Ile Ala Lys Thr Pro Ala Leu Arg Lys Phe Val Phe Val Phe
65 70 75 80

His Leu Cys Leu Val Asp Leu Leu Ala Ala Leu Thr Leu Met Pro Leu
85 90 95

Ala Met Leu Ser Ser Ser Ala Leu Phe Asp His Ala Leu Phe Gly Glu
100 105 110

Val Ala Cys Arg Leu Tyr Leu Phe Leu Ser Val Cys Phe Val Ser Leu
115 120 125

Ala Ile Leu Ser Val Ser Ala Ile Asn Val Glu Arg Tyr Tyr Tyr Val
130 135 140

Val His Pro Met Arg Tyr Glu Val Arg Met Thr Leu Gly Leu Val Ala
145 150 155 160

Ser Val Leu Val Gly Val Trp Val Lys Ala Leu Ala Met Ala Ser Val
165 170 175

Pro Val Leu Gly Arg Val Ser Trp Glu Glu Gly Ala Pro Ser Val Pro
180 185 190

Pro Gly Cys Ser Leu Gln Trp Ser His Ser Ala Tyr Cys Gln Leu Phe
195 200 205

Val Val Val Phe Ala Val Leu Tyr Phe Leu Leu Pro Leu Leu Leu Ile
210 215 220

Leu Val Val Tyr Cys Ser Met Phe Arg Val Ala Arg Val Ala Ala Met
225 230 235 240

Gln His Gly Pro Leu Pro Thr Trp Met Glu Thr Pro Arg Gln Arg Ser
245 250 255

Glu Ser Leu Ser Ser Arg Ser Thr Met Val Thr Ser Ser Gly Ala Pro
260 265 270

Gln Thr Thr Pro His Arg Thr Phe Gly Gly Gly Lys Ala Ala Val Val
275 280 285

Leu Leu Ala Val Gly Gly Gln Phe Leu Leu Cys Trp Leu Pro Tyr Phe
290 295 300

Ser Phe His Leu Tyr Val Ala Leu Ser Ala Gln Pro Ile Ser Thr Gly
305 310 315 320

Gln Val Glu Ser Val Val Thr Trp Ile Gly Tyr Phe Cys Phe Thr Ser
325 330 335

Asn Pro Phe Phe Tyr Gly Cys Leu Asn Arg Gln Ile Arg Gly Glu Leu
340 345 350

Ser Lys Gln Phe Val Cys Phe Phe Lys Pro Ala Pro Glu Glu Glu Leu
355 360 365

Arg Leu Pro Ser Arg Glu Gly Ser Ile Glu Glu Asn Phe Leu Gln Phe
370 375 380

Leu Gln Gly Thr Gly Cys Pro Ser Glu Ser Trp Val Ser Arg Pro Leu
385 390 395 400

Pro Ser Pro Lys Gln Glu Pro Pro Ala Val Asp Phe Arg Ile Pro Gly
405 410 415

Gln Ile Ala Glu Glu Thr Ser Glu Phe Leu Glu Gln Gln Leu Thr Ser
420 425 430

Asp Ile Ile Met Ser Asp Ser Tyr Leu Arg Pro Ala Ala Ser Pro Arg
435 440 445

Leu Glu Ser
450

<210> 19
<211> 321
<212> PRT
<213> Homo sapiens

<400> 19
Met Asn Gln Thr Leu Asn Ser Ser Gly Thr Val Glu Ser Ala Leu Asn
1 5 10 15

Tyr Ser Arg Gly Ser Thr Val His Thr Ala Tyr Leu Val Leu Ser Ser
20 25 30

Leu Ala Met Phe Thr Cys Leu Cys Gly Met Ala Gly Asn Ser Met Val
35 40 45

Ile Trp Leu Leu Gly Phe Arg Met His Arg Asn Pro Phe Cys Ile Tyr
50 55 60

Ile Leu Asn Leu Ala Ala Ala Asp Leu Leu Phe Leu Phe Ser Met Ala
65 70 75 80

Ser Thr Leu Ser Leu Glu Thr Gln Pro Leu Val Asn Thr Thr Asp Lys
85 90 95

Val His Glu Leu Met Lys Arg Leu Met Tyr Phe Ala Tyr Thr Val Gly
100 105 110

Leu Ser Leu Leu Thr Ala Ile Ser Thr Gln Arg Cys Leu Ser Val Leu
115 120 125

Phe Pro Ile Trp Phe Lys Cys His Arg Pro Arg His Leu Ser Ala Trp
130 135 140

Val Cys Gly Leu Leu Trp Thr Leu Cys Leu Leu Met Asn Gly Leu Thr
145 150 155 160

Ser Ser Phe Cys Ser Lys Phe Leu Lys Phe Asn Glu Asp Arg Cys Phe
165 170 175

Arg Val Asp Met Val Gln Ala Ala Leu Ile Met Gly Val Leu Thr Pro
180 185 190

Val Met Thr Leu Ser Ser Leu Thr Leu Phe Val Trp Val Arg Arg Ser
195 200 205

Ser Gln Gln Trp Arg Arg Gln Pro Thr Arg Leu Phe Val Val Val Leu
210 215 220

Ala Ser Val Leu Val Phe Leu Ile Cys Ser Leu Pro Leu Ser Ile Tyr
225 230 235 240

Trp Phe Val Leu Tyr Trp Leu Ser Leu Pro Pro Glu Met Gln Val Leu
245 250 255

Cys Phe Ser Leu Ser Arg Leu Ser Ser Ser Val Ser Ser Ser Ala Asn
260 265 270

Pro Val Ile Tyr Phe Leu Val Gly Ser Arg Arg Ser His Arg Leu Pro
275 280 285

Thr Arg Ser Leu Gly Thr Val Leu Gln Gln Ala Leu Arg Glu Glu Pro
290 295 300

Glu Leu Glu Gly Gly Glu Thr Pro Thr Val Gly Thr Asn Glu Met Gly
305 310 315 320

Ala

<210> 20
<211> 333
<212> PRT
<213> Homo sapiens

<400> 20
Met Glu Lys Val Asp Met Asn Thr Ser Gln Glu Gln Gly Leu Cys Gln
1 5 10 15

Phe Ser Glu Lys Tyr Lys Gln Val Tyr Leu Ser Leu Ala Tyr Ser Ile

20 25 30

Ile Phe Ile Leu Gly Leu Pro Leu Asn Gly Thr Val Leu Trp His Phe
35 40 45

Trp Gly Gln Thr Lys Arg Trp Ser Cys Ala Thr Thr Tyr Leu Val Asn
50 55 60

Leu Met Val Ala Asp Leu Leu Tyr Val Leu Leu Pro Phe Leu Ile Ile
65 70 75 80

Thr Tyr Ser Leu Asp Asp Arg Trp Pro Phe Gly Glu Leu Leu Cys Lys
85 90 95

Leu Val His Phe Leu Phe Tyr Ile Asn Leu Tyr Gly Ser Ile Leu Leu
100 105 110

Leu Thr Cys Ile Ser Val His Gln Phe Leu Gly Val Cys His Pro Leu
115 120 125

Cys Ser Leu Pro Tyr Arg Thr Arg Arg His Ala Trp Leu Gly Thr Ser
130 135 140

Thr Thr Trp Ala Leu Val Val Leu Gln Leu Leu Pro Thr Leu Ala Phe
145 150 155 160

Ser His Thr Asp Tyr Ile Asn Gly Gln Met Ile Trp Tyr Asp Met Thr
165 170 175

Ser Gln Glu Asn Phe Asp Arg Leu Phe Ala Tyr Gly Ile Val Leu Thr
180 185 190

Leu Ser Gly Phe Leu Ser Leu Leu Gly His Phe Gly Val Leu Phe Thr
195 200 205

Asp Gly Gln Glu Pro Asp Gln Ala Arg Gly Glu Pro His Glu Asp Arg

210 215 220

Gln His Ser Pro Ser Gln Val His Pro Asp His Pro Thr Gly Val Trp
225 230 235 240

Pro Leu His Pro Leu Phe Cys Ala Leu Pro Tyr His Ser Leu Leu Leu
245 250 255

Pro His His Leu Leu Ser Ala Phe Ser Gly Leu Pro Ala Leu Asp Gly
260 265 270

Ser Gln Cys Gly Leu Gln Asp Met Glu Ala Ser Gly Glu Cys Glu Gln
275 280 285

Leu Pro Gln Pro Ser Pro Val Leu Ser Phe Lys Gly Gly Lys Asn Arg
290 295 300

Val Arg Leu Leu Gln Lys Leu Arg Gln Asn Lys Leu Gly Glu His Pro
305 310 315 320

Ala Gly Arg Lys Arg Cys Pro Gly Leu Asn Arg Ser Gly
325 330

<210> 21
<211> 508
<212> PRT
<213> Homo sapiens

<400> 21
Met Thr Ser Thr Cys Thr Asn Ser Thr Arg Glu Ser Asn Ser Ser His
1 5 10 15

Thr Cys Met Pro Leu Ser Lys Met Pro Ile Ser Leu Ala His Gly Ile
20 25 30

Ile Arg Ser Thr Val Leu Val Ile Phe Leu Ala Ala Ser Phe Val Gly
35 40 45

Asn Ile Val Leu Ala Leu Val Leu Gln Arg Lys Pro Gln Leu Leu Gln
50 55 60

Val Thr Asn Arg Phe Ile Phe Asn Leu Leu Val Thr Asp Leu Leu Gln
65 70 75 80

Ile Ser Leu Val Ala Pro Trp Val Val Ala Thr Ser Val Pro Leu Phe
85 90 95

Trp Pro Leu Asn Ser His Phe Cys Thr Ala Leu Val Ser Leu Thr His
100 105 110

Leu Phe Ala Phe Ala Ser Val Asn Thr Ile Val Val Val Ser Val Asp
115 120 125

Arg Tyr Leu Ser Ile Ile His Pro Leu Ser Tyr Pro Ser Lys Met Thr
130 135 140

Gln Arg Arg Gly Tyr Leu Leu Leu Tyr Gly Thr Trp Ile Val Ala Ile
145 150 155 160

Leu Gln Ser Thr Pro Pro Leu Tyr Gly Trp Gly Gln Ala Ala Phe Asp
165 170 175

Glu Arg Asn Ala Leu Cys Ser Met Ile Trp Gly Ala Ser Pro Ser Tyr
180 185 190

Thr Ile Leu Ser Val Val Ser Phe Ile Val Ile Pro Leu Ile Val Met
195 200 205

Ile Ala Cys Tyr Ser Val Val Phe Cys Ala Ala Arg Arg Gln His Ala
210 215 220

Leu Leu Tyr Asn Val Lys Arg His Ser Leu Glu Val Arg Val Lys Asp
225 230 235 240

Cys Val Glu Asn Glu Asp Glu Glu Gly Ala Glu Lys Lys Glu Glu Phe
245 250 255

Gln Asp Glu Ser Glu Phe Arg Arg Gln His Glu Gly Glu Val Lys Ala
260 265 270

Lys Glu Gly Arg Met Glu Ala Lys Asp Gly Ser Leu Lys Ala Lys Glu
275 280 285

Gly Ser Thr Gly Thr Ser Glu Ser Ser Val Glu Ala Arg Gly Ser Glu
290 295 300

Glu Val Arg Glu Ser Ser Thr Val Ala Ser Asp Gly Ser Met Glu Gly
305 310 315 320

Lys Glu Gly Ser Thr Lys Val Glu Glu Asn Ser Met Lys Ala Asp Lys
325 330 335

Gly Arg Thr Glu Val Asn Gln Cys Ser Ile Asp Leu Gly Glu Asp Asp
340 345 350

Met Glu Phe Gly Glu Asp Asp Ile Asn Phe Ser Glu Asp Asp Val Glu
355 360 365

Ala Val Asn Ile Pro Glu Ser Leu Pro Pro Ser Arg Arg Asn Ser Asn
370 375 380

Ser Asn Pro Pro Leu Pro Arg Cys Tyr Gln Cys Lys Ala Ala Lys Val
385 390 395 400

Ile Phe Ile Ile Ile Phe Ser Tyr Val Leu Ser Leu Gly Pro Tyr Cys
405 410 415

Phe Leu Ala Val Leu Ala Val Trp Val Asp Val Glu Thr Gln Val Pro
420 425 430

Gln Trp Val Ile Thr Ile Ile Ile Trp Leu Phe Phe Leu Gln Cys Cys
435 440 445

Ile His Pro Tyr Val Tyr Gly Tyr Met His Lys Thr Ile Lys Lys Glu
450 455 460

Ile Gln Asp Met Leu Lys Lys Phe Phe Cys Lys Glu Lys Pro Pro Lys
465 470 475 480

Glu Asp Ser His Pro Asp Leu Pro Gly Thr Glu Gly Gly Thr Glu Gly
485 490 495

Lys Ile Val Pro Ser Tyr Asp Ser Ala Thr Phe Pro
500 505

<210> 22
<211> 1305
<212> DNA
<213> Homo sapiens

<400> 22
atggaggatc tctttagccc ctcaattctg ccgccggcgc ccaacatttc cgtgcccatc 60
ttgctgggct ggggtctcaa cctgaccttg gggcaaggag cccctgcctc tgggccgccc 120
agccgccgcg tccgcctggt gttcctgggg gtcatcctgg tggtggcggt ggcaggcaac 180
accacagtgc tgtgccgcct gtgcggcggc ggcgggccct gggcgggccc caagcgtcgc 240
aagatggact tcctgctggt gcagctggcc ctggcggacc tgtacgcgtg cgggggcacg 300
gcgctgtcac agctggcctg ggaactgctg ggcgagcccc gcgcggccac gggggacctg 360
gcgtgccgct tcctgcagct gctgcaggca tccgggcggg gcgcctcggc ccacctcgtg 420
gtgctcatcg ccctcgagcg ccggcgcgcg gtgcgtcttc cgcacggccg gccgctgccc 480
gcgcgtgccc tcgccgccct gggctggctg ctggcactgc tgctggcgct gcccccggcc 540
ttcgtggtgc gcggggactc cccctcgccg ctgccgccgc cgccgccgcc aacgtccctg 600
cagccaggcg cgcccccggc cgcccgcgcc tggccggggc agcgtcgctg ccacgggatc 660

ttcgcgcccc tgccgcgctg gcacctgcag gtctacgcgt tctacgaggc cgtcgcgggc 720
ttcgtcgcgc ctgttacggt cctgggcgtc gcttgcggcc acctactctc cgtctggtgg 780
cggcaccggc cgcaggcccc cgcggctgca gcgccctggt cggcgagccc aggtcgagcc 840
cctgcgccca gcgcgctgcc ccgcgccaag gtgcagagcc tgaagatgag cctgctgctg 900
gcgctgctgt tcgtgggctg cgagctgccc tactttgccg cccggctggc ggccgcgtgg 960
tcgtccgggc ccgcgggaga ctgggaggga gagggcctgt cggcggcgct gcgcgtggtg 1020
gcgatggcca acagcgctct caatcccttc gtctacctct tcttccaggc gggcgactgc 1080
cggctccggc gacagctgcg gaagcggctg ggctctctgt gctgcgcgcc gcagggaggc 1140
gcggaggacg aggaggggcc ccggggccac caggcgctct accgccaacg ctggccccac 1200
cctcattatc accatgctcg gcgggaaccg ctggacgagg gcggcttgcg cccaccccct 1260
ccgcgcccca gacccctgcc ttgctcctgc gaaagtgcct tctag 1305

<210> 23
<211> 1356
<212> DNA
<213> Homo sapiens

<400> 23
atggagtcct cacccatccc ccagtcatca gggaactctt ccactttggg gagggtccct 60
caaaccccag gtccctctac tgccagtggg gtcccggagg tggggctacg ggatgttgct 120
tcggaatctg tggccctctt cttcatgctc ctgctggact tgactgctgt ggctggcaat 180
gccgctgtga tggccgtgat cgccaagacg cctgccctcc gaaaatttgt cttcgtcttc 240
cacctctgcc tggtggacct gctggctgcc ctgaccctca tgcccctggc catgctctcc 300
agctctgccc tctttgacca cgccctcttt ggggaggtgg cctgccgcct ctacttgttt 360
ctgagcgtgt gctttgtcag cctggccatc ctctcggtgt cagccatcaa tgtggagcgc 420
tactattacg tagtccaccc catgcgctac gaggtgcgca tgacgctggg gctggtggcc 480
tctgtgctgg tgggtgtgtg ggtgaaggcc ttggccatgg cttctgtgcc agtgttggga 540
agggtctcct gggaggaagg agctcccagt gtcccccccag gctgttcact ccagtggagc 600
cacagtgcct actgccagct tttgtggtg gtctttgctg tcctttactt tctgttgccc 660
ctgctcctca tacttgtggt ctactgcagc atgttccgag tggcccgcgt ggctgccatg 720
cagcacgggc cgctgcccac gtggatggag acaccccggc aacgctccga atctctcagc 780
agccgctcca cgatggtcac cagctcgggg gccccccaga ccaccccaca ccggacgttt 840
gggggaggga aagcagcagt ggttctcctg gctgtggggg gacagttcct gctctgttgg 900
ttgccctact tctctttcca cctctatgtt gccctgagtg ctcagcccat ttcaactggg 960
caggtggaga gtgtggtcac ctggattggc tacttttgct tcacttccaa ccctttcttc 1020

tatggatgtc tcaaccggca gatccggggg gagctcagca agcagtttgt ctgcttcttc 1080
aagccagctc cagaggagga gctgaggctg cctagccggg agggctccat tgaggagaac 1140
ttcctgcagt tccttcaggg gactggctgt ccttctgagt cctgggtttc ccgacccccta 1200
cccagcccca agcaggagcc acctgctgtt gactttcgaa tcccaggcca gatagctgag 1260
gagacctctg agttcctgga gcagcaactc accagcgaca tcatcatgtc agacagctac 1320
ctccgtcctg ccgcctcacc ccggctggag tcatga 1356

<210> 24
<211> 966
<212> DNA
<213> Homo sapiens

<400> 24
atgaaccaga ctttgaatag cagtgggacc gtggagtcag ccctaaacta ttccagaggg 60
agcacagtgc acacggccta cctggtgctg agctccctgg ccatgttcac ctgcctgtgc 120
gggatggcag gcaacagcat ggtgatctgg ctgctgggct ttcgaatgca caggaacccc 180
ttctgcatct atatcctcaa cctggcggca gccgacctcc tcttcctctt cagcatggct 240
tccacgctca gcctggaaac ccagcccctg gtcaatacca ctgacaaggt ccacgagctg 300
atgaagagac tgatgtactt tgcctacaca gtgggcctga gcctgctgac ggccatcagc 360
acccagcgct gtctctctgt cctcttccct atctggttca agtgtcaccg gcccaggcac 420
ctgtcagcct gggtgtgtgg cctgctgtgg acactctgtc tcctgatgaa cgggttgacc 480
tcttccttct gcagcaagtt cttgaaattc aatgaagatc ggtgcttcag ggtggacatg 540
gtccaggccg ccctcatcat gggggtctta accccagtga tgactctgtc cagcctgacc 600
ctctttgtct gggtgcggag gagctcccag cagtggcggc ggcagcccac acggctgttc 660
gtggtggtcc tggcctctgt cctggtgttc ctcatctgtt ccctgcctct gagcatctac 720
tggtttgtgc tctactggtt gagcctgccg cccgagatgc aggtcctgtg cttcagcttg 780
tcacgcctct cctcgtccgt aagcagcagc gccaaccccg tcatctactt cctggtgggc 840
agccggagga gccacaggct gcccaccagg tccctgggga ctgtgctcca acaggcgctt 900
cgcgaggagc ccgagctgga aggtggggag acgcccaccg tgggcaccaa tgagatgggg 960
gcttga 966

<210> 25
<211> 1002
<212> DNA

<213> Homo sapiens

<400> 25
atggagaagg tggacatgaa tacatcacag gaacaaggtc tctgccagtt ctcagagaag 60
tacaagcaag tctacctctc cctggcctac agtatcatct ttatcctagg gctgccacta 120
aatggcactg tcttgtggca cttctggggc caaaccaagc gctggagctg tgccaccacc 180
tatctggtga acctgatggt ggccgacctg ctttatgtgc tattgccctt cctcatcatc 240
acctactcac tagatgacag gtggcccttc ggggagctgc tctgcaagct ggtgcacttc 300
ctgttctata tcaaccttta cggcagcatc ctgctgctga cctgcatctc tgtgcaccag 360
ttcctaggtg tgtgccaccc actgtgttcg ctgccctacc ggacccgcag gcatgcctgg 420
ctgggcacca gcaccacctg ggccctggtg gtcctccagc tgctgcccac actggccttc 480
tcccacacgg actacatcaa tggccagatg atctggtatg acatgaccag ccaagagaat 540
tttgatcggc tttttgccta cggcatagtt ctgacattgt ctggctttct ttccctcctt 600
ggtcattttg gtgtgctatt cactgatggt caggagcctg atcaagccag aggagaacct 660
catgaggaca ggcaacacag cccgagccag gtccatccgg accatcctac tggtgtgtgg 720
cctcttcacc ctctgttttg tgcccttcca tatcactcgc tccttctacc tcaccatctg 780
ctttctgctt tctcaggact gccagctctt gatggcagcc agtgtggcct acaagatatg 840
gaggcctctg gtgagtgtga gcagctgcct caacccagtc ctgtactttc tttcaagggg 900
ggcaaaaata gagtcaggct cctccagaaa ctgaggcaga acaagttggg tgagcatcca 960
gctgggagga agagatgccc agggttgaac agatctgggt aa 1002

<210> 26
<211> 1527
<212> DNA
<213> Homo sapiens

<400> 26
atgacgtcca cctgcaccaa cagcacgcgc gagagtaaca gcagccacac gtgcatgccc 60
ctctccaaaa tgcccatcag cctggcccac ggcatcatcc gctcaaccgt gctggttatc 120
ttcctcgccg cctctttcgt cggcaacata gtgctggcgc tagtgttgca gcgcaagccg 180
cagctgctgc aggtgaccaa ccgttttatc tttaacctcc tcgtcaccga cctgctgcag 240
atttcgctcg tggccccctg ggtggtggcc acctctgtgc ctctcttctg gcccctcaac 300
agccacttct gcacggccct ggttagcctc acccacctgt tcgccttcgc cagcgtcaac 360
accattgtct tggtgtcagt ggatcgctac ttgtccatca tccaccctct ctcctacccg 420
tccaagatga cccagcgccg cggttacctg ctcctctatg gcacctggat tgtggccatc 480

ctgcagagca ctcctccact ctacggctgg ggccaggctg cctttgatga gcgcaatgct 540
ctctgctcca tgatctgggg ggccagcccc agctacacta ttctcagcgt ggtgtccttc 600
atcgtcattc cactgattgt catgattgcc tgctactccg tggtgttctg tgcagcccgg 660
aggcagcatg ctctgctgta caatgtcaag agacacagct tggaagtgcg agtcaaggac 720
tgtgtggaga atgaggatga agagggagca gagaagaagg aggagttcca ggatgagagt 780
gagtttcgcc gccagcatga aggtgaggtc aaggccaagg agggcagaat ggaagccaag 840
gacggcagcc tgaaggccaa ggaaggaagc acggggacca gtgagagtag tgtagaggcc 900
aggggcagcg aggaggtcag agagagcagc acggtggcca gcgacggcag catggagggt 960
aaggaaggca gcaccaaagt tgaggagaac agcatgaagg cagacaaggg tcgcacagag 1020
gtcaaccagt gcagcattga cttgggtgaa gatgacatgg agtttggtga agacgacatc 1080
aatttcagtg aggatgacgt cgaggcagtg aacatcccgg agagcctccc acccagtcgt 1140
cgtaacagca acagcaaccc tcctctgccc aggtgctacc agtgcaaagc tgctaaagtg 1200
atcttcatca tcattttctc ctatgtgcta tccctggggc cctactgctt tttagcagtc 1260
ctggccgtgt gggtggatgt cgaaacccag gtaccccagt gggtgatcac cataatcatc 1320
tggcttttct tcctgcagtg ctgcatccac ccctatgtct atggctacat gcacaagacc 1380
attaagaagg aaatccagga catgctgaag aagttcttct gcaaggaaaa gcccccgaaa 1440
gaagatagcc acccagacct gcccggaaca gagggtggga ctgaaggcaa gattgtccct 1500
tcctacgatt ctgctacttt tccttga 1527

<210> 27
<211> 28
<212> DNA
<213> Artificial Sequence

<220>
<223> Description of Artificial Sequence:an artificially synthesized primer sequence

<400> 27
atggaggatc tctttagccc ctcaattc 28

<210> 28
<211> 28
<212> DNA

<213> Artificial Sequence

<220>
<223> Description of Artificial Sequence:an artificially synthesized primer sequence

<400> 28
ctagaaggca ctttcgcagg agcaaggc 28

<210> 29
<211> 29
<212> DNA
<213> Artificial Sequence

<220>
<223> Description of Artificial Sequence:an artificially synthesized primer sequence

<400> 29
atggagtcct cacccatccc ccagtcatc 29

<210> 30
<211> 29
<212> DNA
<213> Artificial Sequence

<220>
<223> Description of Artificial Sequence:an artificially synthesized primer sequence

<400> 30
tcatgactcc agccggggtg aggcggcag 29

<210> 31
<211> 26
<212> DNA
<213> Artificial Sequence

<220>
<223> Description of Artificial Sequence:an artificially synthesized primer sequence

<400> 31
atgaaccaga ctttgaatag cagtgg 26

<210> 32
<211> 28
<212> DNA
<213> Artificial Sequence

<220>
<223> Description of Artificial Sequence:an artificially synthesized primer sequence

<400> 32
tcaagccccc atctcattgg tgcccacg 28

<210> 33
<211> 28
<212> DNA
<213> Artificial Sequence

<220>
<223> Description of Artificial Sequence:an artificially synthesized primer sequence

<400> 33

atggagaagg tggacatgaa tacatcac 28

<210> 34
<211> 29
<212> DNA
<213> Artificial Sequence

<220>
<223> Description of Artificial Sequence:an artificially synthesized primer sequence

<400> 34
ttacccagat ctgttcaacc ctgggcatc 29

<210> 35
<211> 28
<212> DNA
<213> Artificial Sequence

<220>
<223> Description of Artificial Sequence:an artificially synthesized primer sequence

<400> 35
atgacgtcca cctgcaccaa cagcacgc 28

<210> 36
<211> 29
<212> DNA
<213> Artificial Sequence

<220>
<223> Description of Artificial Sequence:an artificially

synthesized primer sequence

<400> 36
tcaaggaaaa gtagcagaat cgtaggaag 29

<210> 37
<211> 20
<212> DNA
<213> Artificial Sequence

<220>
<223> Description of Artificial Sequence:an artificially synthesized primer sequence

<400> 37
ccaggagcgt ttctatgcct 20

<210> 38
<211> 20
<212> DNA
<213> Artificial Sequence

<220>
<223> Description of Artificial Sequence:an artificially synthesized primer sequence

<400> 38
tgtgatcttt gctccctgca 20

<210> 39
<211> 28
<212> DNA
<213> Artificial Sequence

<220>
<223> Description of Artificial Sequence:an artificially synthesized TaqMan probe sequence

<220>
<221> misc_binding
<222> (1)
<223> Label FAM (6-carboxy-fluorescein)

<220>
<221> misc_binding
<222> (28)
<223> Label TAMRA (6-carboxy-N,N,N',N'-tetramethylrhodamine)

<400> 39
tcagaacctg ccagcattga atagtgcc 28

<210> 40
<211> 20
<212> DNA
<213> Artificial Sequence

<220>
<223> Description of Artificial Sequence:an artificially synthesized primer sequence

<400> 40
atctgctttg ccccgtatgt 20

<210> 41
<211> 20
<212> DNA

<213> Artificial Sequence

<220>
<223> Description of Artificial Sequence:an artificially synthesized primer sequence

<400> 41
accgccttgc tgtaggtcag 20

<210> 42
<211> 22
<212> DNA
<213> Artificial Sequence

<220>
<223> Description of Artificial Sequence:an artificially synthesized TaqMan probe sequence

<220>
<221> misc_binding
<222> (1)
<223> Label FAM (6-carboxy-fluorescein)

<220>
<221> misc_binding
<222> (22)
<223> Label TAMRA (6-carboxy-N,N,N',N'-tetramethylrhodamine)

<400> 42
tcgtgccctt cgtcaccgtg aa 22

<210> 43
<211> 21

<212> DNA
<213> Artificial Sequence

<220>
<223> Description of Artificial Sequence:an artificially
synthesized primer sequence

<400> 43
cccagcatcc ataccagaaa a 21

<210> 44
<211> 21
<212> DNA
<213> Artificial Sequence

<220>
<223> Description of Artificial Sequence:an artificially
synthesized primer sequence

<400> 44
ctgtgtccct ctcatgccaa a 21

<210> 45
<211> 28
<212> DNA
<213> Artificial Sequence

<220>
<223> Description of Artificial Sequence:an artificially
synthesized TaqMan probe sequence

<220>
<221> misc_binding
<222> (1)

<223> Label FAM (6-carboxy-fluorescein)

<220>
<221> misc_binding
<222> (28)
<223> Label TAMRA (6-carboxy-N,N,N',N'-tetramethylrhodamine)

<400> 45
tgagaaggca gagattccca tccttcct 28

<210> 46
<211> 19
<212> DNA
<213> Artificial Sequence

<220>
<223> Description of Artificial Sequence:an artificially synthesized primer sequence

<400> 46
tcgccatgag caacagcat 19

<210> 47
<211> 21
<212> DNA
<213> Artificial Sequence

<220>
<223> Description of Artificial Sequence:an artificially synthesized primer sequence

<400> 47
cactggactt accgccattg t 21

<210> 48
<211> 29
<212> DNA
<213> Artificial Sequence

<220>
<223> Description of Artificial Sequence:an artificially synthesized TaqMan probe sequence

<220>
<221> misc_binding
<222> (1)
<223> Label FAM (6-carboxy-fluorescein)

<220>
<221> misc_binding
<222> (29)
<223> Label TAMRA (6-carboxy-N,N,N',N'-tetramethylrhodamine)

<400> 48
agatcatgtt gctccactgg aaggcttct 29

<210> 49
<211> 23
<212> DNA
<213> Artificial Sequence

<220>
<223> Description of Artificial Sequence:an artificially synthesized primer sequence

<400> 49

ggatctcttt agcccctcaa ttc 23

<210> 50
<211> 21
<212> DNA
<213> Artificial Sequence

<220>
<223> Description of Artificial Sequence:an artificially synthesized primer sequence

<400> 50
aaggtcaggt tgagacccca g 21

<210> 51
<211> 25
<212> DNA
<213> Artificial Sequence

<220>
<223> Description of Artificial Sequence:an artificially synthesized TaqMan probe sequence

<220>
<221> misc_binding
<222> (1)
<223> Label FAM (6-carboxy-fluorescein)

<220>
<221> misc_binding
<222> (25)
<223> Label TAMRA (6-carboxy-N,N,N',N'-tetramethylrhodamine)

<400> 51
aacatttccg tgcccatctt gctgg 25

<210> 52
<211> 21
<212> DNA
<213> Artificial Sequence

<220>
<223> Description of Artificial Sequence:an artificially synthesized primer sequence

<400> 52
gctgttgact ttcgaatccc a 21

<210> 53
<211> 23
<212> DNA
<213> Artificial Sequence

<220>
<223> Description of Artificial Sequence:an artificially synthesized primer sequence

<400> 53
acggaggtag ctgtctgaca tga 23

<210> 54
<211> 26
<212> DNA
<213> Artificial Sequence

<220>

<223> Description of Artificial Sequence:an artificially synthesized TaqMan probe sequence

<220>
<221> misc_binding
<222> (1)
<223> Label FAM (6-carboxy-fluorescein)

<220>
<221> misc_binding
<222> (26)
<223> Label TAMRA (6-carboxy-N,N,N',N'-tetramethylrhodamine)

<400> 54
tgagttcctg gagcagcaac tcacca 26

<210> 55
<211> 20
<212> DNA
<213> Artificial Sequence

<220>
<223> Description of Artificial Sequence:an artificially synthesized primer sequence

<400> 55
ggctttcgaa tgcacaggaa 20

<210> 56
<211> 20
<212> DNA
<213> Artificial Sequence

<220>

<223> Description of Artificial Sequence:an artificially synthesized primer sequence

<400> 56

ggaagccatg ctgaagagga 20

<210> 57

<211> 28

<212> DNA

<213> Artificial Sequence

<220>

<223> Description of Artificial Sequence:an artificially synthesized TaqMan probe sequence

<220>

<221> misc_binding

<222> (1)

<223> Label FAM (6-carboxy-fluorescein)

<220>

<221> misc_binding

<222> (28)

<223> Label TAMRA (6-carboxy-N,N,N',N'-tetramethylrhodamine)

<400> 57

ttctgcatct atatcctcaa cctggcgg 28

<210> 58

<211> 21

<212> DNA

<213> Artificial Sequence

<220>
<223> Description of Artificial Sequence:an artificially synthesized primer sequence

<400> 58
tggcctcttc accctctgtt t 21

<210> 59
<211> 21
<212> DNA
<213> Artificial Sequence

<220>
<223> Description of Artificial Sequence:an artificially synthesized primer sequence

<400> 59
atcaagagct ggcagtcctg a 21

<210> 60
<211> 30
<212> DNA
<213> Artificial Sequence

<220>
<223> Description of Artificial Sequence:an artificially synthesized TaqMan probe sequence

<220>
<221> misc_binding
<222> (1)
<223> Label FAM (6-carboxy-fluorescein)

<220>
<221> misc_binding
<222> (30)
<223> Label TAMRA
(6-carboxy-N,N,N',N'-tetramethylrhodamine)

<400> 60
tccatatcac tcgctccttc tacctcacca 30

<210> 61
<211> 19
<212> DNA
<213> Artificial Sequence

<220>
<223> Description of Artificial Sequence:an artificially synthesized primer sequence

<400> 61
ccaaaatgcc catcagcct 19

<210> 62
<211> 20
<212> DNA
<213> Artificial Sequence

<220>
<223> Description of Artificial Sequence:an artificially synthesized primer sequence

<400> 62
gcactatgtt gccgacgaaa 20

<210> 63
<211> 26
<212> DNA
<213> Artificial Sequence

<220>
<223> Description of Artificial Sequence:an artificially synthesized TaqMan probe sequence

<220>
<221> misc_binding
<222> (1)
<223> Label FAM (6-carboxy-fluorescein)

<220>
<221> misc_binding
<222> (26)
<223> Label TAMRA (6-carboxy-N,N,N',N'-tetramethylrhodamine)

<400> 63
catccgctca accgtgctgg ttatct 26